(19) Europäisches Patentamt
European Patent Office
Office européen des brevets

(11) Veröffentlichungsnummer: **0 272 301 B1**

(12) # EUROPÄISCHE PATENTSCHRIFT

(45) Veröffentlichungstag der Patentschrift: **11.11.92**

(51) Int. Cl.$^5$: **C09K 19/38**, C09K 19/30, C09K 19/34

(21) Anmeldenummer: **87904248.9**

(22) Anmeldetag: **19.06.87**

(86) Internationale Anmeldenummer:
**PCT/EP87/00317**

(87) Internationale Veröffentlichungsnummer:
**WO 88/00227 (14.01.88 88/02)**

(54) **POLYMERISIERBARE FLÜSSIGKRISTALLMATERIALIEN UND FLÜSSIGKRISTALLINE PHASEN AUFWEISENDE POLYMERE.**

(30) Priorität: **27.06.86 DE 3621581**

(43) Veröffentlichungstag der Anmeldung:
**29.06.88 Patentblatt 88/26**

(45) Bekanntmachung des Hinweises auf die Patenterteilung:
**11.11.92 Patentblatt 92/46**

(84) Benannte Vertragsstaaten:
**CH DE FR GB IT LI NL**

(56) Entgegenhaltungen:
**EP-A- 0 172 450**
**DE-A- 3 533 333**

(73) Patentinhaber: **MERCK PATENT GESELLSCHAFT MIT BESCHRÄNKTER HAFTUNG**
**Frankfurter Strasse 250 Postfach 4119**
**W-6100 Darmstadt(DE)**

(72) Erfinder: **DORSCH, Dieter**
**Reuterallee 24**
**W-6100 Darmstadt(DE)**
Erfinder: **EIDENSCHINK, Rudolf**
**Konrad-Adenauer-Str. 1**
**W-6109 Mühltal(DE)**
Erfinder: **WÄCHTLER, Andreas**
**Goethestr. 34**
**W-6103 Griesheim(DE)**
Erfinder: **RIEGER, Bernhard**
**Südring 31**
**W-6102 Pfungstadt(DE)**
Erfinder: **FINKELMANN, Heino**
**Fillibachstr. 31**
**W-7800 Freiburg(DE)**

EP 0 272 301 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft polymerisierbare Flüssigkristallmaterialien sowie flüssigkristalline Phasen aufweisende Polyacrylate, Polymethacrylate oder Polysiloxane, die chemisch gebundene mesogene Gruppen aufweisen, in denen eine querpolarisierende Nitrilgruppe enthalten ist.

Es ist bereits eine Reihe von flüssigkristallinen Seitenkettenpolymeren bekannt. So werden beispielsweise in der DE-OS 29 44 591 und der EP-PS 0 060 335 Organopolysiloxane und in der DE-OS 28 31 909 sowie bei Springer und Weigelt, Makromol. Chem. 184 (1983) 1489, Polymethacrylate mit mesogenen Seitengruppen beschrieben.

Beispielsweise sind auch mit 4'-Cyanbiphenyl-4-yl als mesogene Gruppe modifizierte Polyacryl- und Polymethacrylsäureester bekannt. Nematische Phasen solcher Polymerzusammensetzungen liegen meist bei Temperaturen oberhalb 100°. Vielfach zeigen solche Materialien auch kristallines Verhalten, verbunden mit dem Fehlen mesomorpher Eigenschaften.

Aufgabe der vorliegenden Erfindung war es, flüssigkristalline Phasen aufweisende Polymermaterialien zu finden, die die beschriebenen Nachteile nicht oder nur in geringem Maß aufweisen.

Es wurde nun gefunden, daß Polymermaterialien, die chemisch gebundene mesogene Gruppen mit einer querpolarisierenden Nitrilgruppe enthalten, überraschend breite Mesophasenbereiche, eine in weiten Grenzen variierbare Doppelbrechung und eine negative dielektrische Anisotropie aufweisen. Sie sind außerdem leicht zu Körpern beliebiger Form mit anisotropen Eigenschaften verarbeitbar und weisen eine hohe chemische Stabilität auf.

Gegenstand der Erfindung sind flüssigkristalline Phasen aufweisende Polyacrylate, Polymethacrylate oder Polisiloxane, die chemisch gebundene mesogene Gruppen der Formel XIaa oder XIcc enthalten

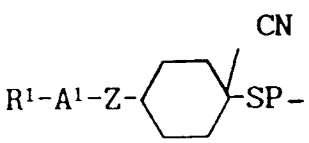

XIaa

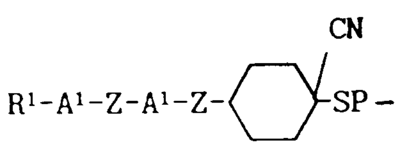

XIcc

worin

- $R^1$ eine Alkylgruppe mit bis zu 15 C-Atomen, worin auch eine oder mehrere $CH_2$-Gruppen durch eine Gruppierung aus der Gruppe -O-, -CO-O-, -O-CO-, -CRR'-T- und -CH=CH-(trans) ersetzt sein können, wobei 2 Heteroatome nicht miteinander verknüpft sind,
- $A^1$ jeweils unabhängig voneinander eine unsubstituierte oder eine durch CN substituierte 1,4-Cyclohexylengruppe, oder eine 1,4-Phenylengruppe.
- Z jeweils -CO-O-, -O-CO-, $-CH_2CH_2$ oder eine Einfachbindung,
- Sp Alkylen mit 2-18 C-Atomen, worin auch eine oder zwei nicht benachbarte $CH_2$-Gruppen durch -O-, -CO-, -O-CO-, -CO-O-, -CRR'-T- oder -CH=CH- ersetzt sein können, oder eine Einfachbindung,
- T -COO-, -OCO- oder eine Einfachbindung,
- R H oder eine Alkylgruppe mit bis zu 6 C-Atomen und
- R' Halogen oder CN

bedeuten.

Vor- und nachstehend haben $R^1$, $A^1$, Z, Sp, T, R, R', $R^2$, $A^3$, $Z^1$, M und $Q^2$ die angegebene Bedeutung sofern nicht ausdrücklich etwas anderes vermerkt ist.

In den Verbindungen der vor- und nachstehenden Formeln bedeutet $R^1$ vorzugsweise Alkyl oder Alkoxy.

Weiterhin bevorzugt sind Verbindungen der vor- und nachstehenden Formeln, in denen $R^1$ eine Alkylgruppe ist, worin eine oder mehrere $CH_2$-Gruppen, vorzugsweise eine $CH_2$-Gruppe, ersetzt ist, vorzugsweise durch eine -CO-, -CO-O-, O-CO- oder CRR'-T-Gruppe. T bedeutet dabei vorzugsweise -CO-O- oder eine Einfachbindung, R ist vorzugsweise H oder eine unverzweigte Alkylgruppe mit bis zu 3 C-Atomen und bedeutet demnach bevorzugt Methyl, Ethyl, Propyl, ferner auch Butyl, Pentyl oder Hexyl. R' ist Halogen oder CN, vorzugsweise Fluor, Chlor oder CN.

$A^1$ ist bevorzugt 1,4-Cyclohexylen, das auch durch CN substituiert vorliegen kann, wobei eine Monosubstitution in der 1- oder 4-Stellung bevorzugt ist.

Z bedeutet jeweils unabhängig voneinander vorzugsweise Einfachbindungen, -CO-O- oder -O-CO-. R, R' und T haben dabei die angegebenen bevorzugten Bedeutungen.

Sp hat vorzugsweise die Bedeutung einer geradkettigen Alkylengruppe mit 2-10 C-Atomen und bedeutet demnach vorzugsweise Ethylen, Propylen, Butylen, Pentylen, Hexylen, Heptylen, Octylen, Nonylen oder Decylen, ferner auch Undecylen, Dodecylen, Tridecylen, Tetradecylen, Pentadecylen, Hexadecylen, Heptadecylen oder Octadecylen. Ferner können die Alkylengruppen aber auch verzweigt sein und demnach beispielsweise Isopropylen, 1-Methylpropylen, Isobutylen, 2-Methylbutylen, 3-Methylbutylen oder 2-Methylpentylen bedeuten. Weiterhin sind für Sp Alkylengruppen bevorzugt, in denen eine oder zwei nicht benachbarte $CH_2$-Gruppen ersetzt sind, vorzugsweise durch -O-, -OCO-, -CO-O-, ferner bevorzugt durch -CRR'-T-,. Halogen, R, R' und T haben dabei die angegebenen bevorzugten Bedeutungen.

Falls $R^1$ Alkylrest oder Alkoxyrest bedeutet, so kann dieser geradkettig oder verzweigt sein. Vorzugsweise ist er geradkettig, hat 2, 3, 4, 5, 6 oder 7 C-Atome und bedeutet demanch bevorzugt Ethyl, Propyl, Butyl, Pentyl, Hexyl, Heptyl, Ethoxy, Propoxy, Butoxy, Pentoxy, Hexoxy oder Heptoxy, ferner Methyl, Octyl, Nonyl, Decyl, Undecyl, Dodecyl, Tridecyl, Tetradecyl, Pentadecyl, Methoxy, Octoxy, Nonoxy, Decoxy, Undecoxy, Dodecoxy, Tridecoxy oder Tetradecoxy.

Oxaalkyl bedeutet vorzugsweise geradkettiges 2-Oxapropyl (= Methoxymethyl), 2- (= Ethoxymethyl) oder 3-Oxabutyl (= 2-Methoxyethyl), 2-, 3- oder 4-Oxapentyl, 2-, 3-, 4- oder 5-Oxahexyl, 2-, 3-, 4-, 5- oder 6-Oxaheptyl, 2-, 3-, 4-, 5-, 6- oder 7-Oxaoctyl, 2-, 3-, 4-, 5-, 6-, 7- oder 8-Oxanonyl, 2-, 3-, 4-, 5-, 6-, 7-, 8- oder 9-Oxadecyl.

Falls $R^1$ einen Alkylrest bedeutet, in dem eine $CH_2$-Gruppe durch -CH=CH- ersetzt ist, so kann dieser geradkettig oder verzweigt sein. Vorzugsweise ist er geradkettig und hat 2 bis 10 C-Atome, und bedeutet demnach besonders Vinyl, Prop-1- oder Prop-2-enyl, But-1-, 2- oder But-3-enyl, Pent-1-, 2-, 3- oder Pent-4-enyl, Hex-1-, 2-, 3-, 4- oder Hex-5-enyl, Hept-1-, 2-, 3-, 4-, 5- oder Hept-6-enyl, Oct-1-, 2-, 3-, 4-, 5-, 6- oder Oct-7-enyl, Non-1-, 2-, 3-, 4-, 5-, 6-, 7- oder Non-8-enyl, Dec-1-, 2-, 3-, 4-, 5-, 6-, 7-, 8- oder Dec-9-enyl.

Verbindungen der Formeln XIaa und XIcc mit verzweigter Flügelgruppe $R^1$ können gelegentlich wegen Verminderung der Neigung zu Kristallisation von Bedeutung sein, insbesondere aber als chirale Bestandteile von Polymeren, wenn sie optisch aktiv sind. Man erhält so cholesterische Polymere, die als thermochrome Folien verwendet werden können, oder auch Polymere mit getilteten smektischen Phasen, die ferroelektrische, piezoelektrische, pyroelektrische und/oder nichtlinear optische Eigenschaften geradzahliger Ordnung, insbesondere 2. Ordnung, besitzen.

Verzweigte Gruppen dieser Art enthalten in der Regel nicht mehr als eine Kenttenverzweigung. Bevorzugte verzweigte Reste $R^1$ sind Isopropyl, 2-Butyl (= 1-Methylpropyl), Isobutyl (= 2-Methylpropyl), 2-Methylbutyl, Isopentyl (= 3-Methylbutyl), 2-Methylpentyl, 3-Methylpentyl, 2-Ethylhexyl, 2-Propylpentyl, 2-Octyl, Isopropoxy, 2-Methylpropoxy, 2-Methylbutoxy, 3-Methylbutoxy, 2-Methylpentoxy, 3-Methylpentoxy, 2-Ethylhexoxy, 1-Methylhexoxy, 1-Methylheptoxy (= 2-Octyloxy), 2-Oxa-3-methylbutyl, 3-Oxa-4-methylpentyl, 4-Methylhexyl, 2-Nonyl, 2-Decyl, 2-Dodecyl, 6-Methyloctoxy, 6-Methyloctanoyloxy, 5-Methylheptyloxycarbonyl, 2-Methylbutyryloxy, 3-Methylvaleryloxy, 4-Methylhexanoyloxy, 2-Chlorpropionyloxy, 2-Chlor-3-methylbutyryloxy, 2-Chlor-4-methylvaleryloxy, 2-Chlor-3-methylvaleryloxy, 2-Methyl-3-oxapentyl, 2-Methyl-3-oxahexyl.

Die Formeln XIaa und XIcc umfassen die Racemate dieser Verbindungen als auch die optischen Antipoden sowie deren Gemische.

Unter den Verbindungen der Formeln XIaa und XIcc sind diejenigen bevorzugt, in denen mindestens einer der darin enthaltenen Reste eine der angegebenen bevorzugten Bedeutungen hat.

Neu sind die polymerisierbaren Flüssigkristallmaterialien der Formel I', die ebenfalls Gegenstand der Erfindung sind.

Die Erfindung betrifft also auch polymerisierbare Flüssigkristallmaterialien der Formel I'

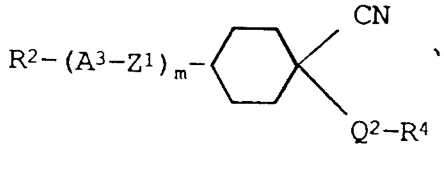

I'

worin

$R^2$ eine Alkylgruppe mit bis zu 15 C-Atomen, worin auch eine oder mehrere $CH_2$-Gruppen durch eine Gruppierung aus der Gruppe -O-, -CO-O-, -O-CO-, -CH=CH-(trans) und -CRR'-T- ersetzt sein

können, wobei 2 Heteroatome nicht miteinander verknüpft sind,

- $A^3$ jeweils eine unsubstituierte oder eine durch CN substituierte 1,4-Cyclohexylengruppe, oder eine 1,4-Phenylengruppe,
- $Z^1$ -CO-O-, -O-CO-, $-CH_2CH_2-$ oder eine Einfachbindung,
- m 1 oder 2
- $Q^2$ Alkylen mit 3-18 C-Atomen, worin auch eine oder zwei nicht benachbarte $CH_2$-Gruppen durch -O-, -CO-, -O-CO-, -CO-O-, -CRR'-T- oder -CH=CH-ersetzt sein können,
- R H oder ein Alkylgruppe mit bis zu 6 C-Atomen,
- R' Halogen oder CN,
- T -CO-O-, -O-CO- oder eine Einfachbindung,
- $R^4$ eine unsubstituierte oder durch eine $CH_3$-Gruppe substituierte Vinylgruppe, oder eine Carboxy- oder Hydroxygruppe,

mit der Maßgabe, daß im Falle $R^4$ = unsubstituiertes Vinyl und $Q^2$ = Alkylen oder Alkylenoxy, die Alkylengruppe mindestens 4 C-Atome aufweist.

Es sind bereits eine ganze Reihe von flüssigkristallinen Polymeren bekannt, beispielsweise mit 4'-Cyanbiphenyl-4-yl als mesogene Gruppe modifizierte Polyacryl- und Polymethacrylsäureester. Für die Darstellung solcher flüssigkristallinen Polymeren mit mesogenen Seitengruppen benötigt man reaktions- oder polymerisationsfähige mesogene Verbindungen.

Es sind auch einige Ausgangsmaterialien bekannt, die auf ein polymeres Rückgrat aufgepfropft werden können, wie z.B.

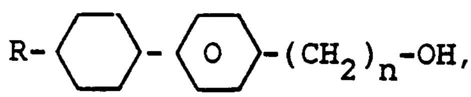 n = 4-7

oder

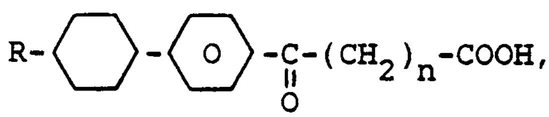 n = 3-6

oder

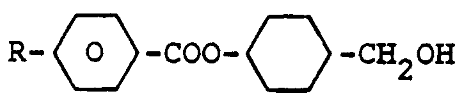

beschrieben in EP 58 981, oder die nach Veresterung mit Verbindungen, die eine olefinisch ungesättigte Gruppe aufweisen, polymerisiert werden können.

Aufgabe der vorliegenden Erfindung war es nun auch, leicht zugängliche, polymerisierbare Flüssigkristallmaterialien zu finden, deren Polymerzusammensetzungen flüssigkristalline Phasen aufweisen.

Es wurde nun gefunden, daß die Verbindungen der Formel I' hervorragend als Vorstufen zur Herstellung von flüssigkristalline Phasen aufweisende Polymerzusammensetzungen geeignet sind, die überraschend breite Mesophasenbereiche, eine in weiten Grenzen variierbare Doppelbrechung aufweisen. Sie sind außerdem leicht zu Körpern beliebiger Form mit anisotropen Eigenschaften verarbeitbar und weisen eine hohe chemische Stabilität auf.

Gegenstand der Erfindung sind die Verbindungen der Formel I' sowie deren Verwendung als polymerisierbare Flüssigkristallmaterialien.

Ferner ist Gegenstand der Erfindung die Verwendung der Verbindungen der Formel I' zur Herstellung von flüssigkristalline Phasen aufweisende Polymerzusammensetzungen charakterisiert in Anspruch 1.

Der Einfachheit halber bedeuten im folgenden für die Formeln I' und ihre Teilformeln Cy jeweils eine unsubstituierte oder eine durch CN substituierte 1,4-Cyclohexylengruppe.

Cyc bedeutet im folgenden die Cyclohexylengruppe der Formeln I', an welcher CN (in axialer Position) und $Q^2$-$R^4$ stehen

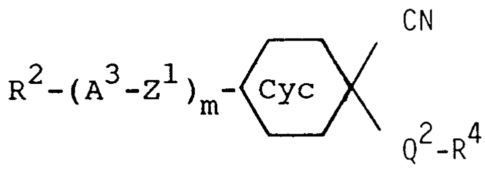

I'

Die Verbindungen der Formel I' umfassen Verbindungen mit zwei Ringen (Teilformeln I'a bis I'b), mit drei Ringen (I'c bis I'f) und mit vier Ringen (I'g bis I'n):

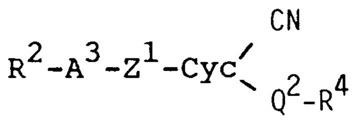

I'a

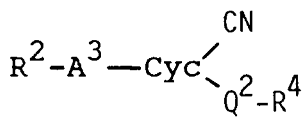

I'b

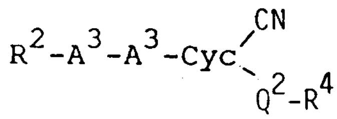

I'c

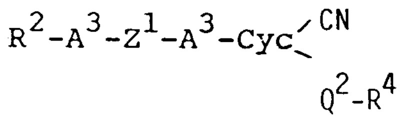

I'd

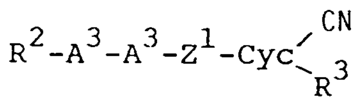

I'e

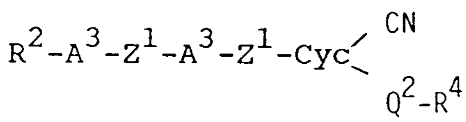

I'f

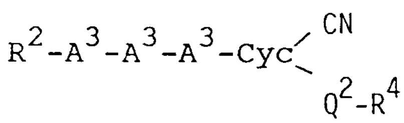

I'g

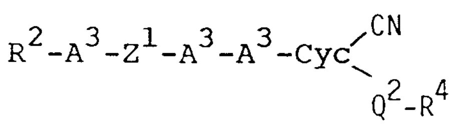

I'h

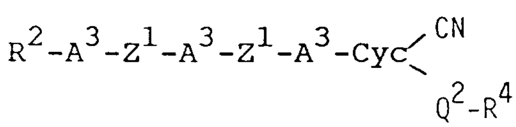

I'i

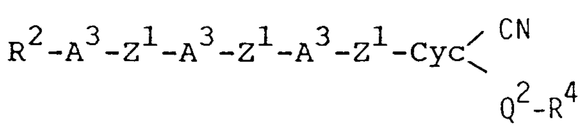

I'j

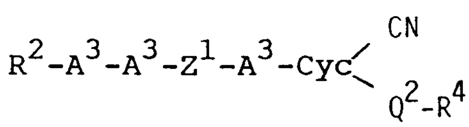

I'k

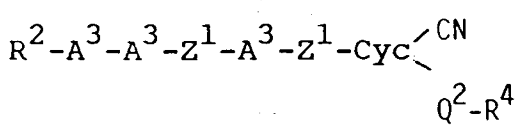

I'l

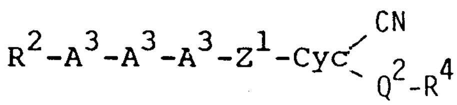 I'm

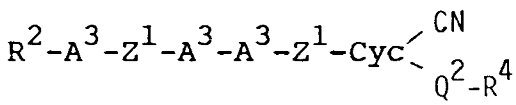 I'n

Darunter sind diejenigen der Formeln I'a, I'b, I'c, I'd, I'e, I'f, I'g und I'h besonders bevorzugt, insbesondere bevorzugt sind Verbindungen der Formeln I'a, I'b, I'c, I'e und I'f.

Die bevorzugten Verbindungen der Formel I'a umfassen solche der Teilformeln I'aa und I'$a_c$:

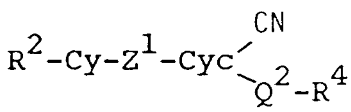 I'aa

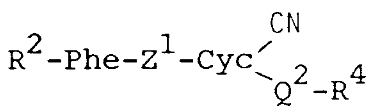 I'ac

Die bevorzugten Verbindungen der Formel I'b umfassen solche der Teilformeln I'ba und I'bb:

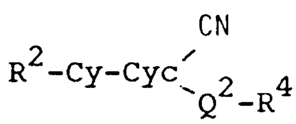 I'ba

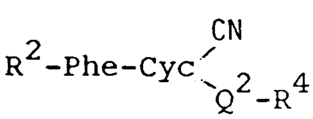 I'bb

Die bevorzugten Verbindungen der Formel I'c umfassen solche der Teilformeln I'ca bis I'cd:

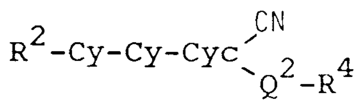 I'ca

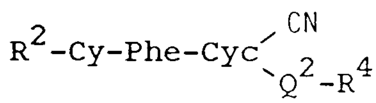 I'cb

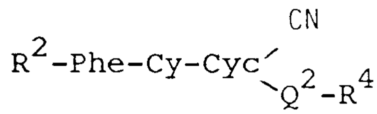 I'cc

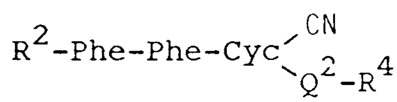 I'cd

Die bevorzugten Verbindungen der Formel I'd umfassen solche der Teilformeln I'da bin I'dd:

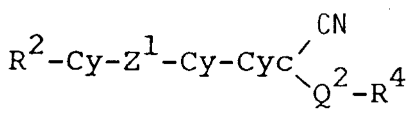 I'da

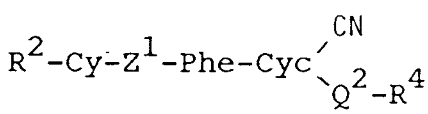 I'db

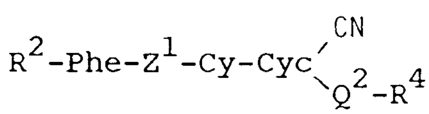 I'dc

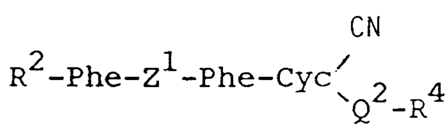 I'dd

Die bevorzugten Verbindungen der Formel I'e umfassen solche der Teilformeln I'ea bis I'ed:

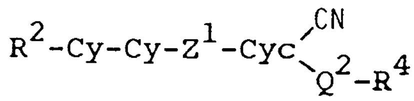
I'ea

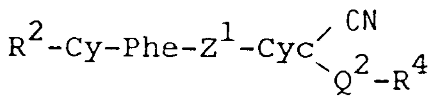
I'eb

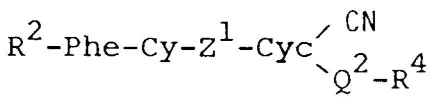
I'ec

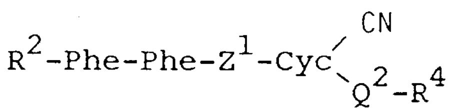
I'ed

Darunter sind diejenigen der Formeln I'ea und I'ed besonders bevorzugt.
Die bevorzugten Verbindungen der Formel I'f umfassen diejenigen der Teilformeln I'fa bis I'fd:

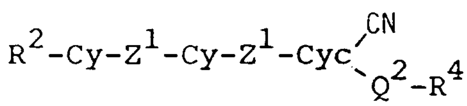
I'fa

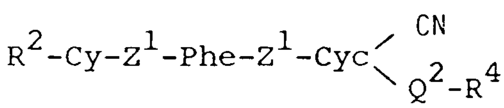
I'fb

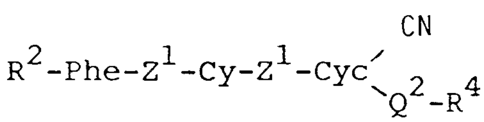
I'fc

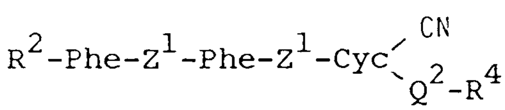
I'fd

Die bevorzugten Verbindungen der Formel I'g umfassen diejenigen der Teilformeln I'ga bis I'gh:

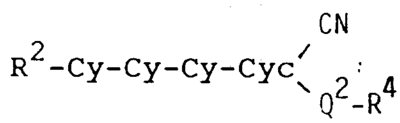 I'ga

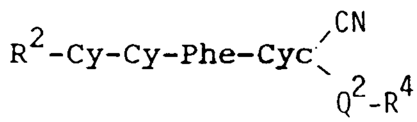 I'gb

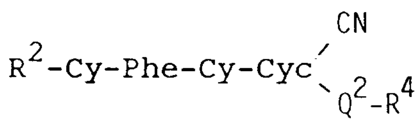 I'gc

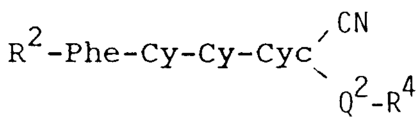 I'gd

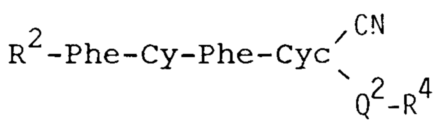 I'ge

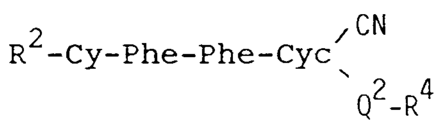 I'gf

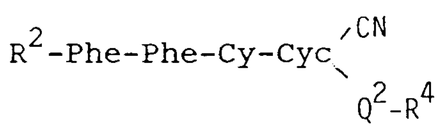 I'gg

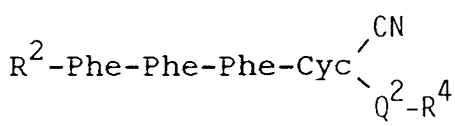 I'gh

Die bevorzugten Verbindungen der Formel I'h umfassen diejenigen der Teilformeln I'ha bis I'hh:

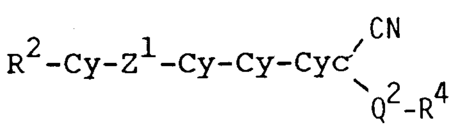 I'ha

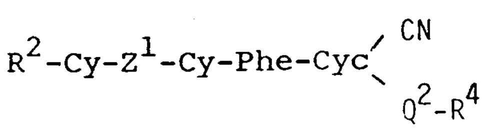 I'hb

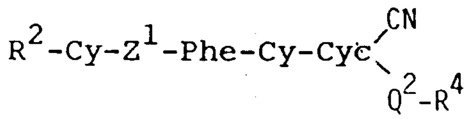 I'hc

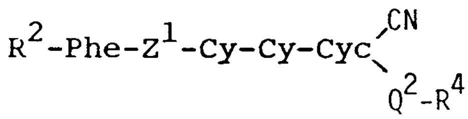 I'hd

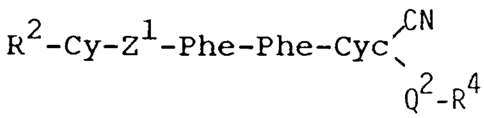 I'he

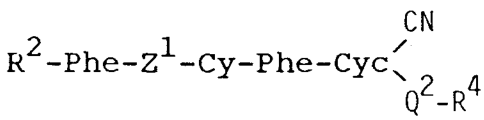 I'hf

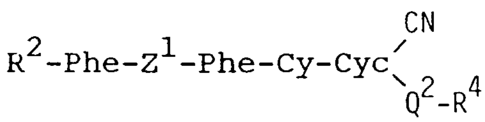 I'hg

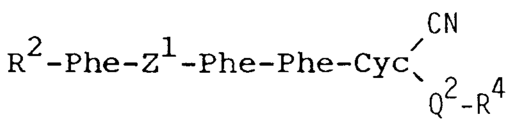 I'hh

Die bevorzugten Verbindungen der Formel I'i umfassen solche der Teilformeln I'ia bis I'ih:

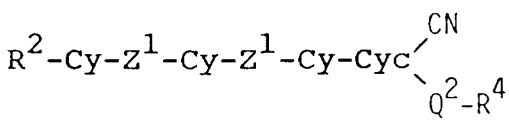 I'ia

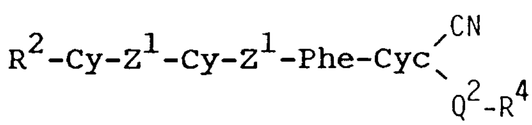 I'ib

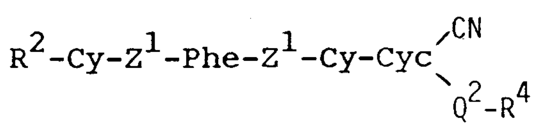 I'ic

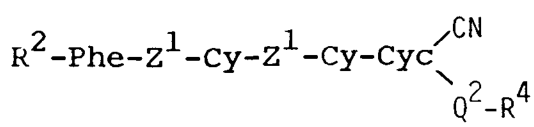 I'id

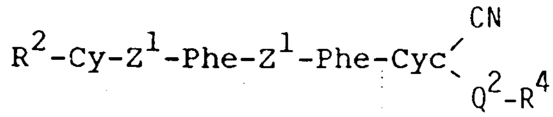

I'ie

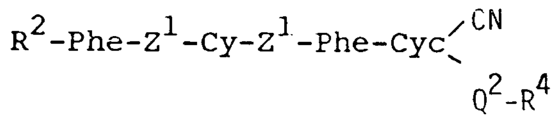

I'if

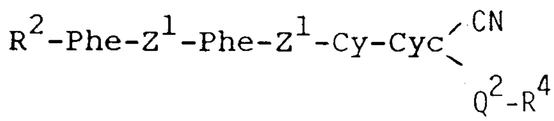

I'ig

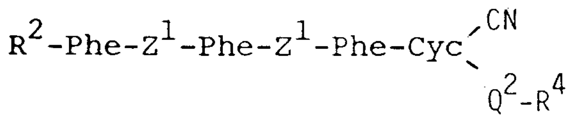

I'ih

Die bevorzugten Verbindungen der Formel I'j umfassen solche der Teilformeln I'ja bis I'jh:

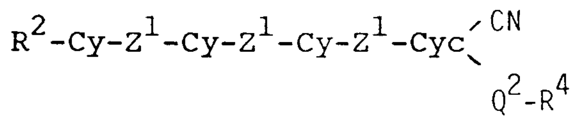

I'ja

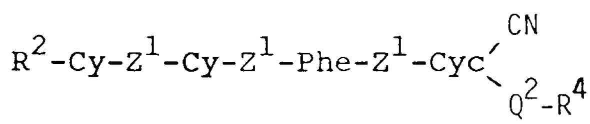

I'jb

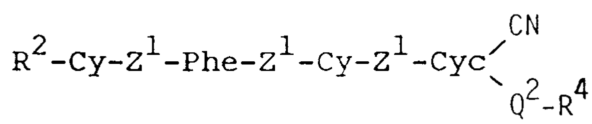

I'jc

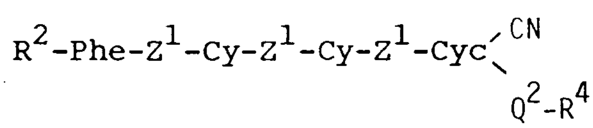

I'jd

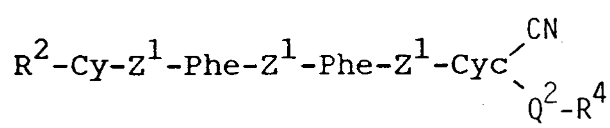

I'je

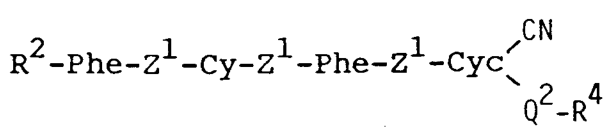

I'jf

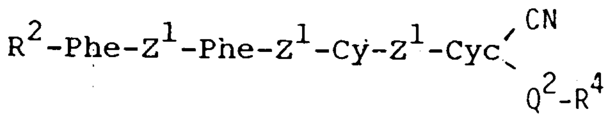 I'jg

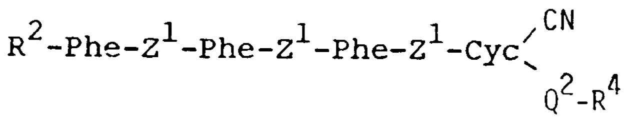 I'jh

Die bevorzugten Verbindungen der Formel I'k umfassen solche der Teilformeln I'ka bis I'kh:

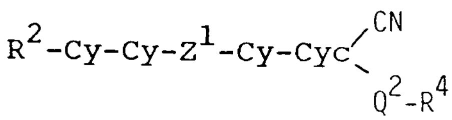 I'ka

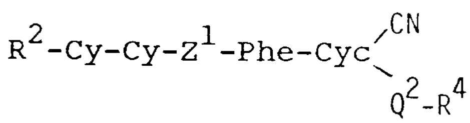 I'kb

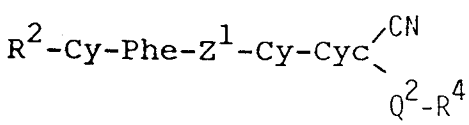 I'kc

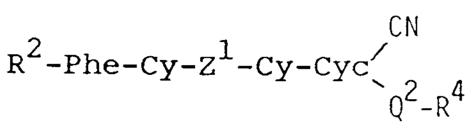 I'kd

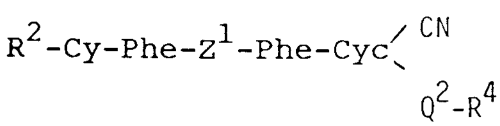 I'ke

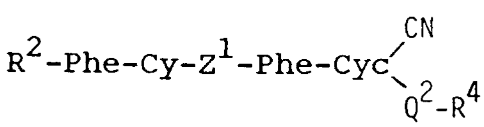 I'kf

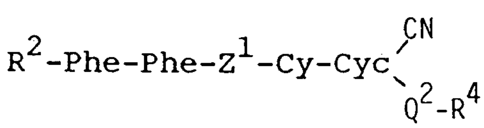 I'kg

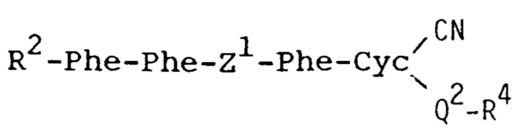 I'kh

Die bevorzugten Verbindungen der Formel I'l umfassen solche der Teilformeln I'la bis I'lh:

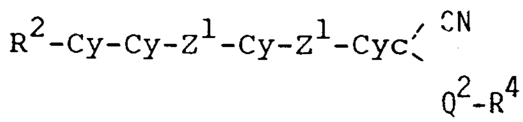
I'la

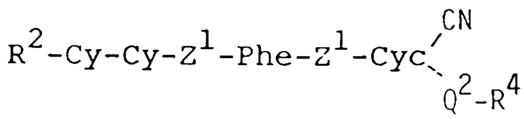
I'lb

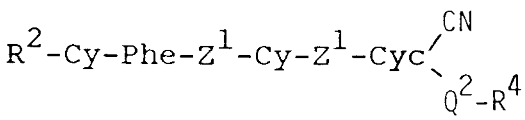
I'lc

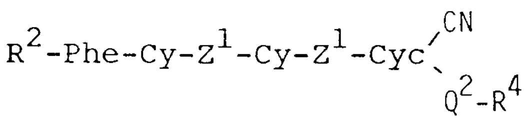
I'ld

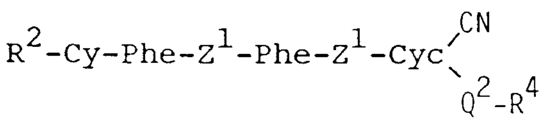
I'le

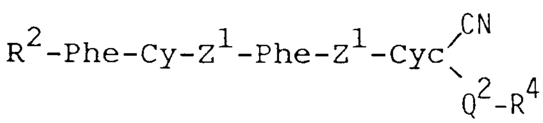
I'lf

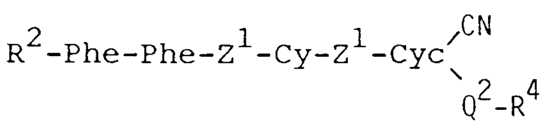
I'lg

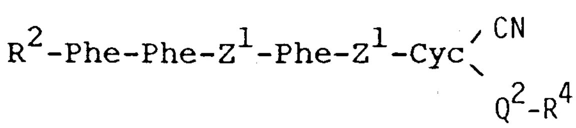
I'lh

Die bevorzugten Verbindungen der Formel I'm umfassen solche der Teilformeln I'ma bis I'mh:

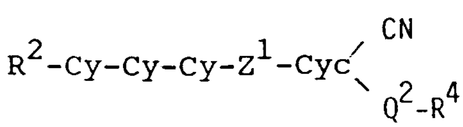
I'ma

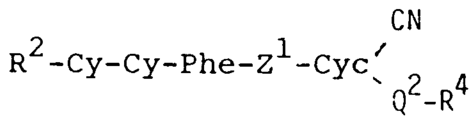

I'mb

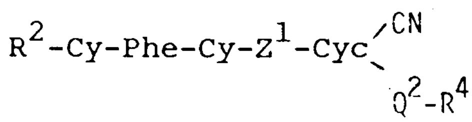

I'mc

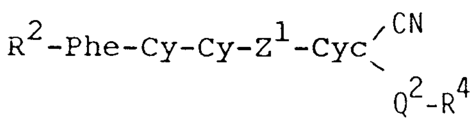

I'md

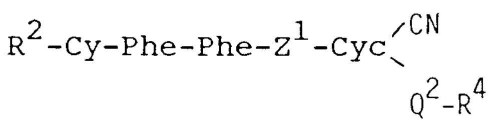

I'me

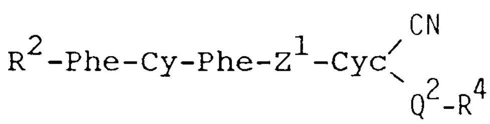

I'mf

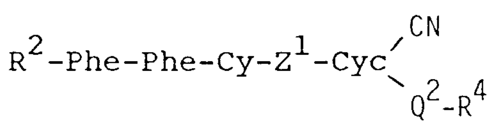

I'mg

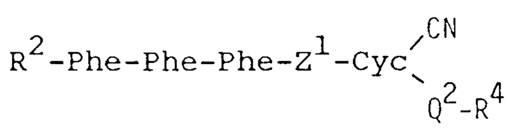

I'mh

Die bevorzugten Verbindungen der Formel I'n umfassen solche der Teilformeln I'na bis I'nh:

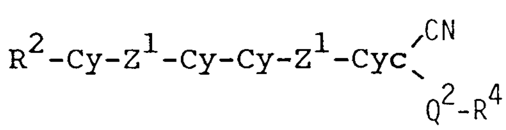

I'na

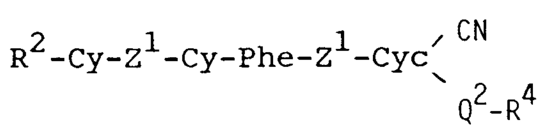

I'nb

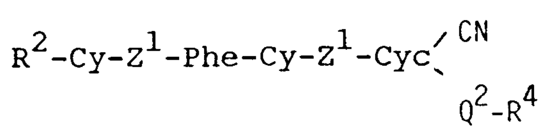

I'nc

$$R^2\text{-Phe-}Z^1\text{-Cy-Cy-}Z^1\text{-Cyc}\begin{matrix}\diagup CN\\ \diagdown Q^2\text{-}R^4\end{matrix} \qquad \text{I'nd}$$

$$R^2\text{-Cy-}Z^1\text{-Phe-Phe-}Z^1\text{-Cyc}\begin{matrix}\diagup CN\\ \diagdown Q^2\text{-}R^4\end{matrix} \qquad \text{I'ne}$$

$$R^2\text{-Phe-}Z^1\text{-Cy-Phe-}Z^1\text{-Cyc}\begin{matrix}\diagup CN\\ \diagdown Q^2\text{-}R^4\end{matrix} \qquad \text{I'nf}$$

$$R^2\text{-Phe-}Z^1\text{-Phe-Cy-}Z^1\text{-Cyc}\begin{matrix}\diagup CN\\ \diagdown Q^2\text{-}R^4\end{matrix} \qquad \text{I'ng}$$

$$R^2\text{-Phe-}Z^1\text{-Phe-Phe-}Z^1\text{-Cyc}\begin{matrix}\diagup CN\\ \diagdown Q^2\text{-}R^4\end{matrix} \qquad \text{I'nh}$$

$Q^2$ bedeutet Alkylen mit 3 bis 18 C-Atomen, vorzugsweise mit 4 bis 11, insbesondere bevorzugt mit 6 bis 8 C-Atomen, demnach bevorzugt Butylen, Pentylen, Hexylen, Heptylen oder Octylen, worin auch eine oder zwei nicht benachbarte $CH_2$-Gruppen ersetzt sein können, vorzugsweise durch -O-, -CO-, -O-CO-, -CO-O- oder -CRR'-T-.

Für $-Q^2-R^4-$ sind folgende Gruppierungen a)-k) ganz besonders bevorzugt:

a) $-(CH_2)_{11}-O-CO-C(CH_3)=CH_2$
b) $-(CH_2)_{11}-OH$
c) $-(CH_2)_9-CH=CH_2$
d) $-O-(CH_2)_6-O-CO-C(CH_3)=CH_2$
e) $-(CH_2)_4-CH=CH_2$
f) $-(CH_2)_6-OH$
g) $-O-(CH_2)_{11}-OH$
h) $-O-(CH_2)_{11}-O-CO-C(CH_3)=CH_2$
i) $-(CH_2)_4-OH$
j) $-(CH_2)_4-O-CO-C(CH_3)=CH_2$
k)

$$-CH_2-\underset{\underset{\displaystyle CH_3}{|}}{CH}-O-(CH_2)_9-CH=CH_2$$

R' bedeutet Halogen, also Fluor, Chlor oder Brom, oder CN, vorzugsweise F, Cl oder CN.

T bedeutet bevorzugt -CO-O- oder eine Einfachbindung und R ist vorzugsweise H oder Methyl, ferner auch Ethyl, Propyl, Butyl, Pentyl oder Hexyl.

Bevorzugt sind für $Q^2$ auch verzweigte Alkylengruppen, mit vorzugsweise 4 bis 8 C-Atomen, demnach bevorzugt 1-Methylpropylen, Isobutylen, 2-Methylbutylen, 3-Methylbutylen, 2-Methylpentylen oder 2-Methylhexylen bedeuten. Darin können ebenfalls wieder $CH_2$-Gruppen durch die angegebenen bevorzugten Gruppen ersetzt sein.

Falls $Q^2$ eine Alkylen- oder Alkylenoxygruppe und $R^4$ eine unsubstituierte Vinylgruppe darstellt, so weist $Q^2$ mindestens 4 C-Atome auf und hat vorzugsweise 6-10 C-Atome.

$Z^1$ bedeutet bevorzugt -CO-O-, $-CH_2-CH_2-$ oder eine Einfachbindung, ferner ist die Gruppe -CRR'-T- bevorzugt mit den angegebenen bevorzugten Bedeutungen für R, R' und R.

$R^2$ bedeutet Vorzugsweise Alkyl, Alkoxy oder Oxaalkyl.

Ferner sind dann Verbindungen der Formel I' bevorzugt, in denen $R^2$ eine Alkylgruppe ist, worin eine oder mehrere $CH_2$-Gruppen ersetzt sind, vorzugsweise durch eine -CO-O-, -OC-O-, -CRR'-T-, oder -CH = CH-Gruppe.

R, R' und T haben dabei die angegebenen bevorzugten Bedeutungen.

Falls $R^2$ ein Alkylrest oder Alkoxyrest bedeutet, so kann dieser geradkettig oder verzweigt sein. Vorzugsweise ist er geradkettig, hat 2, 3, 4, 5, 6, 7 oder 8 C-Atome und bedeutet demnach bevorzugt Ethyl, Propyl, Butyl, Pentyl, Hexyl, Heptyl, Octyl, Ethoxy, Propoxy, Butoxy, Pentoxy, Kexoxy, Heptoxy oder Octoxy, ferner Methyl, Nonyl, Decyl, Undecyl, Dodecyl, Tridecyl, Tetradecyl, Pentadecyl, Methoxy, Nonoxy, Decoxy, Undexocy, Dodecoxy, Tridecoxy oder Tetradecoxy.

Oxaalkyl bedeutet vorzugsweise geradkettiges 2-Oxapropyl (= Methoxymethyl), 2- (= Ethoxymethyl) oder 3-Oxabutyl (= 2-Methoxyethyl), 2-, 3- oder 4-Oxapentyl, 2-, 3-, 4-oder 5-Oxahexyl, 2-, 3-, 4-, 5- oder 6-Oxaheptyl, 2-, 3-, 4-, 5-, 6- oder 7-Oxaoctyl, 2-, 3-, 4-, 5-, 6-, 7- oder 8-Oxanonyl, 2-, 3-, 4-, 5-, 6-, 7-, 8- oder 9-Oxadecyl.

Falls $R^2$ ein Alkylrest bedeutet, in dem eine $CH_2$-Gruppe durch -CH = CH- ersetzt ist, so kann dieser geradkettig oder verzweigt sein. Vorzugsweise ist er geradkettig und hat 2 bis 10 C-Atome. Er bedeutet demnach besonders Vinyl, Prop-1- oder Prop-2-enyl, But-1-, 2- oder But-3-enyl, Pent-1-, 2-, 3- oder Pent-4-enyl, Hex-1-, 2-, 3-, 4- oder Hex-5-enyl, Hept-1-, 2-, 3-, 4-, 5- oder Hept-6-enyl, Oct-1-, 2-, 3-, 4-, 5-, 6- oder Oct-7-enyl, Non-1-, 2-, 3-, 4-, 5-, 6-, 7- oder Non-8-enyl, Dec-1-, 2-, 3-, 4-, 5-, 6-, 7-, 8- oder Dec-9-enyl.

Verbindungen der Formeln I' mit verzweigter Flügelgruppe $R^2$ können gelegentlich wegen Verminderung der Neigung zu Kristallisation als Comonomere von Bedeutung sein, insbesondere aber als chirale Bestandteile von Polymeren, wenn sie optisch aktiv sind. Man erhält so mit diesen Comonomeren cholesterische Phasen, die als thermochrome Folien verwendet werden können, oder auch Polymere mit getilteten smektischen Phasen.

Verzweigte Gruppen dieser Art enthalten in der Regel nicht mehr als eine Kettenverzweigung. Bevorzugte verzweigte Reste $R^2$ oder $R^3$ sind Isopropyl, 2-Butyl (= 1-Methylpropyl), Isobutyl (= 2-Methylpropyl), 2-Methylbutyl, Isopentyl (= 3-Methylbutyl), 2-Methylpentyl, 3-Methylpentyl, 2-Ethylhexyl, 2-Propylpentyl, 2-Octyl, Isopropoxy, 2-Methylpropoxy, 2-Methylbutoxy, 3-Methylbutoxy, 2-Methylpentoxy, 3-Methylpentoxy, 2-Ethylhexoxy, 1-Methylhexoxy, 2-Octyloxy, 2-Oxa-3-methylbutyl, 3-Oxa-4-methylpentyl, 4-Methylhexyl, 2-Nonyl, 2-Decyl, 2-Dodecyl, 6-Methyloctoxy, 6-Methyl-octanoyloxy, 5-Methylheptyloxycarbonyl, 2-Methylbutyryloxy, 3-Methylvaleryloxy, 4-Methylhexanoyloxy, 2-Chlorpropionyloxy, 2-Chlor-3-methylbutyryloxy, 2-Chlor-4-methylvaleryloxy, 2-Chlor-3-methylvaleryloxy, 2-Methyl-3-oxapentyl, 2-Methyl-3-oxahexyl.

Die Formel I' umfaßt sowohl die Racemate dieser Verbindungen als auch die optischen Antipoden sowie deren Gemische.

Unter den Verbindungen der Formel I' und allen ihren Teilformeln sind diejenigen bevorzugt, in denen mindestens einer der darin enthaltenen Reste eine der angegebenen bevorzugten Bedeutungen hat.

Eine kleinere Gruppe von besonders bevorzugten Verbindungen der Formel I' sind die folgenden Verbindungen der Formeln I'1 bis I'15:

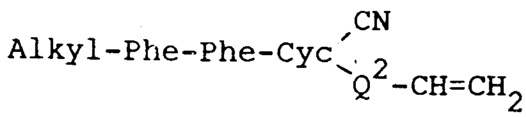

I'1

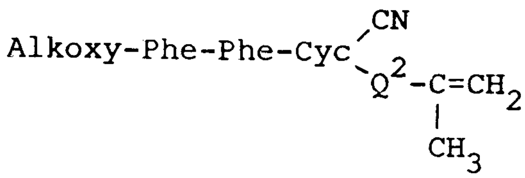

I'2

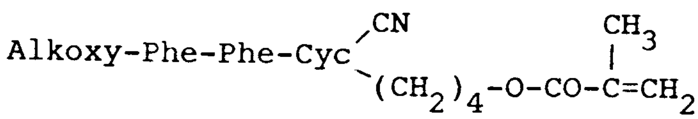

I'3

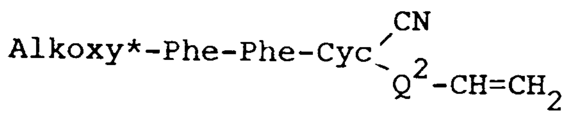

I'4

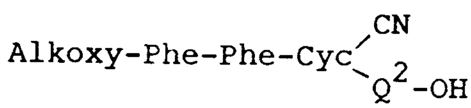

I'5

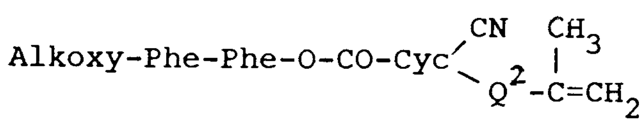

I'7

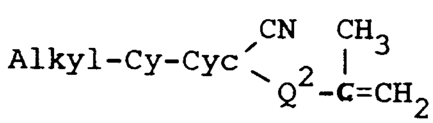

I'10

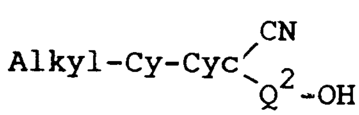

I'11

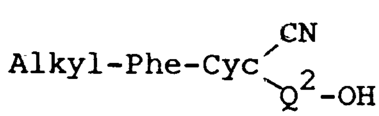

I'12

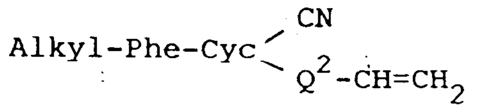

I'13

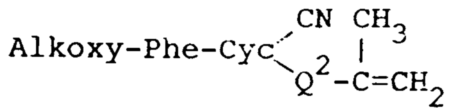

I'14

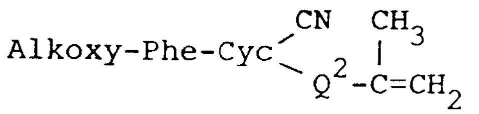

I'15

Darin bedeutet Alkyl* bzw. Alkoxy* einen optisch aktiven Alkyl- bzw. Alkoxyrest.

Gegenstand der Erfindung ist ferner die Verwendung der Verbindungen der Formel I als Vorstufen für die Herstellung von Polymeren, die flüssigkristalline Phasen aufweisen, vorzugsweise von solchen Polymerphasen charakterisiert in Anspruch 1.

Die erfindungsgemäßen Polymermaterialien können aus den Verbindungen der Formeln I' , XIaa oder XIcc auch durch Copolymerisation mit weiteren olefinisch ungesättigten Monomeren hergestellt werden. Als Comonomere eignen sich beispielsweise $C_1$-$C_{20}$-Alkylester der Acryl- und/oder der Methacrylsäure, STyrol, $\alpha$-Methylstyrol, 4-Methylstyrol, 4-Methylstyrol, Acrylnitril, Methycrylnitril sowie Methylenmalonester.

Sofern $R^4$ in Formel I' eine Vinylgruppe bedeutet, erfolgt die Polymerisation in an sich bekannter Weise durch Einwirkung von Strahlungs-, Wärme- oder elektrischer Energie sowie durch Einwirkung radikalischer oder ionischer Katalysatoren wie z. B. beschrieben in Ocian, Principles of Polymerization, McGraw-Hill, New York, oder die Polymerisation erfolgt als Gruppentransferpolymerisation mit Silylketenacetalen als Initiator und Lewis-Basen als Co-Initiator (z.B. beschrieben von O.W. Webster et al., J. Am. Chem. Soc. 1983, 105, 5706-5708).

Als Strahlungsenergie eignen sich UV-, Laser-, Röntgen- und radioaktive Strahlen. Elektrische Energie kann beispielsweise durch Elektrolyseverfahren erzeugt werden. Beispiele für radikalische Katalysatoren sind Kaliumpersulfat, Dibenzoylperoxid, Azo-biisobutyronitril, Di-tertbutyl-peroxid und Cyclohexanonperoxid. Ionische Katalysatoren sind alkali-organische Verbindungen wie Phenyllithium und Naphthalinnatrium oder Lewissäuren wie $BF_3$, $AlCl_3$, $SnCl_4$ und $TiCl_4$ oder Metallkomplexe in Form von Aluminium- oder Titanverbindungen. Die Monomeren können in Lösung, Suspension, Emulsion oder Substanz polymerisiert werden.

Sofern $R^4$ eine Hydroxy- oder Carboxygruppe bedeutet, können die Verbindungen der Formel I' entweder polymerisiert bzw. polykondensiert werden oder aber auch auf ein polymeres Rückgrat aufgepfropft werden.

Besonders bevorzugt bedeutet hierbei $R^4$ OH oder COOH. Die Aufpfropfungsreaktion kann nach an sich bekannten Methoden, wie z.B. Veresterung, Amidierung, Umesterung, Umamidierung, Acetalisierung oder Veretherung erfolgen, die in der Literatur beschrieben sind [z.B. in Standardwerken wie Houben-Weyl, Methoden der Org. Chemie, Georg-Thieme-Verlag, Stuttgart oder C.M. Paleos et al., J. Polym. Sci. Polym. Chem. 19 (1981, 1427].

Eine bevorzugte Aufpfropfungsreaktion besteht in der Umsetzung von Verbindungen der Formel I', XIaa oder XIcc mit Organopolysiloxanen. Hierzu werden, wie z.B. in EP-PS 0 060 335 beschrieben, lineare oder cyclische Organowasserstoffpolysiloxane mit ehtylenisch ungesättigten Verbindungen der Formel I' bzw. XIaa oder XIcc äquimolaren Mengen, bezogen auf die Menge Siloxan-Wasserstoff, in Gegenwart eines die Addition von Silan-Wasserstoff an aliphatische Mehrfachbindungen fördernden Katalysators umgesetzt.

Als polymeres Rückgrat kommen prinzipiell alle Polymeren in Frage, deren Ketten eine gewisse Flexibilität aufweisen. Es kann sich hierbei um lineare, verzweigte oder cyclische Polymerketten handeln. Der Polymerisationsgrad beträgt normalerweise mindestens 10, vorzugsweise 20-100. Es kommen jedoch auch Oligomere, insbesondere cyclische Oligomere, mit 3 bis 15, insbesondere mit 4 bis 7 Monomereinheiten, in Frage.

Vorzugsweise werden Polymere mit C-C-Hauptketten, insbesondere Polyacrylate, -methacrylate, -$\alpha$-halogenacrylate, -$\alpha$-cyanacrylate, -acrylamide, -acrylnitrile oder -methylenmalonate eingesetzt. Weiterhin bevorzugt sind auch Polymere mit Heteroatomen in der Hauptkette, beispielsweise Polyether, -ester, -amide, -imide oder -urethane oder insbesondere Polysiloxane.

Die erfindungsgemäßen flüssigkristallinen Phasen aufweisende Polymerzusammensetzungen weisen vorzugsweise 20-100 % an mesogenen Gruppen mit einem querpolarisierenden Strukturelement entsprechend der Formeln I bis X auf. Insbesonders bevorzugt ist ein Gehalt an 50-100 %.

Die Verbindungen der Formel I' können nach an sich bekannten Methoden hergestellt werden, wie sie in der Literatur (z. B. in den Standardwerken wie Houben-Weyl, Methoden der organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

So können Verbindungen der Formel I' mit den reaktionsfähigen Gruppen $R^4$ dadurch erhalten werden, daß man z. B. in Verbindungen, die ansonsten der Formel I' entsprechen, eine Alkylgruppe zur Vinylgruppe dehydriert, oder eine Carboxylgruppe zur Hydroxygruppe reduziert,

Diese Herstellungsverfahren sind bekannte Methoden, die in der Literatur (z. B. in Standardwerken wie Houben-Weyl, Methoden der organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Verbindungen der Formel I' können z. B. hergestellt werden nach folgenden Methoden:

An Verbindungen der Formel II'

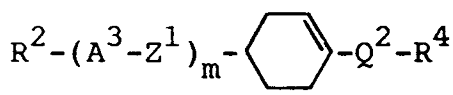 II'

worin $R^2$, $A^3$, $Z^1$, m, $Q^2$ und $R^4$ die angegebene Bedeutung haben, können Verbindungen der Formel HX, mit X F, Cl, Br oder CN, angelagert werden. Dabei kann man von bekannten Reaktionsbedingungen und Varianten für Additionsreaktionen gebrauch machen.

Man kann auch Verbindungen, die sonst der Formel I' entsprechen, aber an Stelle von $R^4$ eine gesättigte und/oder nicht reaktionsfähige Gruppe enthalten, durch Oxidation, Reduktions- oder Austauschreaktionen in die Verbindungen der Formel I' mit einer reaktionsfähigen Gruppe $R^4$ überführen.

Ferner kann man Verbindungen der Formeln III' oder IV'

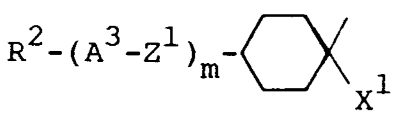 III'

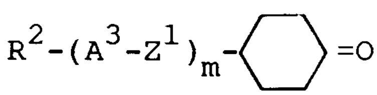 IV'

worin $R^2$, $A^3$, $Z^1$ und die angegebenen Bedeutungen haben und $X^1$ H oder OH bedeutet, durch Umsetzung mit Reduktions- oder Alkylierungsmitteln, durch Umsetzung mit Alkylhalogeniden oder -sulfonaten und gegebenenfalls anschließender Oxidation, oder mit Carbonsäuren oder den reaktionsfähigen Derivaten von Carbonsäuren oder Kohlensäuren zu Verbindungen der Formel I' umsetzen.

Durch Umsetzung von Verbindungen der Formel VI'

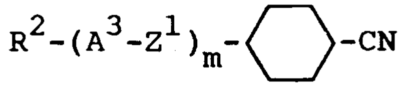 VI'

mit einer Verbindung der Formel VII'

$X^2$-$Q^2$-$R^4$ VII'

wobei $R^2$, $A^3$, $Z^1$, m, $Q^2$ und $R^4$ die angegebenen Bedeutungen haben und $X^2$ Cl, Br, J, OH oder eine reaktionsfähig veresterte OH-Gruppe bedeutet, erhält man Nitrile der Formel I'.

Die Verbindungen der Formel I' können also auch hergestellt werden, indem man eine Verbindung, die sonst der Formel I entspricht, aber an Stelle von H-Atomen eine oder mehrere reduzierbare Gruppen und/oder C-C-Bindungen enthält, reduziert.

Als reduzierbare Gruppen kommen vorzugsweise -CH=CH-Gruppen in Betracht, ferner z.B. freie oder veresterte Hydroxygruppen, aromatisch gebundene Halogenatome oder Carbonylgruppen. Bevorzugte Ausgangsstoffe für die Reduktion entsprechen der Formel I', können aber an Stelle einer $-CH_2CH_2$-Gruppe eine -CH=CH-Gruppe und/oder an Stelle einer $-CH_2$-Gruppe eine -CO-Gruppe und/oder an Stelle eines H-Atoms eine freie oder eine funktionell (z.B. in Form ihres p-Toluolsulfonats) abgewandelte OH-Gruppe enthalten.

Die Reduktion kann z.B. erfolgen durch katalytische Hydrierung bei Temperaturen zwischen etwa 0° und etwa 200° sowie Drucken zwischen etwa 1 und 200 bar in einem inerten Lösungsmittel, z.B. einem Alkohol wie Methanol, Ethanol oder Isopropanol, einem Ether wie Tetrahydrofuran (THF) oder Dioxan, einem Ester wie Ethylacetat, einer Carbonsäure wie Essigsäure oder einem Kohlenwasserstoff wie Cyclohexan. Als Katalysatoren eignen sich zweckmäßig Edelmetalle wie Pt oder Pd, die in Form von Oxiden (z.B. $PtO_2$, PdO), auf einem Träger (z.B. Pd auf Kohle, Calciumcarbonat oder Strontiumcarbonat) oder in feinverteilter Form eingesetzt werden können.

Ketone können auch nach den Methoden von Clemmensen (mit Zink, amalgamiertem Zink oder Zinn und Salzsäure, zweckmäßig in wäßrig-alkoholischer Lösung oder in heterogener Phase mit Wasser/Toluol bei Temperaturen zwischen etwa 80 und 120°) oder Wolff-Kishner (mit Hydrazin, zweckmäßig in Gegenwart von Alkali wie KOH oder NaOH in einem hochsiedenden Lösungsmittel wie Diethylenglykol oder Triethylenglykol bei Temperaturen zwischen etwa 100 und 200°) zu den entsprechenden Verbindungen der Formel I', die Alkylgruppen und/oder $-CH_2CH_2$-Brücken enthalten, reduziert werden.

Weiterhin sind Reduktionen mit komplexen Hydriden möglich. Beispielsweise können Arylsulfonyloxygruppen mit $LiAlH_4$ reduktiv entfernt werden, insbesondere p-Toluolsulfonyloxymethylgruppen zu Methylgruppen reduziert werden, zweckmäßig in einem inerten Lösungsmittel wie Diethylether oder THF bei Temperaturen zwischen etwa 0 und 100°. Doppelbindungen können (auch in Gegenwart von CN-Gruppen!) mit $NaBH_4$ oder Tributylzinnhydrid in Methanol hydriert werden.

Ester der Formel I' können auch durch Veresterung entsprechender Carbonsäuren (oder ihrer reaktionsfähigen Derivate) mit Alkoholen bzw. Phenolen (oder ihren reaktionsfähigen Derivaten) erhalten werden.

Als reaktionsfähige Derivate der genannten Carbonsäuren eignen sich insbesondere die Säurehalogenide, vor allem die Chloride und Bromide, ferner die Anhydride, z.B. auch gemischte Anhydride, Azide oder Ester, insbesondere Alkylester mit 1 - 4 C-Atomen in der Alkylgruppe.

Als reaktionsfähige Derivate der genannten Alkohole bzw. Phenole kommen insbesondere die entsprechenden Metallalkoholate bzw. Phenolate, vorzugsweise eines Alkalimetalls wie Na oder K, in Betracht.

Die Veresterung wird vorteilhaft in Gegenwart eines inerten Lösungsmittels durchgeführt. Gut geeignet sind insbesondere Ether wie Diethylether, Di-n-butylether, THF, Dioxan oder Anisol, Ketone wie Aceton, Butanon oder Cyclohexanon, Amide wie DMF oder Phosphorsäurehexamethyltriamid, Kohlenwasserstoffe wie Benzol, Toluol oder Xylol, Halogenkohlenwasserstoffe wie Tetrachlorkohlenstoff oder Tetrachlorethylen und Sulfoxide wie Dimethylsulfoxid oder Sulfolan. Mit Wasser nicht mischbare Lösungsmittel können gleichzeitig vorteilhaft zum azeotropen Abdestillieren des bei der Veresterung gebildeten Wassers verwendet werden. Gelegentlich kann auch ein Überschuß einer organischen Base, z.B. Pyridin, Chinolin oder Triethylamin als Lösungsmittel für die Veresterung angewandt werden. Die Veresterung kann auch in Abwesenheit eines Lösungsmittels, z.B. durch einfaches Erhitzen der Komponenten in Gegenwart von Natriumacetat, durchgeführt werden. Die Reaktionstemperatur liegt gewöhnlich zwischen -50° und +250°, vorzugsweise zwischen -20° und +80°. Bei diesen Temperaturen sind die Veresterungsreaktionen in der Regel nach 15 Minuten bis 48 Stunden beendet.

Im einzelnen hängen die Reaktionsbedingungen für die Veresterung weitgehend von der Natur der verwendeten Ausgangsstoffe ab. So wird eine freie Carbonsäure mit einem freien Alkohol oder Phenol in der Regel in Gegenwart einer starken Säure, beispielsweise einer Mineralsäure wie Salzsäure oder Schwefelsäure, umgesetzt. Möglich ist auch eine Veresterung in Gegenwart von Dicyclohexylcarbodiimid, evtl. unter Zusatz einer Base wie z.B. 4-Dimethylaminopyridin. Eine bevorzugte Reaktionsweise ist die Umsetzung eines Säureanhydrids oder insbesondere eines Säurechlorids mit einem Alkohol, vorzugsweise in einem basischen Milieu, wobei als Basen insbesondere Alkalimetallhydroxide wie Natrium- oder Kaliumhydroxid, Alkalimetallcarbonate bzw. -hydrogencarbonate wie Natriumcarbonat, Kaliumcarbonat oder Kaliumhydrogencarbonat, Alkalimetallacetate wie Natrium- oder Kaliumacetat, Erdalkalimetallhydroxide wie Calciumhydroxid oder organische Basen wie Triethylamin, Pyridin, Lutidin, Kollidin oder Chinolin von Bedeutung sind. Eine weitere bevorzugte Ausführungsform der Veresterung besteht darin, daß man den Alkohol bzw. das Phenol zunächst in das Natrium- oder Kaliumalkoholat bzw. -phenolat überführt, z.B. durch Behandlung mit ethanolischer Natron- oder Kalilauge, dieses isoliert und zusammen mit Natriumhydrogencarbonat oder Kaliumcarbonat unter Rühren in Aceton oder Diethylether suspendiert und diese Suspension mit einer Lösung des Säurechlorids oder Anhydrids in Diethylether, Aceton oder DMF versetzt, zweckmäßig bei Temperaturen zwischen etwa -25° und +20°.

Zur Herstellung von Nitrilen der Formel I' können entsprechende Säureamide, z.B. solche in denen an Stelle des Restes X eine $CONH_2$-Gruppe steht, dehydratisiert werden. Die Amide sind z.B. aus entsprechenden Estern oder Säurehalogeniden durch Umsetzung mit Ammoniak erhältlich. Als wasserabspaltende Mittel eignen sich beispielsweise anorganische Säurechloride wie $SOCl_2$, $PCl_3$, $PCl_5$, $POCl_3$, $SO_2Cl_2$, $COCl_2$, ferner $P_2O_5$, $P_2S_5$, $AlCl_3$ (z.B. als Doppelverbindungen mit NaCl), aromatische Sulfonsäuren und Sulfonsäurehalogenide. Man kann dabei in Gegenwart oder Abwesenheit eines inerten Lösungsmittels bei Temperaturen zwischen etwa 0° und 150° arbeiten; als Lösungsmittel kommen z.B. Basen wie Pyridin oder Triethylamin, aromatische Kohlenwasserstoffe wie Benzol, Toluol, oder Xylol oder Amide wie DMF in Betracht.

Zur Herstellung der vorstehend genannten Nitrile der Formel I' kann man auch entsprechende Säurehalogenide, vorzugsweise die Chloride, mit Sulfamid umsetzen, zweckmäßig in einem inerten Lösungsmittel wie Tetramethylensulfon bei Temperaturen zwischen etwa 80° und 150°, vorzugsweise bei 120°. Nach üblicher Aufarbeitung kann man direkt die Nitrile isolieren.

Ether der Formel I' sind durch Veretherung entsprechender Hydroxyverbindungen, vorzugsweise entsprechender Phenole, erhältlich, wobei die Hydroxyverbindung zweckmäßig zunächst in ein entsprechendes Metallderivat, z.B. durch Behandeln mit NaH, $NaNH_2$, NaOH, KOH, $Na_2CO_3$ oder $K_2CO_3$ in das entsprechende Alkalimetallalkoholat oder Alkalimetallphenolat übergeführt wird. Dieses kann dann mit dem entsprechenden Alkylhalogenid, -sulfonat oder Dialkylsulfat umgesetzt werden, zweckmäßig in einem inerten Lösungsmittel wie Aceton, 1,2-Dimethoxyethan, DMF oder Dimethylsulfoxid oder auch einem Überschuß an wäßriger oder wäßrig-alkoholischer NaOH oder KOH bei Temperaturen zwischen etwa 20° und 100°.

Cyclohexanonderivate der Formel I' sind weiterhin durch säurekatalysierte Umlagerungen der entsprechenden Epoxide nach literaturbekannten Verfahren, z.B. durch Behandeln mit $BF_3$-Etherat, zugänglich. Die Epoxide sind durch Epoxidierung der entsprechenden Cyclohexanderivate nach Standardverfahren erhältlich.

Zur Herstellung von Nitrilen der Formel I' können auch entsprechende Chlor- oder Bromverbindungen der Formel I' mit einem Cyanid umgesetzt werden, zweckmäßig mit einem Metallcyanid wie NaCN, KCN oder $Cu_2(CN)_2$, z.B. in Gegenwart von Pyridin in einem inerten Lösungsmittel wie DMF oder N-Methylpyrrolidon bei Temperaturen zwischen 20° und 200°.

Die niedermolekularen Verbindungen der Formel I' bzw. XIaa oder XIcc weisen teilweise breite Mesophasenbereiche auf. Verbindungen der Formel I' oder XIaa oder XIcc, die keine Mesophasen aufweisen, sind jedoch auch zur Herstellung der erfindungsgemäßen Polymermaterialien geeignet.

Die Herstellung von Homo- oder Copolymeren aus den polymerisationsfähigen Verbindungen der Formel I',XIaa oder XIcc oder deren polymerisationsfähigen Derivaten erfolgt vorzugsweise durch radikalische Polymerisation. Die Reaktion wird beispielsweise durch UV-Bestrahlung oder Radikalbildner gestartet. Die Monomeren können in Lösung oder in Substanz polymerisiert werden.

Erfindungsgemäße flüssigkristalline Phasen aufweisende Copolymermaterialien werden durch Copolymerisation von polymerisationsfähigen Verbindungen der Formel I',XIaa oder XIcc oder deren polymerisationsfähigen Derivaten mit Monomeren erhalten, die keine mesogenen Reste tragen, die andere mesogene Reste tragen, die chirale Reste tragen oder die Farbstoffreste (DE-OS 32 11 400) tragen.

Die Copolymerisation mit solchen Monomeren führt, ausgehend von einer Monomerenmischung mit der Konzentration $X_1$, nur dann zu einem Copolymerisat mit dem einbauverhältnis entsprechend der Monomerkonzentration $X_1$, wenn die Copolymerisationsparameter der Monomerkomponenten von vergleichbarer Größenordnung sind. Das ist besonders dann von Bedeutung, wenn problemlos, z. B. ohne Berücksichtigung der Reaktionskinetik, ein Copolymer bestimmter Zusammensetzung hergestellt werden soll. Deshalb wählt man vorzugsweise Monomerkomponenten, die vergleichbare Copolymerisationsparameter aufweisen, etwa Acrylsäure- oder Methacrylsäurealkylester, die sich in erster Linie durch den Substituenten der Alkylkette unterscheiden.

Die Copolymerisation mit Monomeren, die keinen mesogenen Rest tragen, führt im allgemeinen zu einer Erniedrigung der Glastemperatur und des Klärpunktes. Durch geeignete Auswahl des Spacers ist es oftmals möglich, den Mesophasenbereich in den für den jeweiligen Anwendungszweck geeigneten Temperaturbereich zu bringen.

Als Monomere mit chiralem Rest können prinzipiell alle derartigen Verbindungen mit asymmetrischen C-Atomen verwendet werden. Das asymmetrische C-Atom kann dabei entweder in der Flügelgruppe, zwischen zwei Ringen oder in der Spacer-Gruppe des mesogenen Restes sitzen.

Schließlich ergeben sich zahlreiche weitere Variationsmöglichkeiten wegen des Umstandes, daß die erfindungsgemäßen Verbindungen flüssigkristalline Eigenschaften mit typischen Polymereigenschaften, wie Fähigkeit zur Schicht-, Folien- und Faserbildung, leichte Verformbarkeit usw., vereinigen. Diese Eigenschaften können in an sich bekannter Weise durch Copolymerisation oder Vermischen mit weiteren Komponenten, durch Variation der Molekulargewichte, durch Zusätze der verschiedensten anorganischen oder organischen Additive und Metalle, durch Vernetzen, z.B. zu einem Elastomer, und durch viele weitere, dem Polymerfachmann geläufige Behandlungen modifiziert werden.

Die erfindungsgemäßen Polymermaterialien lassen sich als Ausgangsmaterial zur Herstellung von organischen Gläsern mit in breiten Bereichen modifizierbaren anisotropen Eigenschaften verwenden.

Derartige Anwendungen ergeben sich beispielsweise auf dem Sektor von Licht- und Sonnenkollektoren oder bei organischen phototropen Gläsern. Ein wichtiges Anwendungsfeld eröffnet sich ferner auf dem Gebiet der optischen Speicher.

Weitere Anwendungsmöglichkeiten erschließen sich auf dem Gebiet der magnetischen Speicher. Insbesondere eignen sich die erfindungsgemäßen Materialien selbst als Materialien mit nichtlinear optischen Eigenschaften oder auch als Matrix für Substanzen mit nicht linear optischen Eigenschaften zur Herstellung von nichtlinear optischen Bauelementen.

Zur Erläuterung der Erfindung dienen folgende Beispiele, wobei K = kristalliner Zustand, S = smektische Phase (der Index bezeichnet den Phasentyp), N = nematischer Zustand, Ch = cholesterische Phase, I = isotrope Phase bedeutet. Die zwischen zwei Symbolen stehende Zahl gibt die Umwandlungstemperatur in Grad Celsius an. Fp. bedeutet Schmelzpunkt und Kp. bedeutet Klärpunkt, G = Glaszustand.

Beispiel 1

Zu einem Gemisch aus 13,1 g trans,trans-4'-Pentylbicyclohexyl-4-carbonitril, 8,8 g 3-Brompropanol und 75 ml THF gibt man bei -78° unter $N_2$ eine Lösung von Lithiumdiisopropylamin (hergestellt aus 50 ml THF, 13,2 g Diisopropylamin und 75 ml 1,6 m Butyllithiumlösung in Hexan) und rührt 1 Stunde.
Man erwärmt langsam auf Raumtemperatur, versetzt mit Wasser und arbeitet die organische Phase auf.
Man erhält 1-γ-Hydroxylpropyl-c-4-(trans-4-pentylcyclohexyl)-cyclohexan-1-r-carbonitril mit K 63° $S_A$ 110° I durch Chromatographie an Kieselgel und Umkristallisation aus Ethanol.

Analog werden hergestellt aus den entsprechenden Ausgangsverbindungen:
1-γ-Hydroxypropyl-c-4-(trans-4-propylcyclohexyl)-cyclohexan-1-r-carbonitril
1-γ-Hydroxypropyl-c-4-(trans-4-butylcyclohexyl)-cyclohexan-1-r-carbonitril
1-γ-Hydroxypropyl-c-4-(trans-4-hexylcyclohexyl)-cyclohexan-1-r-carbonitril
1-γ-Hydroxypropyl-c-4-(trans-4-heptylcyclohexyl)-cyclohexan-1-r-carbonitril
1-γ-Hydroxypropyl-c-4-(trans-4-octylcyclohexyl)-cyclohexan-1-r-carbonitril
1-γ-Hydroxypropyl-c-4-(trans-4-nonylcyclohexyl)-cyclohexan-1-r-carbonitril
1-δ-Hydroxybutyl-c-4-(trans-4-propylcyclohexyl)-cyclohexan-1-r-carbonitril
1-δ-Hydroxybutyl-c-4-(trans-4-butylcyclohexyl)-cyclohexan-1-r-carbonitril
1-δ-Hydroxybutyl-c-4-(trans-4-pentylcyclohexyl)-cyclohexan-1-r-carbonitril
1-δ-Hydroxybutyl-c-4-(trans-4-hexylcyclohexyl)-cyclohexan-1-r-carbonitril
1-δ-Hydroxybutyl-c-4-(trans-4-heptylcyclohexyl)-cyclohexan-1-r-carbonitril
1-δ-Hydroxybutyl-c-4-(trans-4-octylcyclohexyl)-cyclohexan-1-r-carbonitril
1-δ-Hydroxybutyl-c-4-(trans-4-nonylcyclohexyl)-cyclohexan-1-r-carbonitril
1-ϵ-Hydroxypentyl-c-4-(trans-4-propylcyclohexyl)-cyclohexan-1-r-carbonitril
1-ϵ-Hydroxypentyl-c-4-(trans-4-butylcyclohexyl)-cyclohexan-1-r-carbonitril
1-ϵ-Hydroxypentyl-c-4-(trans-4-pentylcyclohexyl)-cyclohexan-1-r-carbonitril
1-ϵ-Hydroxypentyl-c-4-(trans-4-hexylcyclohexyl)-cyclohexan-1-r-carbonitril
1-ϵ-Hydroxypentyl-c-4-(trans-4-heptylcyclohexyl)-cyclohexan-1-r-carbonitril
1-ϵ-Hydroxypentyl-c-4-(trans-4-octylcyclohexyl)-cyclohexan-1-r-carbonitril
1-ϵ-Hydroxypentyl-c-4-(trans-4-nonylcyclohexyl)-cyclohexan-1-r-carbonitril
1-γ-Hydroxypropyl-c-4-(4-propylphenyl)-cyclohexan-1-r-carbonitril
1-γ-Hydroxypropyl-c-4-(4-butylphenyl)-cyclohexan-1-r-carbonitril
1-γ-Hydroxypropyl-c-4-(4-pentylphenyl)-cyclohexan-1-r-carbonitril
1-γ-Hydroxypropyl-c-4-(4-hexylphenyl)-cyclohexan-1-r-carbonitril
1-γ-Hydroxypropyl-c-4-(4-heptylphenyl)-cyclohexan-1-r-carbonitril
1-γ-Hydroxypropyl-c-4-(4-octylphenyl)-cyclohexan-1-r-carbonitril
1-γ-Hydroxypropyl-c-4-(4-nonylphenyl)-cyclohexan-1-r-carbonitril
1-γ-Hydroxypropyl-c-4-(4-decylphenyl)-cyclohexan-1-r-carbonitril
1-γ-Hydroxypropyl-c-4-(4-ethoxyphenyl)-cyclohexan-1-r-carbonitril
1-γ-Hydroxypropyl-c-4-(4-propoxyphenyl)-cyclohexan-1-r-carbonitril
1-γ-Hydroxypropyl-c-4-(4-butoxyphenyl)-cyclohexan-1-r-carbonitril
1-γ-Hydroxypropyl-c-4-(4-pentoxyphenyl)-cyclohexan-1-r-carbonitril

1-γ-Hydroxypropyl-c-4-(4-hexoxyphenyl)-cyclohexan-1-r-carbonitril
1-δ-Hydroxybutyl-c-4-(4-propylphenyl)-cyclohexan-1-r-carbonitril
1-δ-Hydroxybutyl-c-4-(4-butylphenyl)-cyclohexan-1-r-carbonitril
1-δ-Hydroxybutyl-c-4-(4-pentylphenyl)-cyclohexan-1-r-carbonitril
1-δ-Hydroxybutyl-c-4-(4-hexylphenyl)-cyclohexan-1-r-carbonitril
1-δ-Hydroxybutyl-c-4-(4-heptylphenyl)-cyclohexan-1-r-carbonitril
1-δ-Hydroxybutyl-c-4-(4-octylphenyl)-cyclohexan-1-r-carbonitril
1-δ-Hydroxybutyl-c-4-(4-nonylphenyl)-cyclohexan-1-r-carbonitril
1-δ-Hydroxybutyl-c-4-(4-decylphenyl)-cyclohexan-1-r-carbonitril
1-δ-Hydroxybutyl-c-4-(4-ethoxyphenyl)-cyclohexan-1-r-carbonitril
1-δ-Hydroxybutyl-c-4-(4-propoxyphenyl)-cyclohexan-1-r-carbonitril
1-δ-Hydroxybutyl-c-4-(4-butoxyphenyl)-cyclohexan-1-r-carbonitril
1-δ-Hydroxybutyl-c-4-(4-pentoxyphenyl)-cyclohexan-1-r-carbonitril
1-δ-Hydroxybutyl-c-4-(4-hexoxyphenyl)-cyclohexan-1-r-carbonitril
1-ε-Hydroxypentyl-c-4-(4-propylphenyl)-cyclohexan-1-r-carbonitril
1-ε-Hydroxypentyl-c-4-(4-butylphenyl)-cyclohexan-1-r-carbonitril
1-ε-Hydroxypentyl-c-4-(4-pentylphenyl)-cyclohexan-1-r-carbonitril
1-ε-Hydroxypentyl-c-4-(4-hexylphenyl)-cyclohexan-1-r-carbonitril
1-ε-Hydroxypentyl-c-4-(4-heptylphenyl)-cyclohexan-1-r-carbonitril
1-ε-Hydroxypentyl-c-4-(4-octylphenyl)-cyclohexan-1-r-carbonitril
1-ε-Hydroxypentyl-c-4-(4-nonylphenyl)-cyclohexan-1-r-carbonitril
1-ε-Hydroxypentyl-c-4-(4-decylphenyl)-cyclohexan-1-r-carbonitril
1-ε-Hydroxypentyl-c-4-(4-ethoxyphenyl)-cyclohexan-1-r-carbonitril
1-ε-Hydroxypentyl-c-4-(4-propoxyphenyl)-cyclohexan-1-r-carbonitril
1-ε-Hydroxypentyl-c-4-(4-butoxyphenyl)-cyclohexan-1-r-carbonitril
1-ε-Hydroxypentyl-c-4-(4-pentoxyphenyl)-cyclohexan-1-r-carbonitril
1-ε-Hydroxypentyl-c-4-(4-hexoxyphenyl)-cyclohexan-1-r-carbonitril

Beispiel 2

16,9 g 1-γ-Cyanopropyl-4-c-(4-trans-pentylcyclohexyl)cyclohexan-1-r-carbonitril (beschrieben in der DE-OS 33 20 024) werden mit 10 g KOH, 20 m Wasser und 40 ml Ethanol 16 h unter Rückfluß erhitzt. Die abgekühlte Lösung wird neutralisiert und der Niederschlag abgesaugt. Nach Umkristallisation aus Ethanol erhält man 3-[1-r-Cyan-4-c-(4-transpentylcyclohexyl)cyclohexyl]-buttersäure, Fp. 154°.

Analog werden hergestellt:

3-[1-r-Cyan-4-c-(4-trans-propylcyclohexyl)cyclohexyl]-buttersäure
3-[1-r-Cyan-4-c-(4-trans-butylcyclohexyl)cyclohexyl]-buttersäure
3-[1-r-Cyan-4-c-(4-trans-hexylcyclohexyl)cyclohexyl]-buttersäure
3-[1-r-Cyan-4-c-(4-trans-heptylcyclohexyl)cyclohexyl]-buttersäure
3-[1-r-Cyan-4-c-(4-trans-octylcyclohexyl)cyclohexyl]-buttersäure
3-[1-r-Cyan-4-c-(4-trans-nonylcyclohexyl)cyclohexyl]-buttersäure
4-[1-r-Cyan-4-c-(4-trans-propylcyclohexyl)cyclohexyl]-valeriansäure
4-[1-r-Cyan-4-c-(4-trans-butylcyclohexyl)cyclohexyl]-valeriansäure
4-[1-r-Cyan-4-c-(4-trans-pentylcyclohexyl)cyclohexyl]-valeriansäure
4-[1-r-Cyan-4-c-(4-trans-hexylcyclohexyl)cyclohexyl]-valeriansäure
4-[1-r-Cyan-4-c-(4-trans-heptylcyclohexyl)cyclohexyl]-valeriansäure
4-[1-r-Cyan-4-c-(4-trans-octylcyclohexyl)cyclohexyl]-valeriansäure
4-[1-r-Cyan-4-c-(4-trans-nonylcyclohexyl)cyclohexyl]-valeriansäure
5-[1-r-Cyan-4-c-(4-trans-propylcyclohexyl)cyclohexyl]-capronsäure
5-[1-r-Cyan-4-c-(4-trans-butylcyclohexyl)cyclohexyl]-capronsäure
5-[1-r-Cyan-4-c-(4-trans-pentylcyclohexyl)cyclohexyl]-capronsäure
5-[1-r-Cyan-4-c-(4-trans-hexylcyclohexyl)cyclohexyl]-capronsäure
5-[1-r-Cyan-4-c-(4-trans-heptylcyclohexyl)cyclohexyl]-capronsäure
4-[1-r-Cyan-4-c-(4-trans-octylcyclohexyl)cyclohexyl]-capronsäure
5-[1-r-Cyan-4-c-(4-trans-nonylcyclohexyl)cyclohexyl]-capronsäure.

Beispiel 3

Zu einer Lösung von 5 g r-1-(3-Butenyl)-t-4-(r-4-pentyl-t-1-cyclohexyl)-cyclohexyl-c-1-carbonitril (herstellbar aus t-4-(r-4-Pentyl-t-1-cyclohexyl)-cyclohexyl-r-1-carbonitril und 1-Brom-3-buten in Gegenwart von Diisopropylamin und n-Butyllithium in THF) in 10 ml THF werden bei 0° 6,3 ml einer 1 M Lösung von $BH_3$ . THF in THF zugegeben und noch 1 Stunde gerührt.

Anschließend gibt man 1,6 ml $H_2O$, 2,1 ml 3 M NaOH und 2,1 ml 30%ige $H_2O_2$ zu und rührt 1 Stunde bei Raumtemperatur.

Die Mischung wird mit Ether versetzt und mit Wasser extrahiert. Die organische Phase wird aufgearbeitet und der Rückstand an einer Kieselgelsäule mit $CH_2Cl_2$/Ethylacetat (85/15) chromatographiert. Man erhält r-1-(4-Hydroxybutyl)-t-4-(r-4-pentyl-t-1-cyclohexyl)-cyclohexyl-c-1-carbonitril mit $S_A$ 100° I.

Analog werden hergestellt:

r-1-(4-Hydroxybutyl)-t-4-(r-4-ethyl-t-1-cyclohexyl)-cyclohexyl-c-1-carbonitril
r-1-(4-Hydroxybutyl)-t-4-(r-4-propyl-t-1-cyclohexyl)-cyclohexyl-c-1-carbonitril
r-1-(4-Hydroxybutyl)-t-4-(r-4-butyl-t-1-cyclohexyl)-cyclohexyl-c-1-carbonitril
r-1-(4-Hydroxybutyl)-t-4-(r-4-hexyl-t-1-cyclohexyl)-cyclohexyl-c-1-carbonitril
r-1-(4-Hydroxybutyl)-t-4-(r-4-heptyl-t-1-cyclohexyl)-cyclohexyl-c-1-carbonitril
r-1-(4-Hydroxybutyl)-t-4-(r-4-octyl-t-1-cyclohexyl)-cyclohexyl-c-1-carbonitril
r-1-(5-Hydroxypentyl)-t-4-(r-4-ethyl-t-1-cyclohexyl)-cyclohexyl-c-1-carbonitril
r-1-(5-Hydroxypentyl)-t-4-(r-4-propyl-t-1-cyclohexyl)-cyclohexyl-c-1-carbonitril
r-1-(5-Hydroxypentyl)-t-4-(r-4-butyl-t-1-cyclohexyl)-cyclohexyl-c-1-carbonitril
r-1-(5-Hydroxypentyl)-t-4-(r-4-pentyl-t-1-cyclohexyl)-cyclohexyl-c-1-carbonitril
r-1-(5-Hydroxypentyl)-t-4-(r-4-hexyl-t-1-cyclohexyl)-cyclohexyl-c-1-carbonitril
r-1-(5-Hydroxypentyl)-t-4-(r-4-heptyl-t-1-cyclohexyl)-cyclohexyl-c-1-carbonitril
r-1-(5-Hydroxypentyl)-t-4-(r-4-octyl-t-1-cyclohexyl)-cyclohexyl-c-1-carbonitril
r-1-(6-Hydroxyhexyl)-t-4-(r-4-ethyl-t-1-cyclohexyl)-cyclohexyl-c-1-carbonitril
r-1-(6-Hydroxyhexyl)-t-4-(r-4-propyl-t-1-cyclohexyl)-cyclohexyl-c-1-carbonitril
r-1-(6-Hydroxyhexyl)-t-4-(r-4-butyl-t-1-cyclohexyl)-cyclohexyl-c-1-carbonitril
r-1-(6-Hydroxyhexyl)-t-4-(r-4-pentyl-t-1-cyclohexyl)-cyclohexyl-c-1-carbonitril
r-1-(6-Hydroxyhexyl)-t-4-(r-4-hexyl-t-1-cyclohexyl)-cyclohexyl-c-1-carbonitril
r-1-(6-Hydroxyhexyl)-t-4-(r-4-heptyl-t-1-cyclohexyl)-cyclohexyl-c-1-carbonitril
r-1-(6-Hydroxyhexyl)-t-4-(r-4-octyl-t-1-cyclohexyl)-cyclohexyl-c-1-carbonitril
r-1-(7-Hydroxyheptyl)-t-4-(r-4-ethyl-t-1-cyclohexyl)-cyclohexyl-c-1-carbonitril
r-1-(7-Hydroxyheptyl)-t-4-(r-4-propyl-t-1-cyclohexyl)-cyclohexyl-c-1-carbonitril
r-1-(7-Hydroxyheptyl)-t-4-(r-4-butyl-t-1-cyclohexyl)-cyclohexyl-c-1-carbonitril
r-1-(7-Hydroxyheptyl)-t-4-(r-4-pentyl-t-1-cyclohexyl)-cyclohexyl-c-1-carbonitril
r-1-(7-Hydroxyheptyl)-t-4-(r-4-hexyl-t-1-cyclohexyl)-cyclohexyl-c-1-carbonitril
r-1-(7-Hydroxyheptyl)-t-4-(r-4-heptyl-t-1-cyclohexyl)-cyclohexyl-c-1-carbonitril
r-1-(7-Hydroxyheptyl)-t-4-(r-4-octyl-t-1-cyclohexyl)-cyclohexyl-c-1-carbonitril
r-1-(8-Hydroxyoctyl)-t-4-(r-4-ethyl-t-1-cyclohexyl)-cyclohexyl-c-1-carbonitril
r-1-(8-Hydroxyoctyl)-t-4-(r-4-propyl-t-1-cyclohexyl)-cyclohexyl-c-1-carbonitril
r-1-(8-Hydroxyoctyl)-t-4-(r-4-butyl-t-1-cyclohexyl)-cyclohexyl-c-1-carbonitril
r-1-(8-Hydroxyoctyl)-t-4-(r-4-pentyl-t-1-cyclohexyl)-cyclohexyl-c-1-carbonitril
r-1-(8-Hydroxyoctyl)-t-4-(r-4-hexyl-t-1-cyclohexyl)-cyclohexyl-c-1-carbonitril
r-1-(8-Hydroxyoctyl)-t-4-(r-4-heptyl-t-1-cyclohexyl)-cyclohexyl-c-1-carbonitril
r-1-(8-Hydroxyoctyl)-t-4-(r-4-octyl-t-1-cyclohexyl)-cyclohexyl-c-1-carbonitril
r-1-(9-Hydroxynonyl)-t-4-(r-4-ethyl-t-1-cyclohexyl)-cyclohexyl-c-1-carbonitril
r-1-(9-Hydroxynonyl)-t-4-(r-4-propyl-t-1-cyclohexyl)-cyclohexyl-c-1-carbonitril
r-1-(9-Hydroxynonyl)-t-4-(r-4-butyl-t-1-cyclohexyl)-cyclohexyl-c-1-carbonitril
r-1-(9-Hydroxynonyl)-t-4-(r-4-pentyl-t-1-cyclohexyl)-cyclohexyl-c-1-carbonitril
r-1-(9-Hydroxynonyl)-t-4-(r-4-hexyl-t-1-cyclohexyl)-cyclohexyl-c-1-carbonitril
r-1-(9-Hydroxynonyl)-t-4-(r-4-heptyl-t-1-cyclohexyl)-cyclohexyl-c-1-carbonitril
r-1-(9-Hydroxynonyl)-t-4-(r-4-octyl-t-1-cyclohexyl)-cyclohexyl-c-1-carbonitril
r-1-(10-Hydroxydecyl)-t-4-(r-4-ethyl-t-1-cyclohexyl)-cyclohexyl-c-1-carbonitril
r-1-(10-Hydroxydecyl)-t-4-(r-4-propyl-t-1-cyclohexyl)-cyclohexyl-c-1-carbonitril
r-1-(10-Hydroxydecyl)-t-4-(r-4-butyl-t-1-cyclohexyl)-cyclohexyl-c-1-carbonitril
r-1-(10-Hydroxydecyl)-t-4-(r-4-pentyl-t-1-cyclohexyl)-cyclohexyl-c-1-carbonitril
r-1-(10-Hydroxydecyl)-t-4-(r-4-hexyl-t-1-cyclohexyl)-cyclohexyl-c-1-carbonitril
r-1-(10-Hydroxydecyl)-t-4-(r-4-heptyl-t-1-cyclohexyl)-cyclohexyl-c-1-carbonitril
r-1-(10-Hydroxydecyl)-t-4-(r-4-octyl-t-1-cyclohexyl)-cyclohexyl-c-1-carbonitril

r-1-(11-Hydroxyundecyl)-t-4-(r-4-ethyl-t-1-cyclohexyl)-cyclohexyl-c-1-carbonitril
r-1-(11-Hydroxyundecyl)-t-4-(r-4-propyl-t-1-cyclohexyl)-cyclohexyl-c-1-carbonitril
r-1-(11-Hydroxyundecyl)-t-4-(r-4-butyl-t-1-cyclohexyl)-cyclohexyl-c-1-carbonitril
r-1-(11-Hydroxyundecyl)-t-4-(r-4-pentyl-t-1-cyclohexyl)-cyclohexyl-c-1-carbonitril
r-1-(11-Hydroxyundecyl)-t-4-(r-4-hexyl-t-1-cyclohexyl)-cyclohexyl-c-1-carbonitril
r-1-(11-Hydroxyundecyl)-t-4-(r-4-heptyl-t-1-cyclohexyl)-cyclohexyl-c-1-carbonitril
r-1-(11-Hydroxyundecyl)-t-4-(r-4-octyl-t-1-cyclohexyl)-cyclohexyl-c-1-carbonitril

Beispiel 4

Zu einer Lösung von 3,9 g r-1-(4-Hydroxybutyl)-t-4-(r-4-pentyl-t-1-cyclohexyl)-cyclohexyl-c-1-carbonitril (Herstellung siehe Beispiel 3), 1,0 g Methacrylsäure, 143 mg 4-Dimethylaminopyridin und 3 mg 2,6-Di-tert.butyl-4-methylphenol in 20 ml $CH_2Cl_2$ gibt man bei 0° 2,7 g Dicyclohexylcarbodiimid in 2 ml $CH_2Cl_2$ und rührt dann noch 2 Stunden bei Raumtemperatur. Der Niederschlag wird abgesaugt, das Filtrat eingeengt und der Rückstand an einer Kieselgelsäule mit $CH_2Cl_2$/Ethylacetat (99/1) chromatographiert. Nach Umkristallisation erhält man 4-[c-1-Cyano-r-4-(r-4-pentyl-t-1-cyclohexyl)-t-1-cyclohexyl]-butylmethacrylat mit Fp. = 36-37°.

Analog werden hergestellt:

4-[c-1-Cyano-r-4-(r-4-ethyl-t-1-cyclohexyl)-t-1-cyclohexyl]-butyl-methacrylat
4-[c-1-Cyano-r-4-(r-4-propyl-t-1-cyclohexyl)-t-1-cyclohexyl]-butyl-methacrylat
4-[c-1-Cyano-r-4-(r-4-butyl-t-1-cyclohexyl)-t-1-cyclohexyl]-butyl-methacrylat
4-[c-1-Cyano-r-4-(r-4-hexyl-t-1-cyclohexyl)-t-1-cyclohexyl]-butyl-methacrylat
4-[c-1-Cyano-r-4-(r-4-heptyl-t-1-cyclohexyl)-t-1-cyclohexyl]-butyl-methacrylat
4-[c-1-Cyano-r-4-(r-4-octyl-t-1-cyclohexyl)-t-1-cyclohexyl]-butyl-methacrylat
5-[c-1-Cyano-r-4-(r-4-ethyl-t-1-cyclohexyl)-t-1-cyclohexyl]-pentyl-methacrylat
5-[c-1-Cyano-r-4-(r-4-propyl-t-1-cyclohexyl)-t-1-cyclohexyl]-pentyl-methacrylat
5-[c-1-Cyano-r-4-(r-4-butyl-t-1-cyclohexyl)-t-1-cyclohexyl]-pentyl-methacrylat
5-[c-1-Cyano-r-4-(r-4-hexyl-t-1-cyclohexyl)-t-1-cyclohexyl]-pentyl-methacrylat
5-[c-1-Cyano-r-4-(r-4-heptyl-t-1-cyclohexyl)-t-1-cyclohexyl]-pentyl-methacrylat
5-[c-1-Cyano-r-4-(r-4-octyl-t-1-cyclohexyl)-t-1-cyclohexyl]-pentyl-methacrylat
6-[c-1-Cyano-r-4-(r-4-ethyl-t-1-cyclohexyl)-t-1-cyclohexyl]-hexyl-methacrylat
6-[c-1-Cyano-r-4-(r-4-propyl-t-1-cyclohexyl)-t-1-cyclohexyl]-hexyl-methacrylat
6-[c-1-Cyano-r-4-(r-4-butyl-t-1-cyclohexyl)-t-1-cyclohexyl]-hexyl-methacrylat
6-[c-1-Cyano-r-4-(r-4-pentyl-t-1-cyclohexyl)-t-1-cyclo-hexyl]-hexyl-methacrylat
6-[c-1-Cyano-r-4-(r-4-hexyl-t-1-cyclohexyl)-t-1-cyclohexyl]-hexyl-methacrylat
6-[c-1-Cyano-r-4-(r-4-heptyl-t-1-cyclohexyl)-t-1-cyclohexyl]-hexyl-methacrylat
6-[c-1-Cyano-r-4-(r-4-octyl-t-1-cyclohexyl)-t-1-cyclohexyl]-hexyl-methacrylat
7-[c-1-Cyano-r-4-(r-4-ethyl-t-1-cyclohexyl)-t-1-cyclohexyl]-heptyl-methacrylat
7-[c-1-Cyano-r-4-(r-4-propyl-t-1-cyclohexyl)-t-1-cyclohexyl]-heptyl-methacrylat
7-[c-1-Cyano-r-4-(r-4-butyl-t-1-cyclohexyl)-t-1-cyclohexyl]-heptyl-methacrylat
7-[c-1-Cyano-r-4-(r-4-pentyl-t-1-cyclohexyl)-t-1-cyclohexyl]-heptyl-methacrylat
7-[c-1-Cyano-r-4-(r-4-hexyl-t-1-cyclohexyl)-t-1-cyclohexyl]-heptyl-methacrylat
7-[c-1-Cyano-r-4-(r-4-heptyl-t-1-cyclohexyl)-t-1-cyclohexyl]-heptyl-methacrylat
7-[c-1-Cyano-r-4-(r-4-octyl-t-1-cyclohexyl)-t-1-cyclohexyl]-heptyl-methacrylat
8-[c-1-Cyano-r-4-(r-4-ethyl-t-1-cyclohexyl)-t-1-cyclohexyl]-octyl-methacrylat
8-[c-1-Cyano-r-4-(r-4-propyl-t-1-cyclohexyl)-t-1-cyclohexyl]-octyl-methacrylat
8-[c-1-Cyano-r-4-(r-4-butyl-t-1-cyclohexyl)-t-1-cyclohexyl]-octyl-methacrylat
8-[c-1-Cyano-r-4-(r-4-hexyl-t-1-cyclohexyl)-t-1-cyclohexyl]-octyl-methacrylat
8-[c-1-Cyano-r-4-(r-4-heptyl-t-1-cyclohexyl)-t-1-cyclohexyl]-octyl-methacrylat
8-[c-1-Cyano-r-4-(r-4-octyl-t-1-cyclohexyl)-t-1-cyclohexyl]-octyl-methacrylat
9-[c-1-Cyano-r-4-(r-4-ethyl-t-1-cyclohexyl)-t-1-cyclohexyl]-nonyl-methacrylat
9-[c-1-Cyano-r-4-(r-4-propyl-t-1-cyclohexyl)-t-1-cyclohexyl]-nonyl-methacrylat
9-[c-1-Cyano-r-4-(r-4-butyl-t-1-cyclohexyl)-t-1-cyclohexyl]-nonyl-methacrylat
9-[c-1-Cyano-r-4-(r-4-hexyl-t-1-cyclohexyl)-t-1-cyclohexyl]-nonyl-methacrylat
9-[c-1-Cyano-r-4-(r-4-heptyl-t-1-cyclohexyl)-t-1-cyclohexyl]-nonyl-methacrylat
9-[c-1-Cyano-r-4-(r-4-octyl-t-1-cyclohexyl)-t-1-cyclohexyl]-nonyl-methacrylat
10-[c-1-Cyano-r-4-(r-4-ethyl-t-1-cyclohexyl)-t-1-cyclohexyl]-decyl-methacrylat
10-[c-1-Cyano-r-4-(r-4-propyl-t-1-cyclohexyl)-t-1-cyclohexyl]-decyl-methacrylat

10-[c-1-Cyano-r-4-(r-4-butyl-t-1-cyclohexyl)-t-1-cyclohexyl]-decyl-methacrylat
10-[c-1-Cyano-r-4-(r-4-hexyl-t-1-cyclohexyl)-t-1-cyclohexyl] -decyl-methacrylat
10-[c-1-Cyano-r-4-(r-4-heptyl-t-1-cyclohexyl)-t-1-cyclohexyl]-decyl-methacrylat
10-[c-1-Cyano-r-4-(r-4-octyl-t-1-cyclohexyl)-t-1-cyclohexyl]-decyl-methacrylat
3-[c-1-Cyano-r-4-(r-4-ethyl-t-1-cyclohexyl)-t-1-cyclohexyl]-propyl-methacrylat
3-[c-1-Cyano-r-4-(r-4-propyl-t-1-cyclohexyl)-t-1-cyclohexyl]-propyl-methacrylat
3-[c-1-Cyano-r-4-(r-4-butyl-t-1-cyclohexyl)-t-1-cyclohexyl]-propyl-methacrylat
3-[c-1-Cyano-r-4-(r-4-hexyl-t-1-cyclohexyl)-t-1-cyclohexyl]-propyl-methacrylat
3-[c-1-Cyano-r-4-(r-4-heptyl-t-1-cyclohexyl)-t-1-cyclohexyl]-propyl-methacrylat
3-[c-1-Cyano-r-4-(r-4-octyl-t-1-cyclohexyl)-t-1-cyclohexyl]-propyl-methacrylat

Beispiel 5

a) Zu einem Gemisch aus 3,5 g Diisopropylamin in 30 ml THF gibt man bei 0° unter Rühren 20 ml einer 15%igen Lösung von n-Butyllithium in Hexan. Dieses Gemisch gibt man dann bei -70° zu einer Lösung aus 10,3 g 4-[4'-Octyloxy-1,1'-biphenyl-4-yl]-cyclohexylcarbonitril und 30 ml THF und rührt noch 2 Stunden. Anschließend gibt man 4,3 g 1-Brom-3-buten zu und läßt das Reaktionsgemisch auf 0° kommen.

Man gibt 5 ml Ethanol zu, gießt die Mischung auf Eiswasser und säuert mit 2 N HCl an. Man extrahiert mit tert.-Butylmethylether und arbeitet die organischen Phasen auf. Nach chromatographischer Reinigung an Kieselgel (Petrolether/Ethylacetat, 9:1) und Umkristallisation erhält man c-4-[4'-Octyloxy-1,1'-biphenyl-4-yl]-1-(3-butenyl)-r-1-cyclohexylcarbonitril mit K 69° $S_C$ 87° $S_A$ 111° N 161°.

b) Zu einem Gemisch aus 6,0 g Olefin, hergestellt nach a), und 12 ml THF gibt man bei 0° 5,4 g einer 1 M Lösung von Boran-THF-Addukt in THF und rührt noch 1 Stunde. Anschließend werden 1,4 ml Wasser, 1,8 ml 3 N NaOH und 1,8 ml 30%ige $H_2O_2$ zugegeben, und es wird 1 Stunde bei Raumtemperatur gerührt.

Die Mischung wird auf Wasser gegossen, mit tert.-Butylmethylether extrahiert, die organischen Phasen aufgearbeitet und der Rückstand an Kieselgel mit $CH_2Cl_2$/Ethylacetat 85:15 chromatographiert. Nach Umkristallisation aus Ethanol erhält man c-4-[4'-Octyloxy-1,1'-biphenyl-4-yl]-1-(4-hydroxybutyl)-r-1-cyclohexylcarbonitril mit K 96° $S_C$ 119° $S_A$ 151° N 164° I.

Analog werden hergestellt:

c-4-[4'-Ethoxy-1,1'-biphenyl-4-yl]-1-(4-hydroxybutyl)-r-1-cyclohexylcarbonitril
c-4-[4'-Propoxy-1,1'-biphenyl-4-yl]-1-(4-hydroxybutyl)-r-1-cyclohexylcarbonitril
c-4-[4'-Butoxy-1,1'-biphenyl-4-yl]-1-(4-hydroxybutyl)-r-1-cyclohexylcarbonitril
c-4-[4'-Pentyloxy-1,1'-biphenyl-4-yl]-1-(4-hydroxybutyl)-r-1-cyclohexylcarbonitril
c-4-[4'-Hexyloxy-1,1'-biphenyl-4-yl]-1-(4-hydroxybutyl)-r-1-cyclohexylcarbonitril
c-4-[4'-Heptyloxy-1,1'-biphenyl-4-yl]-1-(4-hydroxybutyl)-r-1-cyclohexylcarbonitril
c-4-[4'-Ethyl-1,1'-biphenyl-4-yl]-1-(4-hydroxybutyl)-r-1-cyclohexylcarbonitril
c-4-[4'-Propyl-1,1'-biphenyl-4-yl]-1-(4-hydroxybutyl)-r-1-cyclohexylcarbonitril
c-4-[4'-Butyl-1,1'-biphenyl-4-yl]-1-(4-hydroxybutyl)-r-1-cyclohexylcarbonitril
c-4-[4'-Pentyl-1,1'-biphenyl-4-yl]-1-(4-hydroxybutyl)-r-1-cyclohexylcarbonitril
c-4-[4'-Ethoxy-1,1'-biphenyl-4-yl]-1-(5-hydroxypentyl)-r-1-cyclohexylcarbonitril
c-4-[4'-Propoxy-1,1'-biphenyl-4-yl]-1-(5-hydroxypentyl)-r-1-cyclohexylcarbonitril
c-4-[4'-Butoxy-1,1'-biphenyl-4-yl]-1-(5-hydroxypentyl)-r-1-cyclohexylcarbonitril
c-4-[4'-Pentyloxy-1,1'-biphenyl-4-yl]-1-(5-hydroxypentyl)-r-1-cyclohexylcarbonitril
c-4-[4'-Hexyloxy-1,1'-biphenyl-4-yl]-1-(5-hydroxypentyl)-r-1-cyclohexylcarbonitril
c-4-[4'-Heptyloxy-1,1'-biphenyl-4-yl]-1-(5-hydroxypentyl)-r-1-cyclohexylcarbonitril
c-4-[4'-Octyloxy-1,1'-biphenyl-4-yl]-1-(5-hydroxypentyl)-r-1-cyclohexylcarbonitril
c-4-[4'-Ethyl-1,1'-biphenyl-4-yl]-1-(5-hydroxypentyl)-r-1-cyclohexylcarbonitril
c-4-[4'-Propyl-1,1'-biphenyl-4-yl]-1-(5-hydroxypentyl)-r-1-cyclohexylcarbonitril
c-4-[4'-Butyl-1,1'-biphenyl-4-yl]-1-(5-hydroxypentyl)-r-1-cyclohexylcarbonitril
C-4-[4'-Pentyl-1,1'-biphenyl-4-yl]-1-(5-hydroxypentyl)-r-1-cyclohexylcarbonitril
c-4-[4'-Ethoxy-1,1'-biphenyl-4-yl]-1-(6-hydroxyhexyl)-r-1-cyclohexylcarbonitril
c-4-[4'-Propoxy-1,1'-biphenyl-4-yl]-1-(6-hydroxyhexyl)-r-1-cyclohexylcarbonitril
c-4-[4'-Butoxy-1,1'-biphenyl-4-yl]-1-(6-hydroxyhexyl)-r-1-cyclohexylcarbonitril
c-4-[4'-Pentyloxy-1,1'-biphenyl-4-yl]-1-(6-hydroxyhexyl)-r-1-cyclohexylcarbonitril
c-4-[4'-Hexyloxy-1,1'-biphenyl-4-yl]-1-(6-hydroxyhexyl)-r-1-cyclohexylcarbonitril
c-4-[4'-Heptyloxy-1,1'-biphenyl-4-yl]-1-(6-hydroxyhexyl)-r-1-cyclohexylcarbonitril

c-4-[4'-Octyloxy-1,1'-biphenyl-4-yl]-1-(6-hydroxyhexyl)-r-1-cyclohexylcarbonitril
c-4-[4'-Ethyl-1,1'-biphenyl-4-yl]-1-(6-hydroxyhexyl)-r-1-cyclohexylcarbonitril
c-4-[4'-Propyl-1,1'-biphenyl-4-yl]-1-(6-hydroxyhexyl)-r-1-cyclohexylcarbonitril
c-4-[4'-Butyl-1,1'-biphenyl-4-yl]-1-(6-hydroxyhexyl)-r-1-cyclohexylcarbonitril
c-4-[4'-Pentyl-1,1'-biphenyl-4-yl]-1-(6-hydroxyhexyl)-r-1-cyclohexylcarbonitril
c-4-[4'-Ethoxy-1,1'-biphenyl-4-yl]-1-(7-hydroxyheptyl)-r-1-cyclohexylcarbonitril
c-4-[4'-Propoxy-1,1'-biphenyl-4-yl]-1-(7-hydroxyheptyl)-r-1-cyclohexylcarbonitril
c-4-[4'-Butoxy-1,1'-biphenyl-4-yl]-1-(7-hydroxyheptyl)-r-1-cyclohexylcarbonitril
c-4-[4'-Pentyloxy-1,1'-biphenyl-4-yl]-1-(7-hydroxyheptyl)-r-1-cyclohexylcarbonitril
c-4-[4'-Hexyloxy-1,1'-biphenyl-4-yl]-1-(7-hydroxyheptyl)-r-1-cyclohexylcarbonitril
c-4-[4'-Heptyloxy-1,1'-biphenyl-4-yl]-1-(7-hydroxyheptyl)-r-1-cyclohexylcarbonitril
c-4-[4'-Octyloxy-1,1'-biphenyl-4-yl]-1-(7-hydroxyheptyl)-r-1-cyclohexylcarbonitril
c-4-[4'-Ethyl-1,1'-biphenyl-4-yl]-1-(7-hydroxyheptyl)-r-1-cyclohexylcarbonitril
c-4-[4'-Propyl-1,1'-biphenyl-4-yl]-1-(7-hydroxyheptyl)-r-1-cyclohexylcarbonitril
c-4-[4'-Butyl-1,1'-biphenyl-4-yl]-1-(7-hydroxyheptyl)-r-1-cyclohexylcarbonitril
c-4-[4'-Pentyl-1,1'-biphenyl-4-yl]-1-(7-hydroxyheptyl)-r-1-cyclohexylcarbonitril
c-4-[4'-Ethoxy-1,1'-biphenyl-4-yl]-1-(8-hydroxyoctyl)-r-1-cyclohexylcarbonitril
c-4-[4'-Propoxy-1,1'-biphenyl-4-yl]-1-(8-hydroxyoctyl)-r-1-cyclohexylcarbonitril
c-4-[4'-Butoxy-1,1'-biphenyl-4-yl]-1-(8-hydroxyoctyl)-r-1-cyclohexylcarbonitril
c-4-[4'-Pentyloxy-1,1'-biphenyl-4-yl]-1-(8-hydroxyoctyl)-r-1-cyclohexylcarbonitril
c-4-[4'-Hexyloxy-1,1'-biphenyl-4-yl]-1-(8-hydroxyoctyl)-r-1-cyclohexylcarbonitril
c-4-[4'-Heptyloxy-1,1'-biphenyl-4-yl]-1-(8-hydroxyoctyl)-r-1-cyclohexylcarbonitril
c-4-[4'-Octyloxy-1,1'-biphenyl-4-yl]-1-(8-hydroxyoctyl)-r-1-cyclohexylcarbonitril
c-4-[4'-Ethyl-1,1'-biphenyl-4-yl]-1-(8-hydroxyoctyl)-r-1-cyclohexylcarbonitril
c-4-[4'-Propyl-1,1'-biphenyl-4-yl]-1-(8-hydroxyoctyl)-r-1-cyclohexylcarbonitril
c-4-[4'-Butyl-1,1'-biphenyl-4-yl]-1-(8-hydroxyoctyl)-r-1-cyclohexylcarbonitril
c-4-[4'-Pentyl-1,1'-biphenyl-4-yl]-1-(8-hydroxyoctyl)-r-1-cyclohexylcarbonitril
c-4-[4'-Ethoxy-1,1'-biphenyl-4-yl]-1-(9-hydroxynonyl)-r-1-cyclohexylcarbonitril
c-4-[4'-Propoxy-1,1'-biphenyl-4-yl]-1-(9-hydroxynonyl)-r-1-cyclohexylcarbonitril
c-4-[4'-Butoxy-1,1'-biphenyl-4-yl]-1-(9-hydroxynonyl)-r-1-cyclohexylcarbonitril
c-4-[4'-Pentyloxy-1,1'-biphenyl-4-yl]-1-(9-hydroxynonyl)-r-1-cyclohexylcarbonitril
c-4-[4'-Hexyloxy-1,1'-biphenyl-4-yl]-1-(9-hydroxynonyl)-r-1-cyclohexylcarbonitril
c-4-[4'-Heptyloxy-1,1'-biphenyl-4-yl]-1-(9-hydroxynonyl)-r-1-cyclohexylcarbonitril
c-4-[4'-Octyloxy-1,1'-biphenyl-4-yl]-1-(9-hydroxynonyl)-r-1-cyclohexylcarbonitril
c-4-[4'-Ethyl-1,1'-biphenyl-4-yl]-1-(9-hydroxynonyl)-r-1-cyclohexylcarbonitril
c-4-[4'-Propyl-1,1'-biphenyl-4-yl]-1-(9-hydroxynonyl)-r-1-cyclohexylcarbonitril
c-4-[4'-Butyl-1,1'-biphenyl-4-yl]-1-(9-hydroxynonyl)-r-1-cyclohexylcarbonitril
c-4-[4'-Pentyl-1,1'-biphenyl-4-yl]-1-(9-hydroxynonyl)-r-1-cyclohexylcarbonitril
c-4-[4'-Ethoxy-1,1'-biphenyl-4-yl]-1-(10-hydroxydecyl)-r-1-cyclohexylcarbonitril
c-4-[4'-Propoxy-1,1'-biphenyl-4-yl]-1-(10-hydroxydecyl)-r-1-cyclohexylcarbonitril
c-4-[4'-Butoxy-1,1'-biphenyl-4-yl]-1-(10-hydroxydecyl)-r-1-cyclohexylcarbonitril
c-4-[4'-Pentyloxy-1,1'-biphenyl-4-yl]-1-(10-hydroxydecyl)-r-1-cyclohexylcarbonitril
c-4-[4'-Hexyloxy-1,1'-biphenyl-4-yl]-1-(10-hydroxydecyl)-r-1-cyclohexylcarbonitril
c-4-[4'-Heptyloxy-1,1'-biphenyl-4-yl)-1-(10-hydroxydecyl)-r-1-cyclohexylcarbonitril
c-4-[4'-Octyloxy-1,1'-biphenyl-4-yl]-1-(10-hydroxydecyl)-r-1-cyclohexylcarbonitril
c-4-[4'-Ethyl-1,1'-biphenyl-4-yl]-1-(10-hydroxydecyl)-r-1-cyclohexylcarbonitril
c-4-[4'-Propyl-1,1'-biphenyl-4-yl]-1-(10-hydroxydecyl)-r-1-cyclohexylcarbonitril
c-4-[4'-Butyl-1,1'-biphenyl-4-yl]-1-(10-hydroxydecyl)-r-1-cyclohexylcarbonitril
c-4-[4'-Pentyl-1,1'-biphenyl-4-yl]-1-(10-hydroxydecyl)-r-1-cyclohexylcarbonitril
c-4-[4'-Ethoxy-1,1'-biphenyl-4-yl]-1-(11-hydroxyundecyl)-r-1-cyclohexylcarbonitril
c-4-[4'-Propoxy-1,1'-biphenyl-4-yl]-1-(11-hydroxyundecyl)-r-1-cyclohexylcarbonitril
c-4-[4'-Butoxy-1,1'-biphenyl-4-yl]-1-(11-hydroxyundecyl)-r-1-cyclohexylcarbonitril
c-4-[4'-Pentyloxy-1,1'-biphenyl-4-yl]-1-(11-hydroxyundecyl)-r-1-cyclohexylcarbonitril
c-4-[4'-Hexyloxy-1,1'-biphenyl-4-yl]-1-(11-hydroxyundecyl)-r-1-cyclohexylcarbonitril
c-4-[4'-Heptyloxy-1,1'-biphenyl-4-yl]-1-(11-hydroxyundecyl)-r-1-cyclohexylcarbonitril
c-4-[4'-Octyloxy-1,1'-biphenyl-4-yl]-1-(11-hydroxyundecyl)-r-1-cyclohexylcarbonitril
c-4-[4'-Ethyl-1,1'-biphenyl-4-yl]-1-(11-hydroxyundecyl)-r-1-cyclohexylcarbonitril
c-4-[4'-Propyl-1,1'-biphenyl-4-yl]-1-(11-hydroxyundecyl)-r-1-cyclohexylcarbonitril

c-4-[4'-Butyl-1,1'-biphenyl-4-yl]-1-(11-hydroxyundecyl)-r-1-cyclohexylcarbonitril
c-4-[4'-Pentyl-1,1'-biphenyl-4-yl]-1-(11-hydroxyundecyl)-r-1-cyclohexylcarbonitril
c-4-[4'-(2-Methylbutyloxy)-1,1'-biphenyl-4-yl]-1-(11-hydroxyundecyl)-r-1-cyclohexylcarbonitril, optisch aktiv
c-4-[4'-(2-Methylbutyloxy)-1,1'-biphenyl-4-yl]-1-(10-hydroxydecyl)-r-1-cyclohexylcarbonitril, optisch aktiv
c-4-[4'-(2-Methylbutyloxy)-1,1'-biphenyl-4-yl]-1-(9-hydroxynonyl)-r-1-cyclohexylcarbonitril, optisch aktiv
c-4-[4'-(2-Methylbutyloxy)-1,1'-biphenyl-4-yl]-1-(8-hydroxyoctyl)-r-1-cyclohexylcarbonitril, optisch aktiv
c-4-[4'-(2-Methylbutyloxy)-1,1'-biphenyl-4-yl]-1-(7-hydroxyheptyl)-r-1-cyclohexylcarbonitril, optisch aktiv
c-4-[4'-(2-Octyloxy)-1,1'-biphenyl-4-yl]-1-(11-hydroxyundecyl)-r-1-cyclohexylcarbonitril, optisch aktiv
c-4-[4'-(2-Octyloxy)-1,1'-biphenyl-4-yl]-1-(10-hydroxydecyl)-r-1-cyclohexylcarbonitril, optisch aktiv
c-4-[4'-(2-Octyloxy)-1,1'-biphenyl-4-yl]-1-(9-hydroxynonyl)-r-1-cyclohexylcarbonitril, optisch aktiv
c-4-[4'-(2-Octyloxy)-1,1'-biphenyl-4-yl]-1-(8-hydroxyoctyl)-r-1-cyclohexylcarbonitril, optisch aktiv
c-4-[4'-(2-Octyloxy)-1,1'-biphenyl-4-yl]-1-(7-hydroxyheptyl)-r-1-cyclohexylcarbonitril, optisch aktiv

Beispiel 6

Man gibt zu einem Gemisch aus 2,2 g c-4-[-4'-Octyloxy-1,1'-biphenyl-4-yl]-1-(4-hydroxybutyl)-r-1-cyclohexylcarbonitril (Darstellung siehe Beispiel 5), 0,41 g Methacrylsäure, 58 mg 4-Dimethylaminopyridin, 2 mg 2,6-Ditert.butyl-4-methylphenol und 10 ml $CH_2Cl_2$ bei 0° eine Lösung von 1,08 g Dicyclohexylcarbodiimid (DCCI) in 2 ml $CH_2Cl_2$ und rührt dann noch 2 Stunden bei Raumtemperatur. Der Niederschlag wird abgesaugt, das Filtrat eingeengt und der Rückstand an Kieselgel mit Petrolether/Ethylacetat (9:1) chromatographiert. Nach Umkristallisation erhält man c-4-[4'-Octyloxy-1,1'-biphenyl-4-yl]-1-(4-methacryloyloxybutyl)-r-1-cyclohexylcarbonitril mit K 81° $S_A$ 124° N 126° I [$S_C$ 39° $S_A$].

Analog werden hergestellt:

c-4-[-4'-Ethoxy-1,1'-biphenyl-4-yl]-1-(4-methacryloyloxybutyl)-r-1-cyclohexylcarbonitril
c-4-[-4'-Propoxy-1,1'-biphenyl-4-yl]-1-(4-methacryloyloxybutyl)-r-1-cyclohexylcarbonitril
c-4-[-4'-Butoxy-1,1'-biphenyl-4-yl]-1-(4-methacryloyloxybutyl)-r-1-cyclohexylcarbonitril
c-4-[-4'-Pentyloxy-1,1'-biphenyl-4-yl]-1-(4-methacryloyloxybutyl)-r-1-cyclohexylcarbonitril
c-4-[-4'-Hexyloxy-1,1'-biphenyl-4-yl]-1-(4-methacryloyloxybutyl)-r-1-cyclohexylcarbonitril
c-4-[-4'-Heptyloxy-1,1'-biphenyl-4-yl]-1-(4-methacryloyloxybutyl)-r-1-cyclohexylcarbonitril
c-4-[-4'-Ethyl-1,1'-biphenyl-4-yl]-1-(4-methacryloyloxybutyl)-r-1-cyclohexylcarbonitril
c-4-[-4'-Propyl-1,1'-biphenyl-4-yl]-1-(4-methacryloyloxybutyl)-r-1-cyclohexylcarbonitril
c-4-[-4'-Butyl-1,1'-biphenyl-4-yl]-1-(4-methacryloyloxybutyl)-r-1-cyclohexylcarbonitril
c-4-[-4'-Pentyl-1,1'-biphenyl-4-yl]-1-(4-methacryloyloxybutyl)-r-1-cyclohexylcarbonitril
c-4-[-4'-Ethoxy-1,1'-biphenyl-5-yl]-1-(5-methacryloyloxypentyl)-r-1-cyclohexylcarbonitril
c-4-[-4'-Propoxy-1,1'-biphenyl-5-yl]-1-(5-methacryloyloxypentyl)-r-1-cyclohexylcarbonitril
c-4-[-4'-Butoxy-1,1'-biphenyl-5-yl]-1-(5-methacryloyloxypentyl)-r-1-cyclohexylcarbonitril
c-4-[-4'-Pentyloxy-1,1'-biphenyl-5-yl]-1-(5-methacryloyloxypentyl)-r-1-cyclohexylcarbonitril
c-4-[-4'-Hexyloxy-1,1'-biphenyl-5-yl]-1-(5-methacryloyloxypentyl)-r-1-cyclohexylcarbonitril
c-4-[-4'-Heptyloxy-1,1'-biphenyl-5-yl]-1-(5-methacryloyloxypentyl)-r-1-cyclohexylcarbonitril
c-4-[-4'-Octyloxy-1,1'-biphenyl-5-yl]-1-(5-methacryloyloxypentyl)-r-1-cyclohexylcarbonitril
c-4-[-4'-Ethyl-1,1'-biphenyl-5-yl]-1-(5-methacryloyloxypentyl)-r-1-cyclohexylcarbonitril
c-4-[-4'-Propyl-1,1'-biphenyl-5-yl]-1-(5-methacryloyloxypentyl)-r-1-cyclohexylcarbonitril
c-4-[-4'-Butyl-1,1'-biphenyl-5-yl]-1-(5-methacryloyloxypentyl)-r-1-cyclohexylcarbonitril
c-4-[-4'-Pentyl-1,1'-biphenyl-5-yl]-1-(5-methacryloyloxypentyl)-r-1-cyclohexylcarbonitril
c-4-[-4'-Ethoxy-1,1'-biphenyl-4-yl]-1-(6-methacryloyloxyhexyl)-r-1-cyclohexylcarbonitril
c-4-[-4'-Propoxy-1,1'-biphenyl-4-yl]-1-(6-methacryloyloxyhexyl)-r-1-cyclohexylcarbonitril
c-4-[-4'-Butoxy-1,1'-biphenyl-4-yl]-1-(6-methacryloyloxyhexyl)-r-1-cyclohexylcarbonitril
c-4-[-4'-Pentyloxy-1,1'-biphenyl-4-yl]-1-(6-methacryloyloxyhexyl)-r-1-cyclohexylcarbonitril
c-4-[-4'-Hexyloxy-1,1'-biphenyl-4-yl]-1-(6-methacryloyloxyhexyl)-r-1-cyclohexylcarbonitril
c-4-[-4'-Heptyloxy-1,1'-biphenyl-4-yl]-1-(6-methacryloyloxyhexyl)-r-1-cyclohexylcarbonitril
c-4-[-4'-Octyloxy-1,1'-biphenyl-4-yl]-1-(6-methacryloyloxyhexyl)-r-1-cyclohexylcarbonitril
c-4-[-4'-Ethyl-1,1'-biphenyl-4-yl]-1-(6-methacryloyloxyhexyl)-r-1-cyclohexylcarbonitril
c-4-[-4'-Propyl-1,1'-biphenyl-4-yl]-1-(6-methacryloyloxyhexyl)-r-1-cyclohexylcarbonitril
c-4-[-4'-Butyl-1,1'-biphenyl-4-yl]-1-(6-methacryloyloxyhexyl)-r-1-cyclohexylcarbonitril
c-4-[-4'-Pentyl-1,1'-biphenyl-4-yl]-1-(6-methacryloyloxyhexyl)-r-1-cyclohexylcarbonitril
c-4-[-4'-Ethoxy-1,1'-biphenyl-4-yl]-1-(7-methacryloyloxyheptyl)-r-1-cyclohexylcarbonitril
c-4-[-4'-Propoxy-1,1'-biphenyl-4-yl]-1-(7-methacryloyloxyheptyl)-r-1-cyclohexylcarbonitril
c-4-[-4'-Butoxy-1,1'-biphenyl-4-yl]-1-(7-methacryloyloxyheptyl)-r-1-cyclohexylcarbonitril

c-4-[-4'-Pentyloxy-1,1'-biphenyl-4-yl]-1-(7-methacryloyloxyheptyl)-r-1-cyclohexylcarbonitril
c-4-[-4'-Hexyloxy-1,1'-biphenyl-4-yl]-1-(7-methacryloyloxyheptyl)-r-1-cyclohexylcarbonitril
c-4-[-4'-Heptyloxy-1,1'-biphenyl-4-yl]-1-(7-methacryloyloxyheptyl)-r-1-cyclohexylcarbonitril
c-4-[-4'-Octyloxy-1,1'-biphenyl-4-yl]-1-(7-methacryloyloxyheptyl)-r-1-cyclohexylcarbonitril
c-4-[-4'-Ethyl-1,1'-biphenyl-4-yl]-1-(7-methacryloyloxyheptyl)-r-1-cyclohexylcarbonitril
c-4-[-4'-Propyl-1,1'-biphenyl-4-yl]-1-(7-methacryloyloxyheptyl)-r-1-cyclohexylcarbonitril
c-4-[-4'-Butyl-1,1'-biphenyl-4-yl]-1-(7-methacryloyloxyheptyl)-r-1-cyclohexylcarbonitril
c-4-[-4'-Pentyl-1,1'-biphenyl-4-yl]-1-(7-methacryloyloxyheptyl)-r-1-cyclohexylcarbonitril
c-4-[-4'-Ethoxy-1,1'-biphenyl-4-yl]-1-(8-methacryloyloxyoctyl)-r-1-cyclohexylcarbonitril
c-4-[-4'-Propoxy-1,1'-biphenyl-4-yl]-1-(8-methacryloyloxyoctyl)-r-1-cyclohexylcarbonitril
c-4-[-4'-Butoxy-1,1'-biphenyl-4-yl]-1-(8-methacryloyloxyoctyl)-r-1-cyclohexylcarbonitril
c-4-[-4'-Pentyloxy-1,1'-biphenyl-4-yl]-1-(8-methacryloyloxyoctyl)-r-1-cyclohexylcarbonitril
c-4-[-4'-Hexyloxy-1,1'-biphenyl-4-yl]-1-(8-methacryloyloxyoctyl)-r-1-cyclohexylcarbonitril
c-4-[-4'-Heptyloxy-1,1'-biphenyl-4-yl]-1-(8-methacryloyloxyoctyl)-r-1-cyclohexylcarbonitril
c-4-[-4'-Octyloxy-1,1'-biphenyl-4-yl]-1-(8-methacryloyloxyoctyl)-r-1-cyclohexylcarbonitril
c-4-[-4'-Ethyl-1,1'-biphenyl-4-yl]-1-(8-methacryloyloxyoctyl)-r-1-cyclohexylcarbonitril
c-4-[-4'-Propyl-1,1'-biphenyl-4-yl]-1-(8-methacryloyloxyoctyl)-r-1-cyclohexylcarbonitril
c-4-[-4'-Butyl-1,1'-biphenyl-4-yl]-1-(8-methacryloyloxyoctyl)-r-1-cyclohexylcarbonitril
c-4-[-4'-Pentyl-1,1'-biphenyl-4-yl]-1-(8-methacryloyloxyoctyl)-r-1-cyclohexylcarbonitril
c-4-[-4'-Ethoxy-1,1'-biphenyl-4-yl]-1-(9-methacryloyloxynonyl)-r-1-cyclohexylcarbonitril
c-4-[-4'-Propoxy-1,1'-biphenyl-4-yl]-1-(9-methacryloyloxynonyl)-r-1-cyclohexylcarbonitril
c-4-[-4'-Butoxy-1,1'-biphenyl-4-yl]-1-(9-methacryloyloxynonyl)-r-1-cyclohexylcarbonitril
c-4-[-4'-Pentyloxy-1,1'-biphenyl-4-yl]-1-(9-methacryloyloxynonyl)-r-1-cyclohexylcarbonitril
c-4-[-4'-Hexyloxy-1,1'-biphenyl-4-yl]-1-(9-methacryloyloxynonyl)-r-1-cyclohexylcarbonitril
c-4-[-4'-Heptyloxy-1,1'-biphenyl-4-yl]-1-(9-methacryloyloxynonyl)-r-1-cyclohexylcarbonitril
c-4-[-4'-Octyloxy-1,1'-biphenyl-4-yl]-1-(9-methacryloyloxynonyl)-r-1-cyclohexylcarbonitril
c-4-[-4'-Ethyl-1,1'-biphenyl-4-yl]-1-(9-methacryloyloxynonyl)-r-1-cyclohexylcarbonitril
c-4-[-4'-Propyl-1,1'-biphenyl-4-yl]-1-(9-methacryloyloxynonyl)-r-1-cyclohexylcarbonitril
c-4-[-4'-Butyl-1,1'-biphenyl-4-yl]-1-(9-methacryloyloxynonyl)-r-1-cyclohexylcarbonitril
c-4-[-4'-Pentyl-1,1'-biphenyl-4-yl]-1-(9-methacryloyloxynonyl)-r-1-cyclohexylcarbonitril
c-4-[-4'-Ethoxy-1,1'-biphenyl-4-yl]-1-(10-methacryloyloxydecyl)-r-1-cyclohexylcarbonitril
c-4-[-4'-Propoxy-1,1'-biphenyl-4-yl]-1-(10-methacryloyloxydecyl)-r-1-cyclohexylcarbonitril
c-4-[-4'-Butoxy-1,1'-biphenyl-4-yl]-1-(10-methacryloyloxydecyl)-r-1-cyclohexylcarbonitril
c-4-[-4'-Pentyloxy-1,1'-biphenyl-4-yl]-1-(10-methacryloyloxydecyl)-r-1-cyclohexylcarbonitril
c-4-[-4'-Hexyloxy-1,1'-biphenyl-4-yl]-1-(10-methacryloyloxydecyl)-r-1-cyclohexylcarbonitril
c-4-[-4'-Heptyloxy-1,1'-biphenyl-4-yl]-1-(10-methacryloyloxydecyl)-r-1-cyclohexylcarbonitril
c-4-[-4'-Octyloxy-1,1'-biphenyl-4-yl]-1-(10-methacryloyloxydecyl)-r-1-cyclohexylcarbonitril
c-4-[-4'-Ethyl-1,1'-biphenyl-4-yl]-1-(10-methacryloyloxydecyl)-r-1-cyclohexylcarbonitril
c-4-[-4'-Propyl-1,1'-biphenyl-4-yl]-1-(10-methacryloyloxydecyl)-r-1-cyclohexylcarbonitril
c-4-[-4'-Butyl-1,1'-biphenyl-4-yl]-1-(10-methacryloyloxydecyl)-r-1-cyclohexylcarbonitril
c-4-[-4'-Pentyl-1,1'-biphenyl-4-yl]-1-(10-methacryloyloxydecyl)-r-1-cyclohexylcarbonitril
c-4-[-4'-Ethoxy-1,1'-biphenyl-4-yl]-1-(11-methacryloyloxyundecyl)-r-1-cyclohexylcarbonitril
c-4-[-4'-Propoxy-1,1'-biphenyl-4-yl]-1-(11-methacryloyloxyundecyl)-r-1-cyclohexylcarbonitril
c-4-[-4'-Butoxy-1,1'-biphenyl-4-yl]-1-(11-methacryloyloxyundecyl)-r-1-cyclohexylcarbonitril
c-4-[-4'-Pentyloxy-1,1'-biphenyl-4-yl]-1-(11-methacryloyloxyundecyl)-r-1-cyclohexylcarbonitril
c-4-[-4'-Hexyloxy-1,1'-biphenyl-4-yl]-1-(11-methacryloyloxyundecyl)-r-1-cyclohexylcarbonitril
c-4-[-4'-Heptyloxy-1,1'-biphenyl-4-yl]-1-(11-methacryloyloxyundecyl)-r-1-cyclohexylcarbonitril
c-4-[-4'-Octyloxy-1,1'-biphenyl-4-yl]-1-(11-methacryloyloxyundecyl)-r-1-cyclohexylcarbonitril
c-4-[-4'-Ethyl-1,1'-biphenyl-4-yl]-1-(11-methacryloyloxyundecyl)-r-1-cyclohexylcarbonitril
c-4-[-4'-Propyl-1,1'-biphenyl-4-yl]-1-(11-methacryloyloxyundecyl)-r-1-cyclohexylcarbonitril
c-4-[-4'-Butyl-1,1'-biphenyl-4-yl]-1-(11-methacryloyloxyundecyl)-r-1-cyclohexylcarbonitril
c-4-[-4'-Pentyl-1,1'-biphenyl-4-yl]-1-(11-methacryloyloxyundecyl)-r-1-cyclohexylcarbonitril
c-4-[-4'-(2-Methylbutyloxy)-1,1'-biphenyl-4-yl]-1-(11-methacryloyloxyundecyl)-r-1-cyclohexylcarbonitril, optisch aktiv
c-4-[-4'-(2-Methylbutyloxy)-1,1'-biphenyl-4-yl]-1-(10-methacryloyloxydecyl)-r-1-cyclohexylcarbonitril, optisch aktiv
c-4-[-4'-(2-Methylbutyloxy)-1,1'-biphenyl-4-yl]-1-(9-methacryloyloxynonyl)-r-1-cyclohexylcarbonitril, optisch aktiv

c-4-[-4'-(2-Methylbutyloxy)-1,1'-biphenyl-4-yl]-1-(8-methacryloyloxyoctyl)-r-1-cyclohexylcarbonitril, optisch aktiv
c-4-[-4'-(2-Methylbutyloxy)-1,1'-biphenyl-4-yl]-1-(7-methacryloyloxyheptyl)-r-1-cyclohexylcarbonitril, optisch aktiv
c-4-[-4'-(2-Octyloxy)-1,1'-biphenyl-4-yl]-1-(11-methacryloyloxyundecyl)-r-1-cyclohexylcarbonitril, optisch aktiv
c-4-[-4'-(2-Octyloxy)-1,1'-biphenyl-4-yl]-1-(10-methacryloyloxydecyl)-r-1-cyclohexylcarbonitril, optisch aktiv
c-4-[-4'-(2-Octyloxy)-1,1'-biphenyl-4-yl]-1-(9-methacryloyloxynonyl)-r-1-cyclohexylcarbonitril, optisch aktiv
c-4-[-4'-(2-Octyloxy)-1,1'-biphenyl-4-yl]-1-(8-methacryloyloxyoctyl)-r-1-cyclohexylcarbonitril, optisch aktiv
c-4-[-4'-(2-Octyloxy)-1,1'-biphenyl-4-yl]-1-(7-methacryloyloxyheptyl)-r-1-cyclohexylcarbonitril, optisch aktiv

Beispiel 7

Analog Beispiel 6 erhält man aus c-4-[4'-Octyloxy-1,1'-biphenyl-4-yl]-1-(4-hydroxybutyl)-r-1-cyclohexylcarbonitril und Acrylsäure das entsprechende c-4-[4'-Octyloxy-1,1'-biphenyl-4-yl]-1-(4-acryloyloxybutyl)-r-1-cyclohexylcarbonitril.

Analog werden hergestellt:

c-4-[-4'-Ethoxy-1,1'-biphenyl-4-yl]-1-(4-acryloyloxybutyl)-r-1-cyclohexylcarbonitril
c-4-[-4'-Propoxy-1,1'-biphenyl-4-yl]-1-(4-acryloyloxybutyl)-r-1-cyclohexylcarbonitril
c-4-[-4'-Butoxy-1,1'-biphenyl-4-yl]-1-(4-acryloyloxybutyl)-r-1-cyclohexylcarbonitril
c-4-[-4'-Pentyloxy-1,1'-biphenyl-4-yl]-1-(4-acryloyloxybutyl)-r-1-cyclohexylcarbonitril
c-4-[-4'-Hexyloxy-1,1'-biphenyl-4-yl]-1-(4-acryloyloxybutyl)-r-1-cyciohexylcarbonitril
c-4-[-4'-Heptyloxy-1,1'-biphenyl-4-yl]-1-(4-acryloyloxybutyl)-r-1-cyclohexylcarbonitril
c-4-[-4'-Ethyl-1,1'-biphenyl-4-yl]-1-(4-acryloyloxybutyl)-r-1-cyclohexylcarbonitril
c-4-[-4'-Propyl-1,1'-biphenyl-4-yl]-1-(4-acryloyloxybutyl)-r-1-cyclohexylcarbonitril
c-4-[-4'-Butyl-1,1'-biphenyl-4-yl]-1-(4-acryloyloxybutyl)-r-1-cyclohexylcarbonitril
c-4-[-4'-Pentyl-1,1'-biphenyl-4-yl]-1-(4-acryloyloxybutyl)-r-1-cyclohexylcarbonitril
c-4-[-4'-Ethoxy-1,1'-biphenyl-5-yl]-1-(5-acryloyloxypentyl)-r-1-cyclohexylcarbonitril
c-4-[-4'-Propoxy-1,1'-biphenyl-5-yl]-1-(5-acryloyloxypentyl)-r-1-cyclohexylcarbonitril
c-4-[-4'-Butoxy-1,1'-biphenyl-5-yl]-1-(5-acryloyloxypentyl)-r-1-cyclohexylcarbonitril
c-4-[-4'-Pentyloxy-1,1'-biphenyl-5-yl]-1-(5-acryloyloxypentyl)-r-1-cyclohexylcarbonitril
c-4-[-4'-Hexyloxy-1,1'-biphenyl-5-yl]-1-(5-acryloyloxypentyl)-r-1-cyclohexylcarbonitril
c-4-[-4'-Heptyloxy-1,1'-biphenyl-5-yl]-1-(5-acryloyloxypentyl)-r-1-cyclohexylcarbonitril
c-4-[-4'-Octyloxy-1,1'-biphenyl-5-yl]-1-(5-acryloyloxypentyl)-r-1-cyclohexylcarbonitril
c-4-[-4'-Ethyl-1,1'-biphenyl-5-yl]-1-(5-acryloyloxypentyl)-r-1-cyclohexylcarbonitril
c-4-[-4'-Propyl-1,1'-biphenyl-5-yl]-1-(5-acryloyloxypentyl)-r-1-cyclohexylcarbonitril
c-4-[-4'-Butyl-1,1'-biphenyl-5-yl]-1-(5-acryloyloxypentyl)-r-1-cyclohexylcarbonitril
c-4-[-4'-Pentyl-1,1'-biphenyl-5-yl]-1-(5-acryloyloxypentyl)-r-1-cyclohexylcarbonitril
c-4-[-4'-Ethoxy-1,1'-biphenyl-4-yl]-1-(6-acryloyloxyhexyl)-r-1-cyclohexylcarbonitril
c-4-[-4'-Propoxy-1,1'-biphenyl-4-yl]-1-(6-acryloyloxyhexyl)-r-1-cyclohexylcarbonitril
c-4-[-4'-Butoxy-1,1'-biphenyl-4-yl]-1-(6-acryloyloxyhexyl)-r-1-cyclohexylcarbonitril
c-4-[-4'-Pentyloxy-1,1'-biphenyl-4-yl]-1-(6-acryloyloxyhexyl)-r-1-cyclohexylcarbonitril
c-4-[-4'-Hexyloxy-1,1'-biphenyl-4-yl]-1-(6-acryloyloxyhexyl)-r-1-cyclohexylcarbonitril
c-4-[-4'-Heptyloxy-1,1'-biphenyl-4-yl]-1-(6-acryloyloxyhexyl)-r-1-cyclohexylcarbonitril
c-4-[-4'-Octyloxy-1,1'-biphenyl-4-yl[-1-(6-acryloyloxyhexyl)-r-1-cyclohexylcarbonitril
c-4-[-4'-Ethyl-1,1'-biphenyl-4-yl]-1-(6-acryloyloxyhexyl)-r-1-cyclohexylcarbonitril
c-4-[-4'-Propyl-1,1'-biphenyl-4-yl]-1-(6-acryloyloxyhexyl)-r-1-cyclohexylcarbonitril
c-4-[-4'-Butyl-1,1'-biphenyl-4-yl]-1-(6-acryloyloxyhexyl)-r-1-cyclohexylcarbonitril
c-4-[-4'-Pentyl-1,1'-biphenyl-4-yl]-1-(6-acryloyloxyhexyl)-r-1-cyclohexylcarbonitril
c-4-[-4'-Ethoxy-1,1'-biphenyl-4-yl]-1-(7-acryloyloxyheptyl)-r-1-cyclohexylcarbonitril
c-4-[-4'-Propoxy-1,1'-biphenyl-4-yl]-1-(7-acryloyloxyheptyl)-r-1-cyclohexylcarbonitril
c-4-[-4'-Butoxy-1,1'-biphenyl-4-yl]-1-(7-acryloyloxyheptyl)-r-1-cyclohexylcarbonitril
c-4-[-4'-Pentyloxy-1,1'-biphenyl-4-yl]-1-(7-acryloyloxyheptyl)-r-1-cyclohexylcarbonitril
c-4-[-4'-Hexyloxy-1,1'-biphenyl-4-yl]-1-(7-acryloyloxyheptyl)-r-1-cyclohexylcarbonitril
c-4-[-4'-Heptyloxy-1,1'-biphenyl-4-yl]-1-(7-acryloyloxyheptyl)-r-1-cyclohexylcarbonitril
c-4-[-4'-Octyloxy-1,1'-biphenyl-4-yl]-1-(7-acryloyloxyheptyl)-r-1-cyclohexylcarbonitril
c-4-[-4'-Ethyl-1,1'-biphenyl-4-yl]-1-(7-acryloyloxyheptyl)-r-1-cyclohexylcarbonitril
c-4-[-4'-Propyl-1,1'-biphenyl-4-yl]-1-(7-acryloyloxyheptyl)-r-1-cyclohexylcarbonitril

c-4-[-4'-Butyl-1,1'-biphenyl-4-yl]-1-(7-acryloyloxyheptyl)-r-1-cyclohexylcarbonitril
c-4-[-4'-Pentyl-1,1'-biphenyl-4-yl]-1-(7-acryloyloxyheptyl)-r-1-cyclohexylcarbonitril
c-4-[-4'-Ethoxy-1,1'-biphenyl-4-yl]-1-(8-acryloyloxyoctyl)-r-1-cyclohexylcarbonitril
c-4-[-4'-Propoxy-1,1'-biphenyl-4-yl]-1-(8-acryloyloxyoctyl)-r-1-cyclohexylcarbonitril
c-4-[-4'-Butoxy-1,1'-biphenyl-4-yl]-1-(8-acryloyloxyoctyl)-r-1-cyclohexylcarbonitril
c-4-[-4'-Pentyloxy-1,1'-biphenyl-4-yl]-1-(8-acryloyloxyoctyl)-r-1-cyclohexylcarbonitril
c-4-[-4'-Hexyloxy-1,1'-biphenyl-4-yl]-1-(8-acryloyloxyoctyl)-r-1-cyclohexylcarbonitril
c-4-[-4'-Heptyloxy-1,1'-biphenyl-4-yl]-1-(8-acryloyloxyoctyl)-r-1-cyclohexylcarbonitril
c-4-[-4'-Octyloxy-1,1'-biphenyl-4-yl]-1-(8-acryloyloxyoctyl)-r-1-cyclohexylcarbonitril
c-4-[-4'-Ethyl-1,1'-biphenyl-4-yl]-1-(8-acryloyloxyoctyl)-r-1-cyclohexylcarbonitril
c-4-[-4'-Propyl-1,1'-biphenyl-4-yl]-1-(8-acryloyloxyoctyl)-r-1-cyclohexylcarbonitril
c-4-[-4'-Butyl-1,1'-biphenyl-4-yl]-1-(8-acryloyloxyoctyl)-r-1-cyclohexylcarbonitril
c-4-[-4'-Pentyl-1,1'-biphenyl-4-yl]-1-(8-acryloyloxyoctyl)-r-1-cyclohexylcarbonitril
c-4-[-4'-Ethoxy-1,1'-biphenyl-4-yl]-1-(9-acryloyloxynonyl)-r-1-cyclohexylcarbonitril
c-4-[-4'-Propoxy-1,1'-biphenyl-4-yl]-1-(9-acryloyloxynonyl)-r-1-cyclohexylcarbonitril
c-4-[-4'-Butoxy-1,1'-biphenyl-4-yl]-1-(9-acryloyloxynonyl)-r-1-cyclohexylcarbonitril
c-4-[-4'-Pentyloxy-1,1'-biphenyl-4-yl]-1-(9-acryloyloxynonyl)-r-1-cyclohexylcarbonitril
c-4-[-4'-Hexyloxy-1,1'-biphenyl-4-yl]-1-(9-acryloyloxynonyl)-r-1-cyclohexylcarbonitril
c-4-[-4'-Heptyloxy-1,1'-biphenyl-4-yl]-1-(9-acryloyloxynonyl)-r-1-cyclohexylcarbonitril
c-4-[-4'-Octyloxy-1,1'-biphenyl-4-yl]-1-(9-acryloyloxynonyl)-r-1-cyclohexylcarbonitril
c-4-[-4'-Ethyl-1,1'-biphenyl-4-yl]-1-(9-acryloyloxynonyl)-r-1-cyclohexylcarbonitril
c-4-[-4'-Propyl-1,1'-biphenyl-4-yl]-1-(9-acryloyloxynonyl)-r-1-cyclohexylcarbonitril
c-4-[-4'-Butyl-1,1'-biphenyl-4-yl]-1-(9-acryloyloxynonyl)-r-1-cyclohexylcarbonitril
c-4-[-4'-Pentyl-1,1'-biphenyl-4-yl]-1-(9-acryloyloxynonyl)-r-1-cyclohexylcarbonitril
c-4-[-4'-Ethoxy-1,1'-biphenyl-4-yl]-1-(10-acryloyloxydecyl)-r-1-cyclohexylcarbonitril
c-4-[-4'-Propoxy-1,1'-biphenyl-4-yl]-1-(10-acryloyloxydecyl)-r-1-cyclohexylcarbonitril
c-4-[-4'-Butoxy-1,1'-biphenyl-4-yl]-1-(10-acryloyloxydecyl)-r-1-cyclohexylcarbonitril
c-4-[-4'-Pentyloxy-1,1'-biphenyl-4-yl]-1-(10-acryloyloxydecyl)-r-1-cyclohexylcarbonitril
c-4-[-4'-Hexyloxy-1,1'-biphenyl-4-yl]-1-(10-acryloyloxydecyl)-r-1-cyclohexylcarbonitril
c-4-[-4'-Heptyloxy-1,1'-biphenyl-4-yl]-1-(10-acryloyloxydecyl)-r-1-cyclohexylcarbonitril
c-4-[-4'-Octyloxy-1,1'-biphenyl-4-yl]-1-(10-acryloyloxydecyl)-r-1-cyclohexylcarbonitril
c-4-[-4'-Ethyl-1,1'-biphenyl-4-yl]-1-(10-acryloyloxydecyl)-r-1-cyclohexylcarbonitril
c-4-[-4'-Propyl-1,1'-biphenyl-4-yl]-1-(10-acryloyloxydecyl)-r-1-cyclohexylcarbonitril
c-4-[-4'-Butyl-1,1'-biphenyl-4-yl]-1-(10-acryloyloxydecyl)-r-1-cyclohexylcarbonitril
c-4-[-4'-Pentyl-1,1'-biphenyl-4-yl]-1-(10-acryloyloxydecyl)-r-1-cyclohexylcarbonitril
c-4-[-4'-Ethoxy-1,1'-biphenyl-4-yl]-1-(11-acryloyloxyundecyl)-r-1-cyclohexylcarbonitril
c-4-[-4'-Propoxy-1,1'-biphenyl-4-yl]-1-(11-acryloyloxyundecyl)-r-1-cyclohexylcarbonitril
c-4-[-4'-Butoxy-1,1'-biphenyl-4-yl]-1-(11-acryloyloxyundecyl)-r-1-cyclohexylcarbonitril
c-4-[-4'-Pentyloxy-1,1'-biphenyl-4-yl]-1-(11-acryloyloxyundecyl)-r-1-cyclohexylcarbonitril
c-4-[-4'-Hexyloxy-1,1'-biphenyl-4-yl]-1-(11-acryloyloxyundecyl)-r-1-cyclohexylcarbonitril
c-4-[-4'-Heptyloxy-1,1'-biphenyl-4-yl]-1-(11-acryloyloxyundecyl)-r-1-cyclohexylcarbonitril
c-4-[-4'-Octyloxy-1,1'-biphenyl-4-yl[-1-(11-acryloyloxyundecyl)-r-1-cyclohexylcarbonitril
c-4-[-4'-Ethyl-1,1'-biphenyl-4-yl]-1-(11-acryloyloxyundecyl)-r-1-cyclohexylcarbonitril
c-4-[-4'-Propyl-1,1'-biphenyl-4-yl]-1-(11-acryloyloxyundecyl)-r-1-cyclohexylcarbonitril
c-4-[-4'-Butyl-1,1'-biphenyl-4-yl]-1-(11-acryloyloxyundecyl)-r-1-cyclohexylcarbonitril
c-4-[-4'-Pentyl-1,1'-biphenyl-4-yl]-1-(11-acryloyloxyundecyl)-r-1-cyclohexylcarbonitril
c-4-[-4'-(2-Methylbutyloxy)-1,1'-biphenyl-4-yl]-1-(11-acryloyloxyundecyl)-r-1-cyclohexylcarbonitril, optisch aktiv
c-4-[-4'-(2-Methylbutyloxy)-1,1'-biphenyl-4-yl]-1-(10-acryloyloxydecyl)-r-1-cyclohexylcarbonitril, optisch aktiv
c-4-[-4'-(2-Methylbutyloxy)-1,1'-biphenyl-4-yl]-1-(9-acryloyloxynonyl)-r-1-cyclohexylcarbonitril, optisch aktiv
c-4-[-4'-(2-Methylbutyloxy)-1,1'-biphenyl-4-yl]-1-(8-acryloyloxyoctyl)-r-1-cyclohexylcarbonitril, optisch aktiv
c-4-[-4'-(2-Methylbutyloxy)-1,1'-biphenyl-4-yl]-1-(7-acryloyloxyheptyl)-r-1-cyclohexylcarbonitril, optisch aktiv
c-4-[-4'-(2-Octyloxy)-1,1'-biphenyl-4-yl]-1-(11-acryloyloxyundecyl)-r-1-cyclohexylcarbonitril, optisch aktiv
c-4-[-4'-(2-Octyloxy)-1,1'-biphenyl-4-yl]-1-(10-acryloyloxydecyl)-r-1-cyclohexylcarbonitril, optisch aktiv
c-4-[-4'-(2-Octyloxy)-1,1'-biphenyl-4-yl]-1-(9-acryloyloxynonyl)-r-1-cyclohexylcarbonitril, optisch aktiv
c-4-[-4'-(2-Octyloxy)-1,1'-biphenyl-4-yl]-1-(8-acryloyloxyoctyl)-r-1-cyclohexylcarbonitril, optisch aktiv
c-4-[-4'-(2-Octyloxy)-1,1'-biphenyl-4-yl]-1-(7-acryloyloxyheptyl)-r-1-cyclohexylcarbonitril, optisch aktiv

Beispiel 8

Man erhitzt ein Gemisch aus 3,0 g r-1-Cyan-c-4-(4'-hydroxy-1,1'-biphenyl-4-yl)-1-octylcyclohexan, 2,0 g 11-Bromundecanol, 0,7 g Kaliumhydroxid, 2 mg Kaliumiodid und 60 ml Ethanol 3 Tage zum Sieden. Das Reaktionsgemisch wird eingedampft, mit 100 ml $H_2O$ aufgeschlämmt, filtriert und nach Umkristallisation des Rückstandes erhält man c-4-[4'-(11-Hydroxyundecyloxy)-1,1'-biphenyl-4-yl]-1-octyl-r-1-cyclohexylcarbonitril.

Analog werden hergestellt:

c-4-[4'-(3-Hydroxypropyloxy)-1,1'-biphenyl-4-yl]-1-octyl-r-1-cyclohexylcarbonitril
c-4-[4'-(4-Hydroxybutyloxy)-1,1'-biphenyl-4-yl]-1-octyl-r-1-cyclohexylcarbonitril
c-4-[4'-(5-Hydroxypentyloxy)-1,1'-biphenyl-4-yl]-1-octyl-r-1-cyclohexylcarbonitril
c-4-[4'-(6-Hydroxyhexyloxy)-1,1'-biphenyl-4-yl]-1-octyl-r-1-cyclohexylcarbonitril
c-4-[4'-(7-Hydroxyheptyloxy)-1,1'-biphenyl-4-yl]-1-octyl-r-1-cyclohexylcarbonitril
c-4-[4'-(8-Hydroxyoctyloxy)-1,1'-biphenyl-4-yl]-1-octyl-r-1-cyalohexylcarbonitril
c-4-[4'-(9-Hydroxynonyloxy)-1,1'-biphenyl-4-yl]-1-octyl-r-1-cyclohexylcarbonitril
c-4-[4'-(10-Hydroxydecyloxy)-1,1'-biphenyl-4-yl]-1-octyl-r-1-cyclohexylcarbonitril
c-4-[4'-(5-Hydroxypentyloxy)-1,1'-biphenyl-4-yl]-1-heptyl-r-1-cyclohexylcarbonitril
c-4-[4'-(6-Hydroxyhexyloxy)-1,1'-biphenyl-4-yl]-1-heptyl-r-1-cyclohexylcarbonitril
c-4-[4'-(7-Hydroxyheptyloxy)-1,1'-biphenyl-4-yl]-1-heptyl-r-1-cyclohexylcarbonitril
c-4-[4'-(8-Hydroxyoctyloxy)-1,1'-biphenyl-4-yl]-1-heptyl-r-1-cyclohexylcarbonitril
c-4-[4'-(9-Hydroxynonyloxy)-1,1'-biphenyl-4-yl]-1-heptyl-r-1-cyclohexylcarbonitril
c-4-[4'-(10-Hydroxydecyloxy)-1,1'-biphenyl-4-yl]-1-heptyl-r-1-cyclohexylcarbonitril
c-4-[4'-(11-Hydroxyundecyloxy)-1,1'-biphenyl-4-yl]-1-heptyl-r-1-cyclohexylcarbonitril
c-4-[4'-(6-Hydroxyhexyloxy)-1,1'-biphenyl-4-yl]-1-hexyl-r-1-cyclohexylcarbonitril
c-4-[4'-(7-Hydroxyheptyloxy)-1,1'-biphenyl-4-yl]-1-hexyl-r-1-cyclohexylcarbonitril
c-4-[4'-(8-Hydroxyoctyloxy)-1,1'-biphenyl-4-yl]-1-hexyl-r-1-cyclohexylcarbonitril
c-4-[4'-(9-Hydroxynonyloxy)-1,1'-biphenyl-4-yl]-1-hexyl-r-1-cyclohexylcarbonitril
c-4-[4'-(10-Hydroxydecyloxy)-1,1'-biphenyl-4-yl]-1-hexyl-r-1-cyclohexylcarbonitril
c-4-[4'-(11-Hydroxyundecyloxy)-1,1'-biphenyl-4-yl]-1-hexyl-r-1-cyclohexylcarbonitril

Beispiel 9

Man erhitzt ein Gemisch aus 2,3 g c-4-[4'-11-Hydroxyundecyloxy)-1,1'-biphenyl-4-yl]-1-octyl-r-1-cyclohexylcarbonitril, 4,8 g Methacrylsäure, 0,2 g Hydrochinon, 0,2 g p-Toluolsulfonsäuremonohydrat und 60 ml Chloroform 3 Tage am Wasserabscheider. Die Reaktionslösung wird gewaschen, getrocknet und eingedampft. Nach Chromatographie des Rückstandes an Kieselgel mit Dichlormethan/Methanol (98:2) erhält man c-4-[4'-(11-Methacryloyloxyundecyloxy)-1,1'-biphenyl-4-yl]-1-octyl-r-1-cyclohexylcarbonitril mit K 49° $S_C$ 71° $S_A$ 106° N 108° I.

Analog werden hergestellt:

c-4-[4'-(3-Methacryloyloxypropyloxy)-1,1'-biphenyl-4-yl]-1-octyl-r-1-cyalohexylcarbonitril
c-4-[4'-(4-Methacryloyloxybutyloxy)-1,1'-biphenyl-4-yl]-1-octyl-r-1-cyclohexylcarbonitril
c-4-[4'-(5-Methacryloyloxypentyloxy)-1,1'-biphenyl-4-yl]-1-octyl-r-1-cyclohexylcarbonitril
c-4-[4'-(6-Methacryloyloxyhexyloxy)-1,1'-biphenyl-4-yl]-1-oatyl-r-1-cyclohexylcarbonitril
c-4-[4'-(7-Methacryloyloxyheptyloxy)-1,1'-biphenyl-4-yl]-1-octyl-r-1-cyclohexylcarbonitril
c-4-[4'-(8-Methacryloyloxyoctyloxy)-1,1'-biphenyl-4-yl]-1-octyl-r-1-cyclohexylcarbonitril
c-4-[4'-(9-Methacryloyloxynonyloxy)-1,1'-biphenyl-4-yl]-1-octyl-r-1-cyclohexylcarbonitril
c-4-[4'-(10-Methacryloyloxydecyloxy)-1,1'-biphenyl-4-yl]-1-octyl-r-1-cyclohexylcarbonitril
c-4-[4'-(5-Methacryloyloxypentyloxy)-1,1'-biphenyl-4-yl]-1-heptyl-r-1-cyclohexylcarbonitril
c-4-[4'-(6-Methacryloyloxyhexyloxy)-1,1'-biphenyl-4-yl]-1-heptyl-r-1-cyclohexylcarbonitril
c-4-[4'-(7-Methacryloyloxyheptyloxy)-1,1'-biphenyl-4-yl]-1-heptyl-r-1-cyclohexylcarbonitril
c-4-[4'-(8-Methacryloyloxyoctyloxy)-1,1'-biphenyl-4-yl]-1-heptyl-r-1-cyclohexylcarbonitril
c-4-[4'-(9-Methacryloyloxynonyloxy)-1,1'-biphenyl-4-yl]-1-heptyl-r-1-cyclohexylcarbonitril
c-4-[4'-(10-Methacryloyloxydecyloxy)-1,1'-biphenyl-4-yl]-1-heptyl-r-1-cyclohexylcarbonitril
c-4-[4'-(11-Methacryloyloxyundecyloxy)-1,1'-biphenyl-4-yl]-1-heptyl-r-1-cyclohexylcarbonitril
c-4-[4'-(5-Methacryloyloxypentyloxy)-1,1'-biphenyl-4-yl]-1-hexyl-r-1-cyclohexylcarbonitril
c-4-[4'-(6-Methacryloyloxyhexyloxy)-1,1'-biphenyl-4-yl]-1-hexyl-r-1-cyclohexylcarbonitril
c-4-[4'-(7-Methacryloyloxyheptyloxy)-1,1'-biphenyl-4-yl]-1-hexyl-r-1-cyclohexylcarbonitril
c-4-[4'-(8-Methacryloyloxyoctyloxy)-1,1'-biphenyl-4-yl]-1-hexyl-r-1-cyclohexylcarbonitril
c-4-[4'-(9-Methacryloyloxynonyloxy)-1,1'-biphenyl-4-yl]-1-hexyl-r-1-cyclohexylcarbonitril

c-4-[4'-(10-Methacryloyloxydecyloxy)-1,1'-biphenyl-4-yl]-1-hexyl-r-1-cyclohexylcarbonitril
c-4-[4'-(11-Methacryloyloxyundecyloxy)-1,1'-biphenyl-4-yl]-1-hexyl-r-1-cyclohexylcarbonitril

Beispiel 10

Analog Beispiel 9 erhält man durch Umsetzung von c-4-[4'-(11-Hydroxyundecyloxy)-1,1'-biphenyl-4-yl]-1-octyl-r-1-cyclohexylcarbonitril mit Acrylsäure das entsprechende c-4-[4'-(11-Acryloyloxyundecyloxy)-1,1'-biphenyl-4-yl]-1-octyl-r-1-cyclohexylcarbonitril.

Analog werden hergestellt:
c-4-[4'-(3-Acryloyloxypropyloxy)-1,1'-biphenyl-4-yl]-1-octyl-r-1-cyclohexylcarbonitril
c-4-[4'-(4-Acryloyloxybutyloxy)-1,1'-biphenyl-4-yl]-1-octyl-r-1-cyclohexylcarbonitril
c-4-[4'-(5-Acryloyloxypentyloxy)-1,1'-biphenyl-4-yl]-1-octyl-r-1-cyclohexylcarbonitril
c-4-[4'-(6-Acryloyloxyhexyloxy)-1,1'-biphenyl-4-yl]-1-octyl-r-1-cyclohexylcarbonitril
c-4-[4'-(7-Acryloyloxyheptyloxy)-1,1'-biphenyl-4-yl]-1-octyl-r-1-cyclohexylcarbonitril
c-4-[4'-(8-Acryloyloxyoctyloxy)-1,1'-biphenyl-4-yl]-1-octyl-r-1-cyclohexylcarbonitril
c-4-[4'-(9-Acryloyloxynonyloxy)-1,1'-biphenyl-4-yl]-1-octyl-r-1-cyclohexylcarbonitril
c-4-[4'-(10-Acryloyloxydecyloxy)-1,1'-biphenyl-4-yl]-1-octyl-r-1-cyclohexylcarbonitril
c-4-[4'-(5-Acryloyloxypentyloxy)-1,1'-biphenyl-4-yl]-1-heptyl-r-1-cyclohexylcarbonitril
c-4-[4'-(6-Acryloyloxyhexyloxy)-1,1'-biphenyl-4-yl]-1-heptyl-r-1-cyclohexylcarbonitril
c-4-[4'-(7-Acryloyloxyheptyloxy)-1,1'-biphenyl-4-yl]-1-heptyl-r-1-cyclohexylcarbonitril
c-4-[4'-(8-Acryloyloxyoctyloxy)-1,1'-biphenyl-4-yl]-1-heptyl-r-1-cyclohexylcarbonitril
c-4-[4'-(9-Acryloyloxynonyloxy)-1,1'-biphenyl-4-yl]-1-heptyl-r-1-cyclohexylcarbonitril
c-4-[4'-(10-Acryloyloxydecyloxy)-1,1'-biphenyl-4-yl]-1-heptyl-r-1-cyclohexylcarbonitril
c-4-[4'-(11-Acryloyloxyundecyloxy)-1,1'-biphenyl-4-yl]-1-heptyl-r-1-cyclohexylcarbonitril
c-4-[4'-(6-Acryloyloxyhexyloxy)-1,1'-biphenyl-4-yl]-1-pentyl-r-1-cyclohexylcarbonitril
c-4-[4'-(7-Acryloyloxyheptyloxy)-1,1'-biphenyl-4-yl]-1-pentyl-r-1-cyclohexylcarbonitril
c-4-[4'-(8-Acryloyloxyoctyloxy)-1,1'-biphenyl-4-yl]-1-pentyl-r-1-cyclohexylcarbonitril
c-4-[4'-(9-Acryloyloxynonyloxy)-1,1'-biphenyl-4-yl]-1-pentyl-r-1-cyclohexylcarbonitril
c-4-[4'-(10-Acryloyloxydecyloxy)-1,1'-biphenyl-4-yl]-1-pentyl-r-1-cyclohexylcarbonitril
c-4-[4'-(11-Acryloyloxyundecyloxy)-1,1'-biphenyl-4-yl]-1-pentyl-r-1-cyclohexylcarbonitril

Beispiel 11

a) Man gibt zu einer Lösung von 46,5 g Diisopropylamin in 400 ml THF bei 0° unter Rühren 275 ml einer 1,6 molaren Lösung von n-Butyllithium in Hexan. Anschließend gibt man diese Lösung bei -70° zu einem Gemisch aus 30,6 g 4-Cyancylohexylcarbonsäure in THF und rührt 2 Stunden, gibt dann 39,1 g 1-Brom-5-hexen zu und läßt die Mischung auf 0° kommen. Man gibt 35 ml Ethanol zu, gießt die Mischung auf Eis, extrahiert mit tert.Butylmethylether, säuert die wäßrige Phase mit 2 N HCl an und extrahiert erneut. Die organische Phase wird aufgearbeitet und nach Umkristallisation aus Ethanol erhält man c-4-Cyano-t-4-(5-hexenyl)-r-1-cyclohexylcarbonsäure.

b) Man gibt zu einer Mischung aus 16,5 g nach a) erhaltenen Carbonsäure, 20,9 g 4-Hydroxy-4'-octyloxy-1,1'-biphenyl, 855 mg 4-Dimethylaminopyridin und 80 ml $CH_2Cl_2$ bei 0° eine Lösung von 14,8 g DCCI in 10 ml $CH_2Cl_2$ und rührt 18 Sunden bei Raumtemperatur. Der Niederschlag wird abgetrennt, das Filtrat aufgearbeitet und nach Umkristallisation erhält man (4'-Octyloxy-1,1'-biphenyl-4-yl)-c-4-cyano-t-(5-hexenyl)-r-1-cyclohexylcarboxylat.

Analog werden hergestellt:
(4'-Heptyloxy-1,1'-biphenyl-4-yl)-c-4-cyano-t-4-(5-hexenyl)-r-1-cyclohexylcarboxylat
(4'-Hexyloxy-1,1'-biphenyl-4-yl)-c-4-cyano-t-4-(5-hexenyl)-r-1-cyclohexylcarboxylat
(4'-Pentyloxy-1,1'-biphenyl-4-yl)-c-4-cyano-t-4-(5-hexenyl)-r-1-cyclohexylcarboxylat
(4'-Butyloxy-1,1'-biphenyl-4-yl)-c-4-cyano-t-4-(5-hexenyl)-r-1-cyclohexylcarboxylat
(4'-Propoxy-1,1'-biphenyl-4-yl)-c-4-cyano-t-4-(5-hexenyl)-r-1-cyclohexylcarboxylat
(4'-Pentyl-1,1'-biphenyl-4-yl)-c-4-cyano-t-4-(5-hexenyl)-r-1-cyclohexylcarboxylat
(4'-Hexyl-1,1'-biphenyl-4-yl)-c-4-cyano-t-4-(5-hexenyl)-r-1-cyclohexylcarboxylat
(4'-Heptyl-1,1'-biphenyl-4-yl)-c-4-cyano-t-4-(5-hexenyl)-r-1-cyclohexylcarboxylat
(4'-Octyloxy-1,1'-biphenyl-4-yl)-c-4-cyano-t-4-(6-heptenyl)-r-1-cyclohexylcarboxylat
(4'-Heptyloxy-1,1'-biphenyl-4-yl)-c-4-cyano-t-4-(6-heptenyl)-r-1-cyclohexylcarboxylat
(4'-Hexyloxy-1,1'-biphenyl-4-yl)-c-4-cyano-t-4-(6-heptenyl)-r-1-cyclohexylcarboxylat
(4'-Pentyloxy-1,1'-biphenyl-4-yl)-c-4-cyano-t-4-(6-heptenyl)-r-1-cyclohexylcarboxylat

(4'-Butyloxy-1,1'-biphenyl-4-yl)-c-4-cyano-t-4-(6-heptenyl)-r-1-cyclohexylcarboxylat
(4'-Propoxy-1,1'-biphenyl-4-yl)-c-4-cyano-t-4-(6-heptenyl)-r-1-cyclohexylcarboxylat
(4'-Pentyl-1,1'-biphenyl-4-yl)-c-4-cyano-t-4-(6-heptenyl)-r-1-cyclohexylcarboxylat
(4'-Hexyl-1,1'-biphenyl-4-yl)-c-4-cyano-t-4-(6-heptenyl)-r-1-cyclohexylcarboxylat
(4'-Heptyl-1,1'-biphenyl-4-yl)-c-4-cyano-t-4-(6-heptenyl)-r-1-cyclohexylcarboxylat
(4'-Octyloxy-1,1'-biphenyl-4-yl)-c-4-cyano-t-4-(7-octenyl)-r-1-cyclohexylcarboxylat
(4'-Heptyloxy-1,1'-biphenyl-4-yl)-c-4-cyano-t-4-(7-octenyl)-r-1-cyclohexylcarboxylat
(4'-Hexyloxy-1,1'-biphenyl-4-yl)-c-4-cyano-t-4-(7-octenyl)-r-1-cyclohexylcarboxylat
(4'-Pentyloxy-1,1'-biphenyl-4-yl)-c-4-cyano-t-4-(7-octenyl)-r-1-cyclohexylcarboxylat
(4'-Butyloxy-1,1'-biphenyl-4-yl)-c-4-cyano-t-4-(7-octenyl)-r-1-cyclohexylcarboxylat
(4'-Propoxy-1,1'-biphenyl-4-yl)-c-4-cyano-t-4-(7-octenyl)-r-1-cyclohexylcarboxylat
(4'-Pentyl-1,1'-biphenyl-4-yl)-c-4-cyano-t-4-(7-octenyl)-r-1-cyclohexylcarboxylat
(4'-Hexyl-1,1'-biphenyl-4-yl)-c-4-cyano-t-4-(7-octenyl)-r-1-cyclohexylcarboxylat
(4'-Heptyl-1,1'-biphenyl-4-yl)-c-4-cyano-t-4-(7-octenyl)-r-1-cyclohexylcarboxylat
(4'-Octyloxy-1,1'-biphenyl-4-yl)-c-4-cyano-t-4-(8-nonenyl)-r-1-cyclohexylcarboxylat
(4'-Heptyloxy-1,1'-biphenyl-4-yl)-c-4-cyano-t-4-(8-nonenyl)-r-1-cyclohexylcarboxylat
(4'-Hexyloxy-1,1'-biphenyl-4-yl)-c-4-cyano-t-4-(8-nonenyl)-r-1-cyclohexylcarboxylat
(4'-Pentyloxy-1,1'-biphenyl-4-yl)-c-4-cyano-t-4-(8-nonenyl)-r-1-cyclohexylcarboxylat
(4'-Butyloxy-1,1'-biphenyl-4-yl)-c-4-cyano-t-4-(8-nonenyl)-r-1-cyclohexylcarboxylat
(4'-Propoxy-1,1'-biphenyl-4-yl)-c-4-cyano-t-4-(8-nonenyl)-r-1-cyclohexylcarboxylat
(4'-Pentyl-1,1'-biphenyl-4-yl)-c-4-cyano-t-4-(8-nonenyl)-r-1-cyclohexylcarboxylat
(4'-Hexyl-1,1'-biphenyl-4-yl)-c-4-cyano-t-4-(8-nonenyl)-r-1-cyclohexylcarboxylat
(4'-Heptyl-1,1'-biphenyl-4-yl)-c-4-cyano-t-4-(8-nonenyl)-r-1-cyclohexylcarboxylat
(4'-Octyloxy-1,1'-biphenyl-4-yl)-c-4-cyano-t-4-(9-decenyl)-r-1-cyclohexylcarboxylat
(4'-Heptyloxy-1,1'-biphenyl-4-yl)-c-4-cyano-t-4-(9-decenyl)-r-1-cyclohexylcarboxylat
(4'-Hexyloxy-1,1'-biphenyl-4-yl)-c-4-cyano-t-4-(9-decenyl)-r-1-cyclohexylcarboxylat
(4'-Pentyloxy-1,1'-biphenyl-4-yl)-c-4-cyano-t-4-(9-decenyl)-r-1-cyclohexylcarboxylat
(4'-Butyloxy-1,1'-biphenyl-4-yl)-c-4-cyano-t-4-(9-decenyl)-r-1-cyclohexylcarboxylat
(4'-Propoxy-1,1'-biphenyl-4-yl)-c-4-cyano-t-4-(9-decenyl)-r-1-cyclohexylcarboxylat
(4'-Pentyl-1,1'-biphenyl-4-yl)-c-4-cyano-t-4-(9-decenyl)-r-1-cyclohexylcarboxylat
(4'-Hexyl-1,1'-biphenyl-4-yl)-c-4-cyano-t-4-(9-decenyl)-r-1-cyclohexylcarboxylat
(4'-Heptyl-1,1'-biphenyl-4-yl)-c-4-cyano-t-4-(9-decenyl)-r-1-cyclohexylcarboxylat
(4'-Octyloxy-1,1'-biphenyl-4-yl)-c-4-cyano-t-4-(10-undecenyl)-r-1-cyclohexylcarboxylat
(4'-Heptyloxy-1,1'-biphenyl-4-yl)-c-4-cyano-t-4-(10-undecenyl)-r-1-cyclohexylcarboxylat
(4'-Hexyloxy-1,1'-biphenyl-4-yl)-c-4-cyano-t-4-(10-undecenyl)-r-1-cyclohexylcarboxylat
(4'-Pentyloxy-1,1'-biphenyl-4-yl)-c-4-cyano-t-4-(10-undecenyl)-r-1-cyclohexylcarboxylat
(4'-Butyloxy-1,1'-biphenyl-4-yl)-c-4-cyano-t-4-(10-undecenyl)-r-1-cyclohexylcarboxylat
(4'-Propoxy-1,1'-biphenyl-4-yl)-c-4-cyano-t-4-(10-undecenyl)-r-1-cyclohexylcarboxylat
(4'-Pentyl-1,1'-biphenyl-4-yl)-c-4-cyano-t-4-(10-undecenyl)-r-1-cyclohexylcarboxylat
(4'-Hexyl-1,1'-biphenyl-4-yl)-c-4-cyano-t-4-(10-undecenyl)-r-1-cyclohexylcarboxylat
(4'-Heptyl-1,1'-biphenyl-4-yl)-c-4-cyano-t-4-(10-undecenyl)-r-1-cyclohexylcarboxylat

Beispiel 12

Analog Beispiel 5b) erhält man aus [4'-Octyloxy-1,1'-biphenyl-4-yl]-c-4-cyano-t-4-hexen-5-yl-r-1-cyclohexylcarboxylat den entsprechenden Alkohol [4'-Octyloxy-1,1'-biphenyl-4-yl]-c-4-cyano-t-4-(6-hydroxyhexyl)-r-1-cyclohexylcarboxylat.

Analog werden hergestellt:
[4'-Heptyloxy-1,1'-biphenyl-4-yl]-c-4-cyano-t-4-(6-hydroxyhexyl)-r-1-cyclohexylcarboxylat
[4'-Hexyloxy-1,1'-biphenyl-4-yl]-c-4-cyano-t-4-(6-hydroxyhexyl)-r-1-cyclohexylcarboxylat
[4'-Pentyloxy-1,1'-biphenyl-4-yl]-c-4-cyano-t-4-(6-hydroxyhexyl)-r-1-cyclohexylcarboxylat
[4'-Butyloxy-1,1'-biphenyl-4-yl]-c-4-cyano-t-4-(6-hydroxyhexyl)-r-1-cyclohexylcarboxylat
[4'-Propoxy-1,1'-biphenyl-4-yl]-c-4-cyano-t-4-(6-hydroxyhexyl)-r-1-cyclohexylcarboxylat
[4'-Pentyl-1,1'-biphenyl-4-yl]-c-4-cyano-t-4-(6-hydroxyhexyl)-r-1-cyclohexylcarboxylat
[4'-Hexyl-1,1'-biphenyl-4-yl]-c-4-cyano-t-4-(6-hydroxyhexyl)-r-1-cyclohexylcarboxylat
[4'-Heptyl-1,1'-biphenyl-4-yl]-c-4-cyano-t-4-(6-hydroyhexyl)-r-1-cyclohexylcarboxylat
[4'-Octyloxy-1,1'-biphenyl-4-yl]-c-4-cyano-t-4-(7-hydroxyheptyl)-r-1-cyclohexylcarboxylat
[4'-Heptyloxy-1,1'-biphenyl-4-yl]-c-4-cyano-t-4-(7-hydroxyheptyl)-r-1-cyclohexylcarboxylat

[4'-Hexyloxy-1,1'-biphenyl-4-yl]-c-4-cyano-t-4-(7-hydroxyheptyl)-r-1-cyclohexylcarboxylat
[4'-Pentyloxy-1,1'-biphenyl-4-yl]-c-4-cyano-t-4-(7-hydroxyheptyl)-r-1-cyclohexylcarboxylat
[4'-Butyloxy-1,1'-biphenyl-4-yl]-c-4-cyano-t-4-(7-hydroxyheptyl)-r-1-cyclohexylcarboxylat
[4'-Propoxy-1,1'-biphenyl-4-yl]-c-4-cyano-t-4-(7-hydroxyheptyl)-r-1-cyclohexylcarboxylat
[4'-Pentyl-1,1'-biphenyl-4-yl]-c-4-cyano-t-4-(7-hydroxyheptyl)-r-1-cyclohexylcarboxylat
[4'-Hexyl-1,1'-biphenyl-4-yl]-c-4-cyano-t-4-(7-hydroxyheptyl)-r-1-cyclohexylcarboxylat
[4'-Heptyl-1,1'-biphenyl-4-yl]-c-4-cyano-t-4-(7-hydroxyheptyl)-r-1-cyclohexylcarboxylat
[4'-Octyloxy-1,1'-biphenyl-4-yl]-c-4-cyano-t-4-(8-hydroxyoctyl)-r-1-cyclohexylcarboxylat
[4'-Heptyloxy-1,1'-biphenyl-4-yl]-c-4-cyano-t-4-(8-hydroxyoctyl)-r-1-cyclohexylcarboxylat
[4'-Hexyloxy-1,1'-biphenyl-4-yl]-c-4-cyano-t-4-(8-hydroxyoctyl)-r-1-cyclohexylcarboxylat
[4'-Pentyloxy-1,1'-biphenyl-4-yl]-c-4-cyano-t-4-(8-hydroxyoctyl)-r-1-cyclohexylcarboxylat
[4'-Butyloxy-1,1'-biphenyl-4-yl]-c-4-cyano-t-4-(8-hydroxyoctyl)-r-1-cyclohexylcarboxylat
[4'-Propoxy-1,1'-biphenyl-4-yl]-c-4-cyano-t-4-(8-hydroxyoctyl)-r-1-cyclohexylcarboxylat
[4'-Pentyl-1,1'-biphenyl-4-yl]-c-4-cyano-t-4-(8-hydroxyoctyl)-r-1-cyclohexylcarboxylat
[4'-Hexyl-1,1'-biphenyl-4-yl]-c-4-cyano-t-4-(8-hydroxyoctyl)-r-1-cyclohexylcarboxylat
[4'-Heptyl-1,1'-biphenyl-4-yl]-c-4-cyano-t-4-(8-hydroxyoctyl)-r-1-cyclohexylcarboxylat
[4'-Octyloxy-1,1'-biphenyl-4-yl]-c-4-cyano-t-4-(9-hydroxynonyl)-r-1-cyclohexylcarboxylat
[4'-Heptyloxy-1,1'-biphenyl-4-yl]-c-4-cyano-t-4-(9-hydroxynonyl)-r-1-cyclohexylcarboxylat
[4'-Hexyloxy-1,1'-biphenyl-4-yl]-c-4-cyano-t-4-(9-hydroxynonyl)-r-1-cyclohexylcarboxylat
[4'-Pentyloxy-1,1'-biphenyl-4-yl]-c-4-cyano-t-4-(9-hydroxynonyl)-r-1-cyclohexylcarboxylat
[4'-Butyloxy-1,1'-biphenyl-4-yl]-c-4-cyano-t-4-(9-hydroxynonyl)-r-1-cyclohexylcarboxylat
[4'-Propoxy-1,1'-biphenyl-4-yl]-c-4-cyano-t-4-(9-hydroxynonyl)-r-1-cyclohexylcarboxylat
[4'-Pentyl-1,1'-biphenyl-4-yl]-c-4-cyano-t-4-(9-hydroxynonyl)-r-1-cyclohexylcarboxylat
[4'-Hexyl-1,1'-biphenyl-4-yl]-c-4-cyano-t-4-(9-hydroxynonyl)-r-1-cyclohexylcarboxylat
[4'-Heptyl-1,1'-biphenyl-4-yl]-c-4-cyano-t-4-(9-hydroxynonyl)-r-1-cyclohexylcarboxylat
[4'-Octyloxy-1,1'-biphenyl-4-yl]-c-4-cyano-t-4-(10-hydroxydecyl)-r-1-cyclohexylcarboxylat
[4'-Heptyloxy-1,1'-biphenyl-4-yl]-c-4-cyano-t-4-(10-hydroxydecyl)-r-1-cyclohexylcarboxylat
[4'-Hexyloxy-1,1'-biphenyl-4-yl]-c-4-cyano-t-4-(10-hydroxydecyl)-r-1-cyclohexylcarboxylat
[4'-Pentyloxy-1,1'-biphenyl-4-yl]-c-4-cyano-t-4-(10-hydroxydecyl)-r-1-cyclohexylcarboxylat
[4'-Butyloxy-1,1'-biphenyl-4-yl]-c-4-cyano-t-4-(10-hydroxydecyl)-r-1-cyclohexylcarboxylat
[4'-Propoxy-1,1'-biphenyl-4-yl]-c-4-cyano-t-4-(10-hydroxydecyl)-r-1-cyclohexylcarboxylat
[4'-Pentyl-1,1'-biphenyl-4-yl]-c-4-cyano-t-4-(10-hydroxydecyl)-r-1-cyclohexylcarboxylat
[4'-Hexyl-1,1'-biphenyl-4-yl]-c-4-cyano-t-4-(10-hydroxydecyl)-r-1-cyclohexylcarboxylat
[4'-Heptyl-1,1'-biphenyl-4-yl]-c-4-cyano-t-4-(10-hydroxydecyl)-r-1-cyclohexylcarboxylat
[4'-Octyloxy-1,1'-biphenyl-4-yl]-c-4-cyano-t-4-(11-hydroxyundecyl)-r-1-cyclohexylcarboxylat
[4'-Heptyloxy-1,1'-biphenyl-4-yl]-c-4-cyano-t-4-(11-hydroxyundecyl)-r-1-cyclohexylcarboxylat
[4'-Hexyloxy-1,1'-biphenyl-4-yl]-c-4-cyano-t-4-(11-hydroxyundecyl)-r-1-cyclohexylcarboxylat
[4'-Pentyloxy-1,1'-biphenyl-4-yl]-c-4-cyano-t-4-(11-hydroxyundecyl)-r-1-cyclohexylcarboxylat
[4'-Butyloxy-1,1'-biphenyl-4-yl]-c-4-cyano-t-4-(11-hydroxyundecyl)-r-1-cyclohexylcarboxylat
[4'-Propoxy-1,1'-biphenyl-4-yl]-c-4-cyano-t-4-(11-hydroxyundecyl)-r-1-cyclohexylcarboxylat
[4'-Pentyl-1,1'-biphenyl-4-yl]-c-4-cyano-t-4-(11-hydroxyundecyl)-r-1-cyclohexylcarboxylat
[4'-Hexyl-1,1'-biphenyl-4-yl]-c-4-cyano-t-4-(11-hydroxyundecyl)-r-1-cyclohexylcarboxylat
[4'-Heptyl-1,1'-biphenyl-4-yl]-c-4-cyano-t-4-(11-hydroxyundecyl)-r-1-cyclohexylcarboxylat

## Beispiel 13

Analog Beispiel 6 erhält man durch Umsetzung von [4'-Octyloxy-1,1'-biphenyl-4-yl]-c-4-cyano-t-4-(6-hydroxyhexyl)-r-1-cyclohexylcarboxylat mit Methacrylsäure in Gegenwart von DCCI das entsprechende [4'-Octyloxy-1,1'-biphenyl-4-yl]-c-4-cyano-t-4-(6-methacryloyloxyhexyl)r-1-cyclohexylcarboxylat.

Analog werden hergestellt:
[4'-Heptyloxy-1,1'-biphenyl-4-yl]-c-4-cyano-t-4-(6 methacryloyloxyhexyl)r-1-cyclohexylcarboxylat
[4'-Hexyloxy-1,1'-biphenyl-4-yl]-c-4-cyano-t-4-(6-methacryloyloxyhexyl)-r-1-cyclohexylcarboxylat
[4'-Pentyloxy-1,1'-biphenyl-4-yl]-c-4-cyano-t-4-(6-methacryloyloxyhexyl)-r-1-cyclohexylcarboxylat
[4'-Butyloxy-1,1'-biphenyl-4-yl]-c-4-cyano-t-4-(6-methacryloyloxyhexyl)-r-1-cyclohexylcarboxylat
[4'-Propoxy-1,1'-biphenyl-4-yl]-c-4-cyano-t-4-(6-methacryloyloxyhexyl)-r-1-cyclohexylcarboxylat
[4'-Pentyl-1,1'-biphenyl-4-yl]-c-4-cyano-t-4-(6-methacryloyloxyhexyl)-r-1-cyclohexylcarboxylat
[4'-Hexyl-1,1'-biphenyl-4-yl]-c-4-cyano-t-4-(6-methacryloyloxyhexyl)-r-1-cyclohexylcarboxylat
[4'-Heptyl-1,1'-biphenyl-4-yl]-c-4-cyano-t-4-(6-methacryloyloxyhexyl)-r-1-cyclohexylcarboxylat

[4'-Octyloxy-1,1'-biphenyl-4-yl]-c-4-cyano-t-4-(7-methacryloyloxyheptyl)-r-1-cyclohexylcarboxylat
[4'-Heptyloxy-1,1'-biphenyl-4-yl]-c-4-cyano-t-4-(7-methacryloyloxyheptyl)-r-1-cyclohexylcarboxylat
[4'-Hexyloxy-1,1'-biphenyl-4-yl]-c-4-cyano-t-4-(7-methacryloyloxyheptyl)-r-1-cyclohexylcarboxylat
[4'-Pentyloxy-1,1'-biphenyl-4-yl]-c-4-cyano-t-4-(7-methacryloyloxyheptyl)-r-1-cyclohexylcarboxylat
[4'-Butyloxy-1,1'-biphenyl-4-yl]-c-4-cyano-t-4-(7-methacryloyloxyheptyl)-r-1-cyclohexylcarboxylat
[4'-Propoxy-1,1'-biphenyl-4-yl]-c-4-cyano-t-4-(7-methacryloyloxyheptyl)-r-1-cyclohexylcarboxylat
[4'-Pentyl-1,1'-biphenyl-4-yl]-c-4-cyano-t-4-(7-methacryloyloxyheptyl)-r-1-cyclohexylcarboxylat
[4'-Hexyl-1,1'-biphenyl-4-yl]-c-4-cyano-t-4-(7-methacryloyloxyheptyl)-r-1-cyclohexylcarboxylat
[4'-Heptyl-1,1'-biphenyl-4-yl]-c-4-cyano-t-4-(7-methacryloyloxyheptyl)-r-1-cyclohexylcarboxylat
[4'-Octyloxy-1,1'-biphenyl-4-yl]-c-4-cyano-t-4-(8-methacryloyloxyoctyl)-r-1-cyclohexylcarboxylat
[4'-Heptyloxy-1,1'-biphenyl-4-yl]-c-4-cyano-t-4-(8-methacryloyloxyoctyl)-r-1-cyclohexylcarboxylat
[4'-Hexyloxy-1,1'-biphenyl-4-yl]-c-4-cyano-t-4-(8-methacryloyloxyoctyl)-r-1-cyclohexylcarboxylat
[4'-Pentyloxy-1,1'-biphenyl-4-yl]-c-4-cyano-t-4-(8-methacryloyloxyoctyl)-r-1-cyclohexylcarboxylat
[4'-Butyloxy-1,1'-biphenyl-4-yl]-c-4-cyano-t-4-(8-methacryloyloxyoctyl)-r-1-cyclohexylcarboxylat
[4'-Propoxy-1,1'-biphenyl-4-yl]-c-4-cyano-t-4-(8-methacryloyloxyoctyl)-r-1-cyclohexylcarboxylat
[4'-Pentyl-1,1'-biphenyl-4-yl]-c-4-cyano-t-4-(8-methacryloyloxyoctyl)-r-1-cyclohexylcarboxylat
[4'-Hexyl-1,1'-biphenyl-4-yl]-c-4-cyano-t-4-(8-methacryloyloxyoctyl)-r-1-cyclohexylcarboxylat
[4'-Heptyl-1,1'-biphenyl-4-yl]-c-4-cyano-t-4-(8-methacryloyloxyoctyl)-r-1-cyclohexylcarboxylat
[4'-Octyloxy-1,1'-biphenyl-4-yl]-c-4-cyano-t-4-(9-methacryloyloxynonyl)-r-1-cyclohexylcarboxylat
[4'-Heptyloxy-1,1'-biphenyl-4-yl]-c-4-cyano-t-4-(9-methacryloyloxynonyl)-r-1-cyclohexylcarboxylat
[4'-Hexyloxy-1,1'-biphenyl-4-yl]-c-4-cyano-t-4-(9-methacryloyloxynonyl)-r-1-cyclohexylcarboxylat
[4'-Pentyloxy-1,1'-biphenyl-4-yl]-c-4-cyano-t-4-(9-methacryloyloxynonyl)-r-1-cyclohexylcarboxylat
[4'-Butyloxy-1,1'-biphenyl-4-yl]-c-4-cyano-t-4-(9-methacryloyloxynonyl)-r-1-cyclohexylcarboxylat
[4'-Propoxy-1,1'-biphenyl-4-yl]-c-4-cyano-t-4-(9-methacryloyloxynonyl)-r-1-cyclohexylcarboxylat
[4'-Pentyl-1,1'-biphenyl-4-yl]-c-4-cyano-t-4-(9-methacryloyloxynonyl)-r-1-cyclohexylcarboxylat
[4'-Hexyl-1,1'-biphenyl-4-yl]-c-4-cyano-t-4-(9-methacryloyloxynonyl)-r-1-cyclohexylcarboxylat
[4'-Heptyl-1,1'-biphenyl-4-yl]-c-4-cyano-t-4-(9-methacryloyloxynonyl)-r-1-cyclohexylcarboxylat
[4'-Octyloxy-1,1'-biphenyl-4-yl]-c-4-cyano-t-4-(10-methacryloyloxydecyl)-r-1-cyclohexylcarboxylat
[4'-Heptyloxy-1,1'-biphenyl-4-yl]-c-4-cyano-t-4-(10-methacryloyloxydecyl)-r-1-cyclohexylcarboxylat
[4'-Hexyloxy-1,1'-biphenyl-4-yl]-c-4-cyano-t-4-(10-methacryloyloxydecyl)-r-1-cyclohexylcarboxylat
[4'-Pentyloxy-1,1'-biphenyl-4-yl]-c-4-cyano-t-4-(10-methacryloyloxydecyl)-r-1-cyclohexylcarboxylat
[4'-Butyloxy-1,1'-biphenyl-4-yl]-c-4-cyano-t-4-(10-methacryloyloxydecyl)-r-1-cyclohexylcarboxylat
[4'-Propoxy-1,1'-biphenyl-4-yl]-c-4-cyano-t-4-(10-methacryloyloxydecyl)-r-1-cyclohexylcarboxylat
[4'-Pentyl-1,1'-biphenyl-4-yl]-c-4-cyano-t-4-(10-methacryloyloxydecyl)-r-1-cyclohexylcarboxylat
[4'-Hexyl-1,1'-biphenyl-4-yl]-c-4-cyano-t-4-(10-methacryloyloxydecyl)-r-1-cyclohexylcarboxylat
[4'-Heptyl-1,1'-biphenyl-4-yl]-c-4-cyano-t-4-(10-methacryloyloxydecyl)-r-1-cyclohexylcarboxylat
[4'-Octyloxy-1,1'-biphenyl-4-yl]-c-4-cyano-t-4-(11-methacryloyloxyundecyl)-r-1-cyclohexylcarboxylat
[4'-Heptyloxy-1,1'-biphenyl-4-yl]-c-4-cyano-t-4-(11-methacryloyloxyundecyl)-r-1-cyclohexylcarboxylat
[4'-Hexyloxy-1,1'-biphenyl-4-yl]-c-4-cyano-t-4-(11-methacryloyloxyundecyl)-r-1-cyclohexylcarboxylat
[4'-Pentyloxy-1,1'-biphenyl-4-yl]-c-4-cyano-t-4-(11-methacryloyloxyundecyl)-r-1-cyclohexylcarboxylat
[4'-Butyloxy-1,1'-biphenyl-4-yl]-c-4-cyano-t-4-(11-methacryloyloxyundecyl)-r-1-cyclohexylcarboxylat
[4'-Propoxy-1,1'-biphenyl-4-yl]-c-4-cyano-t-4-(11-methacryloyloxyundecyl)-r-1-cyclohexylcarboxylat
[4'-Pentyl-1,1'-biphenyl-4-yl]-c-4-cyano-t-4-(11-methacryloyloxyundecyl)-r-1-cyclohexylcarboxylat
[4'-Hexyl-1,1'-biphenyl-4-yl]-c-4-cyano-t-4-(11-methacryloyloxyundecyl)-r-1-cyclohexylcarboxylat
[4'-Heptyl-1,1'-biphenyl-4-yl]-c-4-cyano-t-4-(11-methacryloyloxyundecyl)-r-1-cyclohexylcarboxylat

Beispiel 14

Analog Beispiel 13 erhält man durch Umsetzung der Hydroxyverbindung mit Acrylsäure das entsprechende [4'-Octyloxy-1,1'-biphenyl-4-yl]-c-4-cyano-t-4-(6-acryloyloxyhexyl)-r-1-cyclohexylcarboxylat.

Analog werden hergestellt:

[4'-Heptyloxy-1,1'-biphenyl-4-yl]-c-4-cyano-t-4-(6-acryloyloxyhexyl)-r-1-cyclohexylcarboxylat
[4'-Hexyloxy-1,1'-biphenyl-4-yl]-c-4-cyano-t-4-(6-acryloyloxyhexyl)-r-1-cyclohexylcarboxylat
[4'-Pentyloxy-1,1'-biphenyl-4-yl]-c-4-cyano-t-4-(6-acryloyloxyhexyl)-r-1-cyclohexylcarboxylat
[4'-Butyloxy-1,1'-biphenyl-4-yl]-c-4-cyano-t-4-(6-acryloyloxyhexyl)-r-1-cyclohexylcarboxylat
[4'-Propoxy-1,1'-biphenyl-4-yl]-c-4-cyano-t-4-(6-acryloyloxyhexyl)-r-1-cyclohexylcarboxylat
[4'-Pentyl-1,1'-biphenyl-4-yl]-c-4-cyano-t-4-(6-acryloyloxyhexyl)-r-1-cyclohexylcarboxylat
[4'-Hexyl-1,1'-biphenyl-4-yl]-c-4-cyano-t-4-(6-acryloyloxyhexyl)-r-1-cyclohexylcarboxylat

[4'-Heptyl-1,1'-biphenyl-4-yl]-c-4-cyano-t-4-(6-acryloyloxyhexyl)-r-1-cyclohexylcarboxylat
[4'-Octyloxy-1,1'-biphenyl-4-yl]-c-4-cyano-t-4-(7-acryloyloxyheptyl)-r-1-cyclohexylcarboxylat
[4'-Heptyloxy-1,1'-biphenyl-4-yl]-c-4-cyano-t-4-(7-acryloyloxyheptyl)-r-1-cyclohexylcarboxylat
[4'-Hexyloxy-1,1'-biphenyl-4-yl]-c-4-cyano-t-4-(7-acryloyloxyheptyl)-r-1-cyclohexylcarboxylat
[4'-Pentyloxy-1,1'-biphenyl-4-yl]-c-4-cyano-t-4-(7-acryloyloxyheptyl)-r-1-cyclohexylcarboxylat
[4'-Butyloxy-1,1'-biphenyl-4-yl]-c-4-cyano-t-4-(7-acryloyloxyheptyl)-r-1-cyclohexylcarboxylat
[4'-Propoxy-1,1'-biphenyl-4-yl]-c-4-cyano-t-4-(7-acryloyloxyheptyl)-r-1-cyclohexylcarboxylat
[4'-Pentyl-1,1'-biphenyl-4-yl]-c-4-cyano-t-4-(7-acryloyloxyheptyl)-r-1-cyclohexylcarboxylat
[4'-Hexyl-1,1'-biphenyl-4-yl]-c-4-cyano-t-4-(7-acryloyloxyheptyl)-r-1-cyclohexylcarboxylat
[4'-Heptyl-1,1'-biphenyl-4-yl]-c-4-cyano-t-4-(7-acryloyloxyheptyl)-r-1-cyclohexylcarboxylat
[4'-Octyloxy-1,1'-biphenyl-4-yl]-c-4-cyano-t-4-(8-acryloyloxyoctyl)-r-1-cyclohexylcarboxylat
[4'-Heptyloxy-1,1'-biphenyl-4-yl]-c-4-cyano-t-4-(8-acryloyloxyoctyl)-r-1-cyclohexylcarboxylat
[4'-Hexyloxy-1,1'-biphenyl-4-yl]-c-4-cyano-t-4-(8-acryloyloxyoctyl)-r-1-cyclohexylcarboxylat
[4'-Pentyloxy-1,1'-biphenyl-4-yl]-c-4-cyano-t-4-(8-acryloyloxyoctyl)-r-1-cyclohexylcarboxylat
[4'-Butyloxy-1,1'-biphenyl-4-yl]-c-4-cyano-t-4-(8-acryloyloxyoctyl)-r-1-cyclohexylcarboxylat
[4'-Propoxy-1,1'-biphenyl-4-yl]-c-4-cyano-t-4-(8-acryloyloxyoctyl)-r-1-cyclohexylcarboxylat
[4'-Pentyl-1,1'-biphenyl-4-yl]-c-4-cyano-t-4-(8-acryloyloxyoctyl)-r-1-cyclohexylcarboxylat
[4'-Hexyl-1,1'-biphenyl-4-yl]-c-4-cyano-t-4-(8-acryloyloxyoctyl)-r-1-cyclohexylcarboxylat
[4'-Heptyl-1,1'-biphenyl-4-yl]-c-4-cyano-t-4-(8-acryloyloxyoctyl)-r-1-cyclohexylcarboxylat
[4'-Octyloxy-1,1'-biphenyl-4-yl]-c-4-cyano-t-4-(9-acryloyloxynonyl)-r-1-cyclohexylcarboxylat
[4'-Heptyloxy-1,1'-biphenyl-4-yl]-c-4-cyano-t-4-(9-acryloyloxynonyl)-r-1-cyclohexylcarboxylat
[4'-Hexyloxy-1,1'-biphenyl-4-yl]-c-4-cyano-t-4-(9-acryloyloxynonyl)-r-1-cyclohexylcarboxylat
[4'-Pentyloxy-1,1'-biphenyl-4-yl]-c-4-cyano-t-4-(9-acryloyloxynonyl)-r-1-cyclohexylcarboxylat
[4'-Butyloxy-1,1'-biphenyl-4-yl]-c-4-cyano-t-4-(9-acryloyloxynonyl)-r-1-cyclohexylcarboxylat
[4'-Propoxy-1,1'-biphenyl-4-yl]-c-4-cyano-t-4-(9-acryloyloxynonyl)-r-1-cyclohexylcarboxylat
[4'-Pentyl-1,1'-biphenyl-4-yl]-c-4-cyano-t-4-(9-acryloyloxynonyl)-r-1-cyclohexylcarboxylat
[4'-Hexyl-1,1'-biphenyl-4-yl]-c-4-cyano-t-4-(9-acryloyloxynonyl)-r-1-cyclohexylcarboxylat
[4'-Heptyl-1,1'-biphenyl-4-yl]-c-4-cyano-t-4-(9-acryloyloxynonyl)-r-1-cyclohexylcarboxylat
[4'-Octyloxy-1,1'-biphenyl-4-yl]-c-4-cyano-t-4-(10-acryloyloxydecyl)-r-1-cyclohexylcarboxylat
[4'-Heptyloxy-1,1'-biphenyl-4-yl]-c-4-cyano-t-4-(10-acryloyloxydecyl)-r-1-cyclohexylcarboxylat
[4'-Hexyloxy-1,1'-biphenyl-4-yl]-c-4-cyano-t-4-(10-acryloyloxydecyl)-r-1-cyclohexylcarboxylat
[4'-Pentyloxy-1,1'-biphenyl-4-yl]-c-4-cyano-t-4-(10-acryloyloxydecyl)-r-1-cyclohexylcarboxylat
[4'-Butyloxy-1,1'-biphenyl-4-yl]-c-4-cyano-t-4-(10-acryloyloxydecyl)-r-1-cyclohexylcarboxylat
[4'-Propoxy-1,1'-biphenyl-4-yl]-c-4-cyano-t-4-(10-acryloyloxydecyl)-r-1-cyclohexylcarboxylat
[4'-Pentyl-1,1'-biphenyl-4-yl]-c-4-cyano-t-4-(10-acryloyloxydecyl)-r-1-cyclohexylcarboxylat
[4'-Hexyl-1,1'-biphenyl-4-yl]-c-4-cyano-t-4-(10-acryloyloxydecyl)-r-1-cyclohexylcarboxylat
[4'-Heptyl-1,1'-biphenyl-4-yl]-c-4-cyano-t-4-(10-acryloyloxydecyl)-r-1-cyclohexylcarboxylat
[4'-Octyloxy-1,1'-biphenyl-4-yl]-c-4-cyano-t-4-(11-acryloyloxyundecyl)-r-1-cyclohexylcarboxylat
[4'-Heptyloxy-1,1'-biphenyl-4-yl]-c-4-cyano-t-4-(11-acryloyloxyundecyl)-r-1-cyclohexylcarboxylat
[4'-Hexyloxy-1,1'-biphenyl-4-yl]-c-4-cyano-t-4-(11-acryloyloxyundecyl)-r-1-cyclohexylcarboxylat
[4'-Pentyloxy-1,1'-biphenyl-4-yl]-c-4-cyano-t-4-(11-acryloyloxyundecyl)-r-1-cyclohexylcarboxylat
[4'-Butyloxy-1,1'-biphenyl-4-yl]-c-4-cyano-t-4-(11-acryloyloxyundecyl)-r-1-cyclohexylcarboxylat
[4'-Propoxy-1,1'-biphenyl-4-yl]-c-4-cyano-t-4-(11-acryloyloxyundecyl)-r-1-cyclohexylcarboxylat
[4'-Pentyl-1,1'-biphenyl-4-yl]-c-4-cyano-t-4-(11-acryloyloxyundecyl)-r-1-cyclohexylcarboxylat
[4'-Hexyl-1,1'-biphenyl-4-yl]-c-4-cyano-t-4-(11-acryloyloxyundecyl)-r-1-cyclohexylcarboxylat
[4'-Heptyl-1,1'-biphenyl-4-yl]-c-4-cyano-t-4-(11-acryloyloxyundecyl)-r-1-cyclohexylcarboxylat

<u>Beispiel 16</u>

a) Man erhitzt eine Lösung von 4,03 g c-4-(4'-Hydroxy-1,1'-biphenyl-4-yl)-1-(11-hydroxyundecyl)-r-1-cyclohexylcarbonitril [herstellbar durch Verseifung von c-4-(4'-Methoxy-1,1'-biphenyl-4-yl)-1-(11-hydroxyundecyl)-r-1-cyclohexylcarbonitril, das anlog Beispiel 5a und b dargestellt werden kann, in Gegenwart von Kalium-tert.butanolat in N-Methylpyrrolidinon], 10 g Methacrylsäure, 0,4 g Hydrochinon und 0,4 g p-Toluolsulfonsäuremonohydrat in 150 ml Chloroform 3 Tage am Wasserabscheider.

Man extrahiert die Reaktionsmischung, arbeitet die organische Phase auf und erhält nach Chromatographie an Kieselgel c-4-(4'-Hydroxy-1,1'-biphenyl-4-yl)-1-(11-methacryloyloxyundecyl)-r-1-cyclohexylcarbonitril.

b) Zu einer Lösung von 3,09 g der in a) hergestellten Verbindung, 819 mg (R)-2-Chlor-3-methyl-butansäure und 73,3 mg 4-Dimethylaminopyridin in 20 ml $CH_2Cl_2$ gibt man 1,21 g DCCI in 5 ml $CH_2Cl_2$ und rührt 18 Stunden bei Raumtemperatur. Der Niederschlag wird abgesaugt, das Filtrat eingeengt und nach Umkristallisation aus Ethanol erhält man optisch aktives c-4-[4'-((R)-2-Chlor-3-methylbutyryloxy)-1,1'-biphenyl-4-yl]-1-(11-methacryloyloxyundecyl)-r-1-cyclohexylcarbonitril.

Analog erhält man die folgenden, optisch aktiven, Verbindungen:

c-4-[4'-(2-Chlor-3-methyl-butyryloxy)-1,1'-biphenyl-4-yl]-1-(10-methacryloyloxydecyl)-r-1-cyclohexylcarbonitril
c-4-[4'-(2-Chlor-3-methyl-butyryloxy)-1,1'-biphenyl-4-yl]-1-(9-methacryloyloxynonyl)-r-1-cyclohexylcarbonitril
c-4-[4'-(2-Chlor-3-methyl-butyryloxy)-1,1'-biphenyl-4-yl]-1-(8-methacryloyloxyoctyl)-r-1-cyclohexylcarbonitril
c-4-[4'-(2-Chlor-3-methyl-butyryloxy)-1,1'-biphenyl-4-yl]-1-(7-methacryloyloxyheptyl)-r-1-cyclohexylcarbonitril
c-4-[4'-(2-Chlor-3-methyl-butyryloxy)-1,1'-biphenyl-4-yl]-1-(6-methacryloyloxyhexyl)-r-1-cyclohexylcarbonitril
c-4-[4'-(2-Chlor-3-methyl-butyryloxy)-1,1'-biphenyl-4-yl]-1-(5-methacryloyloxypentyl)-r-1-cyclohexylcarbonitril
c-4-[4'-(2-Chlor-3-methyl-butyryloxy)-1,1'-biphenyl-4-yl]-1-(11-acryloyloxyundecyl)-r-1-cyclohexylcarbonitril
c-4-[4'-(2-Chlor-3-methyl-butyryloxy)-1,1'-biphenyl-4-yl]-1-(10-acryloyloxydecyl)-r-1-cyclohexylcarbonitril
c-4-[4'-(2-Chlor-3-methyl-butyryloxy)-1,1'-biphenyl-4-yl]-1-(9-acryloyloxynonyl)-r-1-cyclohexylcarbonitril
c-4-[4'-(2-Chlor-3-methyl-butyryloxy)-1,1'-biphenyl-4-yl]-1-(8-acryloyloxyoctyl)-r-1-cyclohexylcarbonitril
c-4-[4'-(2-Chlor-3-methyl-butyryloxy)-1,1'-biphenyl-4-yl]-1-(7-acryloyloxyheptyl)-r-1-cyclohexylcarbonitril
c-4-[4'-(2-Chlor-3-methyl-butyryloxy)-1,1'-biphenyl-4-yl]-1-(6-acryloyloxyhexyl)-r-1-cyclohexylcarbonitril
c-4-[4'-(2-Chlor-3-methyl-butyryloxy)-1,1'-biphenyl-4-yl]-1-(5-acryloy1oxypentyl)-r-1-cyclohexylcarbonitril

Beispiel 17

a) Zu einer Lösung von 4,48 g c-4-[4'-Octyloxy-1,1'-biphenyl-4-yl]-1-[(S)-2-hydroxypropyl]-r-1-cyclohexylcarbonitril und 4,2 g 11-Iodundecen-1 in 50 ml 1,2-Dimethoxyethan werden 252 mg Natriumhydrid gegeben und 18 Stunden bei Raumtemperatur gerührt. Man gießt die Mischung auf Eiswasser und extrahiert mit $CH_2Cl_2$. Nach Aufarbeitung der organischen Phase und Chromatographie an Kieselgel erhält man c-4-[4'-Octyloxy-1,1'-biphenyl-4-yl]-1-[2-methyl-3-oxa-tetradec-13-enyl]-r-1-cyclohexylcarbonitril (optisch aktiv).

b) Das in a) dargestellte Olefinderivat wird analog Beispiel 5b) umgesetzt zu c-4-[4'-Octyloxy-1,1'-biphenyl-4-yl]-1-[2-methyl-3-oxa-14-hydroxy-tetradecyl]-r-1-cyclohexylcarbonitril (optisch aktiv), welches analog Beispiel 6 mit Methacrylsäure umgesetzt wird zu c-4-[4'-Octyloxy-1,1'-biphenyl-4-yl]-1-[2-methyl-3-oxa-14-(methacryloyloxy)-tetradecyl]-r-1-cyclohexylcarbonitril (optisch aktiv).

Analog werden hergestellt:

c-4-[4'-Ethoxy-1,1'-biphenyl-4-yl]-1-[2-methyl-3-oxa-14-(methacryloyloxy)-tetradecyl]-r-1-cyclohexylcarbonitril, optisch aktiv
c-4-[4'-Propoxy-1,1'-biphenyl-4-yl]-1-[2-methyl-3-oxa-14-(methacryloyloxy)-tetradecyl]-r-1-cyclohexylcarbonitril, optisch aktiv
c-4-[4'-Butyloxy-1,1'-biphenyl-4-yl]-1-[2-methyl-3-oxa-14-(methacryloyloxy)-tetradecyl]-r-1-cyclohexylcarbonitril, optisch aktiv
c-4-[4'-Pentyloxy-1,1'-biphenyl-4-yl]-1-[2-methyl-3-oxa-14-(methacryloyloxy)-tetradecyl]-r-1-cyclohexylcarbonitril, optisch aktiv
c-4-[4'-Hexyloxy-1,1'-biphenyl-4-yl]-1-[2-methyl-3-oxa-14-(methacryloyloxy)-tetradecyl]-r-1-cyclohexylcarbonitril, optisch aktiv
c-4-[4'-Heptyloxy-1,1'-biphenyl-4-yl]-1-[2-methyl-3-oxa-14-(methacryloyloxy)-tetradecyl]-r-1-cyclohexylcarbonitril, optisch aktiv
c-4-[4'-Butyl-1,1'-biphenyl-4-yl]-1-[2-methyl-3-oxa-14-(methacryloyloxy)-tetradecyl]-r-1-cyclohexylcarbonitril, optisch aktiv
c-4-[4'-Pentyl-1,1'-biphenyl-4-yl]-1-[2-methyl-3-oxa-14-(methacryloyloxy)-tetradecyl]-r-1-cyclohexylcarbonitril, optisch aktiv
c-4-[4'-Hexyl-1,1'-biphenyl-4-yl]-1-[2-methyl-3-oxa-14-(methacryloyloxy)-tetradecyl]-r-1-cyclohexylcarbonitril, optisch aktiv
c-4-[4'-Heptyl-1,1'-biphenyl-4-yl]-1-[2-methyl-3-oxa-14-(methacryloyloxy)-tetradecyl]-r-1-cyclohexylcarbonitril, optisch aktiv
c-4-[4'-Octyloxy-1,1'-biphenyl-4-yl]-1-[2-methyl-3-oxa-14-(acryloyloxy)-tetradecyl]-r-1-cyclohexylcarbonitril, optisch aktiv
c-4-[4'-Ethoxy-1,1'-biphenyl-4-yl]-1-[2-methyl-3-oxa-14-(acryloyloxy)-tetradecyl]-r-1-cyclohexylcarbonitril, optisch aktiv
c-4-[4'-Propoxy-1,1'-biphenyl-4-yl]-1-[2-methyl-3-oxa-14-(acryloyloxy)-tetradecyl]-r-1-cyclohexylcarbonitril,

optisch aktiv
c-4-[4'-Butyloxy-1,1'-biphenyl-4-yl]-1-[2-methyl-3-oxa-14-(acryloyloxy)-tetradecyl]-r-1-cyclohexylcarbonitril, optisch aktiv
c-4-[4'-Pentyloxy-1,1'-biphenyl-4-yl]-1-[2-methyl-3-oxa-14-(acryloyloxy)-tetradecyl]-r-1-cyclohexylcarbonitril, optisch aktiv
c-4-[4'-Hexyloxy-1,1'-biphenyl-4-yl]-1-[2-methyl-3-oxa-14-(acryloyloxy)-tetradecyl]-r-1-cyclohexylcarbonitril, optisch aktiv
c-4-[4'-Heptyloxy-1,1'-biphenyl-4-yl]-1-[2-methyl-3-oxa-14-(acryloyloxy)-tetradecyl]-r-1-cyclohexylcarbonitril, optisch aktiv
c-4-[4'-Butyl-1,1'-biphenyl-4-yl]-1-[2-methyl-3-oxa-14-(acryloyloxy)-tetradecyl]-r-1-cyclohexylcarbonitril, optisch aktiv
c-4-[4'-Pentyl-1,1'-biphenyl-4-yl]-1-[2-methyl-3-oxa-14-(acryloyloxy)-tetradecyl]-r-1-cyclohexylcarbonitril, optisch aktiv
c-4-[4'-Hexyl-1,1'-biphenyl-4-yl]-1-[2-methyl-3-oxa-14-(acryloyloxy)-tetradecyl]-r-1-cyclohexylcarbonitril, optisch aktiv
c-4-[4'-Heptyl-1,1'-biphenyl-4-yl]-1-[2-methyl-3-oxa-14-(acryloyloxy)-tetradecyl]-r-1-cyclohexylcarbonitril, optisch aktiv

Beispiel A

Eine Lösung von 2 g 4-[c-1-Cyano-r-4-(r-4-pentyl-t-1-cylohexyl)-t-1-cyclohexyl]-butylmethacrylat (herstellbar nach Beispiel 4) und 16,4 mg Azobisisobutyronitril in 10 ml Toluol wird unter Stickstoff 20 Stunden auf 60° erhitzt. Das Polymer wird zweimal aus Ethanol umgefällt und man erhält eine farbloses, faseriges Polymer mit $S_A$ 172° I.

Beispiel C

Eine Lösung von 1,3 g c-4-[4'-Octyloxy-1,1'-biphenyl-4-yl]-1-(4-methacryloyloxybutyl)-r-1-cyclohexylcarbonitril (aus Beispiel 6) und 8 mg Azobisisobutyronitril in 6,5 ml Toluol wird unter Stickstoff 20 Stunden auf 60° erhitzt. Nach Reinigung durch Umfällen aus Ethanol erhält man ein farbloses, faseriges Polymer mit $S_C$ 180° $S_A$ 260° I.

Beispiel D

Eine Lösung von 942 mg c-4-[4'-(11-Methacryloyloxyundecyloxy)-1,1'-biphenyl-4-yl]-1-octyl-r-1-carbonitril (aus Beispiel 9) und 5 mg Azobisisobutyronitril in 5 ml Toluol wird unter $N_2$ 20 Stunden auf 60 ° erhitzt. Das Polymer wird aus Methanol umgefällt und man erhält ein weißes, faseriges Polymer mit der Phasenabfolge G 70° $S_X$ 121° $S_C$ 140° $S_A$ 202° I.

Beispiel E

Analog Beispiel C erhält man aus 4,8 g [4'-Octyloxy-1,1'-biphenyl-4-yl]-c-4-cyano-t-4-(6-methacryloyloxyhexyl)-r-1-cyclohexylcarboxylat (aus Beispiel 13) ein weißes, faseriges Polymer, das einen breiten flüssigkristallinen Bereich zeigt.

Beispiel F

Analog Beispiel C erhält man aus [4'-Octyloxy-1,1'-biphenyl-4-yl]-c-4-cyano-t-4-(6-acryloyloxyhexyl)-r-1-cyclohexylcarboxylat (aus Beispiel 14) ein Polymer, das einen breiten flüssigkristallinen Bereich zeigt.

Beispiel H

Man polymerisiert c-4-[-4'-(R)-2-Chlor-3-methylbutyryloxy-1,1'-biphenyl-4-yl]-1-(11-methacryloyloxyundecyl)-r-1-cyclohexylcarbonitril (aus Beispiel 16) mit Azobisisobutyronitril in U-Mehthylpyrrolidinon unter $N_2$ bei 60° 20 Stunden lang und erhält ein farbloses Polymer mit breitem flüssigkristallinem Phasenbereich.

Beispiel I

Analog Beispiele H erhält man aus c-4-[4'-(2-Methylbutyloxy)-1,1'-biphenyl-4-yl]-1-(11-methacryloylundecyl)-r-1-cyclohexylcarbonitril (optisch aktiv) das entsprechende Polymerprodukt mit breitem flüssigkristallinem Phasenbereich.

Beispiel J

Eine Lösung von 1,10 g c-4-[4'-(2-Methylbutyloxy)-1,1'-biphenyl-4-yl]-1-(undec-10-enyl)-r-1-cyclohexylcarbonitril (optisch aktiv), 120 mg Polymethylhydridosiloxan und 80 µl einer 0,4%igen Lösung von Hexachloroplatinsäure in THF/Ethanol (98:2) wird auf 80° erhitzt Nach 2 Tagen ist die Aufpfropfung beendet und man erhält das Polymer mit breitem flüssigkristallinem Phasenbereich durch Ausfällen aus Ethanol.

Beispiel K

Analog Beispiel H wird c-4-[4'-Octyloxy-1,1'-biphenyl-4-yl]-1-[2-methyl-3-oxa-14-(methacryloyloxy)-tetradecyl]-r-1-cyclohexylcarbonitril (optisch aktiv) polmerisiert und man erhält ein farbloses Polymer mit breitem flüssigkristallinem Phasenbereich.

**Patentansprüche**

**1.** Flüssigkristalline Phasen aufweisende Polyacrylate, Polymethacrylate oder Polysiloxane, die chemisch gebundene mesogene Gruppen der Formel XIaa oder XIcc enthalten

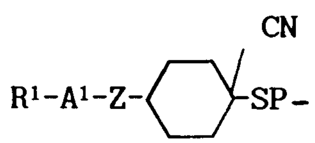

XIaa

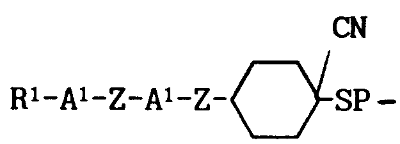

XIcc

worin

R[1] eine Alkylgruppe mit bis zu 15 C-Atomen, worin auch eine oder mehrere $CH_2$-Gruppen durch eine Gruppierung aus der Gruppe -O-, -CO-O-, -O-CO-, -CRR'-T- und -CH=CH-(trans) ersetzt sein können, wobei 2 Heteroatome nicht miteinander verknüpft sind,

A[1] jeweils unabhängig voneinander eine unsubstituierte oder eine durch CN substituierte 1,4-Cyclohexylengruppe, oder eine 1,4-Phenylengruppe,

Z jeweils -CO-O-, -O-CO-, $-CH_2CH_2$ oder eine Einfachbindung,

Sp Alkylen mit 2-18 C-Atomen, worin auch eine oder zwei nicht benachbarte $CH_2$-Gruppen durch -O-, -CO-, -O-CO-, -CO-O-, -CRR'-T- oder -CH=CH-ersetzt sein können, oder eine Einfachbindung,

T -COO-, -OCO- oder eine Einfachbindung,

R H oder eine Alkylgruppe mit bis zu 6 C-Atomen und

R' Halogen oder CN

bedeuten.

**2.** Verwendung von Polymerzusammensetzungen nach Anspruch 1 als organische Substrate in der Elektronik für die Faser- und Folientechnik.

**3.** Verwendung von Polymerzusammensetzungen nach Anspruch 1 als Materialien für die nichtlineare Optik.

**4.** Verbindungen der Formel I'

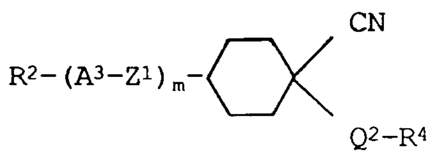

I'

worin

- $R^2$ eine Alkylgruppe mit bis zu 15 C-Atomen, worin auch eine oder mehrere $CH_2$-Gruppen durch eine Gruppierung aus der Gruppe -O-, -CO-O-, -O-CO-, -CH=CH-(trans) und -CRR'-T- ersetzt sein können, wobei 2 Heteroatome nicht miteinander verknüpft sind,
- $A^3$ jeweils eine unsubstituierte oder eine durch CN substituierte 1,4-Cyclohexylengruppe, oder eine 1,4-Phenylengruppe,
- $Z^1$ -CO-O-, -O-CO-, $-CH_2CH_2-$ oder eine Einfachbindung,
- m 1 oder 2,
- $Q^2$ Alkylen mit 3-18 C-Atomen, worin auch eine oder zwei nicht benachbarte $CH_2$-Gruppen durch -O-, -CO-, -O-CO-, -CO-O-, -CRR'-T- oder CH=CH-ersetzt sein können,
- R H oder ein Alkylgruppe mit bis zu 6 C-Atomen,
- R' Halogen oder CN,
- T -CO-O-, -O-CO- oder eine Einfachbindung,
- $R^4$ eine unsubstituierte oder durch eine $CH_3$-Gruppe substituierte Vinylgruppe, oder eine Carboxy- oder Hydroxygruppe,

mit der Maßgabe, daß im Falle $R^4$ = unsubstituiertes Vinyl und $Q^2$ = Alkylen oder Alkylenoxy, die Alkylengruppe mindestens 4 C-Atome aufweist.

5. Verwendung der Verbindungen der Formel I' nach Anspruch 4 als polymerisierbare Flüssigkristallmaterialien.

6. Verwendung der Verbindungen der Formel I' nach Anspruch 4 zur Herstellung von flüssigkristalline Phasen aufweisende Polymerzusammensetzungen nach Anspruch 1.

**Claims**

1. Polyacrylates, polymethacrylates or polysiloxanes which exhibit liquid-crystalline phases and which contain chemically bonded mesogenic groups of the formula XIaa or XIcc

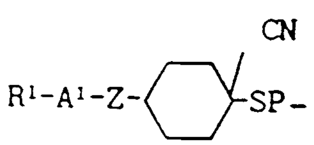

XIaa

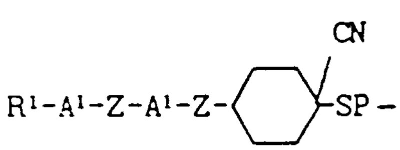

XIcc

in which

- $R^1$ is an alkyl group which has up to 15 C atoms and in which one or more $CH_2$ groups can also be replaced by a grouping belonging to the group comprising -O-, -CO-O-, -O-CO-, -CRR'-T- and -CH=CH-(trans), 2 heteroatoms not being attached to one another,
- the $A^1$ independently of one another are each an unsubstituted or a CN-substituted 1,4-cyclohexylene group, or a 1,4-phenylene group,
- the Zs are each -CO-O-, -O-CO-, $-CH_2CH_2$, or a single bond,
- Sp is alkylene which has 2-18 C atoms and in which one or two non-adjacent $CH_2$ groups can also be replaced by -O-, -CO-, -O-CO-, -CO-O-, -CRR'-T- or -CH=CH-, or is a single

bond,

T is -COO-, -OCO- or a single bond,

R is H or an alkyl group having up to 6 C atoms, and

R' is halogen or CN.

**2.** Use of a polymer composition according to Claim 1 as an organic substrate in electronics for fibre and films technology.

**3.** Use of a polymer composition according to Claim 1 as a material for non-linear optics.

**4.** Compounds of the formula I'

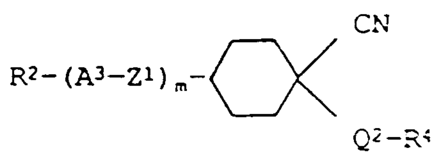

I'

in which

$R^2$ is an alkyl group which has up to 15 C atoms and in which one or more $CH_2$ groups can also be replaced by a grouping belonging to the group comprising -O-, -CO-O-, -O-CO-, -CH=CH- (trans) and -CRR'-T-, 2 heteroatoms not being attached to one another,

$A^3$ is in each case a 1,4-cyclohexylene group which is unsubstituted or substituted by CN, or is a 1,4-phenylene group,

$Z^1$ is -CO-O- -O-CO- $-CH_2CH_2-$ or a single bond,

m is 1 or 2,

$Q^2$ is alkylene which has 3-18 C atoms and in which one or two non-adjacent $CH_2$ groups can also be replaced by -O-, -CO-, -O-CO-, -CO-O-, -CRR'-T- or -CH=CH-,

R is H or an alkyl group having up to 6 C atoms,

R' is halogen or CN,

T is -CO-O-, -O-CO- or a single bond,

$R^4$ is a vinyl group which is unsubstituted or substituted by a $CH_3$ group, or is a carboxyl or hydroxyl group,

subject to the proviso that, in the event that $R^4$ = unsubstituted vinyl and $Q^2$ = alkylene or alkyleneoxy, the alkylene group contains at least 4 C atoms.

**5.** Use of the compounds of the formula I' according to Claim 4 as the polymerisable liquid-crystal materials.

**6.** Use of the compounds of the formula I' according to Claim 4 for the preparation of polymer compositions, according to Claim 1, which exhibit liquid-crystalline phases.

## Revendications

**1.** Polyacrylates, polyméthacrylates ou polysiloxanes présentant des phases à cristaux liquides, qui contiennent des groupes mésogènes chimiquement liés de formule XI aa ou XIcc

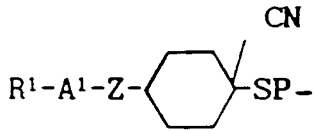

XIaa

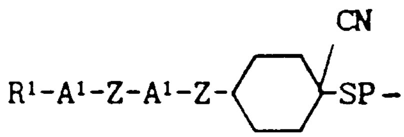

XIcc

où

$R^1$ désigne un groupe alkyle ayant jusqu'à 15 atomes de carbone, dans lequel également un ou plusieurs groupes $CH_2$ peuvent être remplacés par un groupement choisi parmi -O-, -CO-O-, -O-CO-, -CRR'-T- et -CH = CH-(trans), tandis que 2 hétéroatomes ne sont pas reliés entre eux,

$A^1$ désigne dans chaque cas, indépendamment l'un de l'autre, un groupe 1,4-cyclohexylène non-substitué ou substitué par CN, ou un groupe 1,4-phénylène,

Z désigne dans chaque cas -CO-O-, -O-CO-, $-CH_2CH_2$ ou une liaison simple,

Sp désigne un groupe alkylène ayant 2-18 atomes de carbone, dans lequel également un ou deux groupes $CH_2$ non vicinaux peuvent être remplacés par -O-, -CO-, -O-CO-, -CO-O-, -CRR'-T- ou -CH = CH-, ou une liaison simple,

T est -COO-, -OCO- ou une liaison simple,

R est H ou un groupe alkyle ayant jusqu'à 6 atomes de carbone, et

R' est un halogène ou CN.

**2.** Utilisation de compositions de polymères selon la revendication 1 comme substrats organiques en électronique pour la technique des fibres et la technique des films.

**3.** Utilisation de compositions de polymères selon la revendication 1 comme matériaux pour l'optique non-linéaire.

**4.** Composés de formule I'

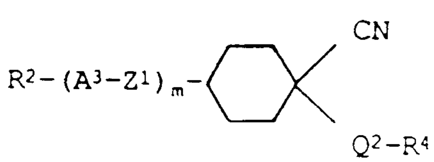

I'

où

$R^2$ désigne un groupe alkyle ayant jusqu'à 15 atomes de carbone, dans lequel également un ou plusieurs groupes $CH_2$ peuvent être remplacés par un groupement choisi parmi -O-, -CO-O-, -O-CO-, -CH = CH-(trans) et -CRR'-T-, tandis que 2 hétéroatomes ne sont pas reliés entre eux,

$A^3$ désigne dans chaque cas un groupe 1,4-cyclohexylène non-substitué ou substitué par CN, ou un groupe 1,4-phénylène,

$Z^1$ désigne -CO-O-, -O-CO-, $-CH_2CH_2-$ ou une liaison simple,

m est 1 ou 2,

$Q^2$ désigne un groupe alkylène ayant 3-18 atomes de carbone, dans lequel également un ou deux groupes $CH_2$ non vicinaux peuvent être remplacés par -O-, -CO-, -O-CO-, -CO-O-, -CRR' -T- ou -CH = CH-,

R est H ou un groupe alkyle ayant jusqu'à 6 atomes de carbone,
R' est un halogène ou CN,
T est -CO-O-, -O-CO- ou une liaison simple,

$R^4$ désigne un groupe vinyle non-substitué ou substitué par un groupe $CH_3$, ou un groupe carboxy ou hydroxy,

avec pour condition que dans le cas où $R^4$ est un groupe vinyle non-substitué et $Q^2$ est un groupe alkylène ou alkylèneoxy, le groupe alkylène présente au moins 4 atomes de carbone.

**5.** Utilisation des composés de formule I' selon la revendication 4 comme matériaux en cristaux liquides polymérisables.

**6.** Utilisation des composés de formule I' selon la revendication 4 pour la préparation de compositions de polymères présentant des phases à cristaux liquides selon la revendication 1.